# EUROPEAN PATENT SPECIFICATION

(11) **EP 3 436 049 B1**
(45) Date of publication and mention of the grant of the patent: **12.01.2022**
(21) Application number: 17773414.2
(22) Date of filing: 29.03.2017
(51) Int. Cl.: A61K 38/22

(54) **ANGIOPOIETIN-LIKE PROTEIN 8 (ANGPTL8)**
ANGIOPOIETIN-ÄHNLICHES PROTEIN 8 (ANGPTL8)
PROTÉINE 8 DE TYPE ANGIOPOÏÉTINE (ANGPTL8)

(30) Priority: 31.03.2016 US 201662315918 P
(43) Date of publication of application: 06.02.2019
(73) Proprietor: Baylor Research Institute, Dallas, TX 75201 (US)
(72) Inventor: CHEN, Shuyuan, Dallas, Texas 75201 (US); GRAYBURN, Paul A., Dallas, Texas 75201 (US)
(74) Representative: J A Kemp LLP
(86) International application number: PCT/IB2017/051801
(87) International publication number: WO 2017/168348

(56) References cited:
- WO-A1-2014/152940
- WO-A1-2016/054494
- US-A1- 2006 020 158
- US-A1- 2015 174 203
- US-A1- 2015 353 618
- CHEN SHUYUAN ET AL: "ANGPTL8 reverses established adriamycin cardiomyopathy by stimulating adult cardiac progenitor cells.", ONCOTARGET 06 DEC 2016, vol. 7, no. 49, 6 December 2016 (2016-12-06), pages 80391-80403, XP009516201, ISSN: 1949-2553
- CHEN JIAXI ET AL: "In vivo targeted delivery of ANGPTL8 gene for beta cell regeneration in rats.", DIABETOLOGIA MAY 2015, vol. 58, no. 5, May 2015 (2015-05), pages 1036-1044, XP035479123, ISSN: 1432-0428
- GUSAROVA et al.: "ANGPTL8 (Betatrophin) Does Not Control Pancreatic Beta Cell Expansion", Cell, vol. 159, no. 3, 23 October 2014 (2014-10-23), pages 691-696, XP029084854,
- PEIN et al.: "Cardiac abnormalities 15 years and more after adriamycin therapy in 229 childhood survivors of a solid tumour at the Institut Gustave Roussy", British Journal of Cancer, vol. 91, no. 1, 5 July 2004 (2004-07-05), pages 37-44, XP055429019,
- SEIDMAN et al.: "Cardiac Dysfunction in the Trastuzumab Clinical Trials Experience", Journal of Clinical Oncology, vol. 20, no. 5, 1 March 2002 (2002-03-01), pages 1215-1221, XP009512582, ISSN: 0732-183X, DOI: 10.1200/JCO.2002.20.5.1215
- FU et al.: "A lipasin/Angptl8 monoclonal antibody lowers mouse serum triglycerides involving increased postprandial activity of the cardiac lipoprotein lipase", Sci Rep., vol. 5, 21 December 2015 (2015-12-21), page 18502, XP055360939,
- TSENG et al.: Emerging Regulation and Function of Betatrophin . Int. J. Mol. Sci., vol. 15, no. 12, 18 December 2014 (2014-12-18), pages 23640-23657, XP055360816,

### SEQUENCE LISTING

<110> BAYLOR RESEARCH INSTITUTE
<120> ANGIOPOIETIN-LIKE PROTEIN 8 (ANGPTL8)
<130> BHCS.P0510WO
<140> Not Yet Assigned
   <141> 2017-03-29
<150> 62/315,918
   <151> 2016-03-31
<160> 2
<170> PatentIn version 3.5
<210> 1
   <211> 888
   <212> DNA
   <213> Homo sapiens
<400> 1
   ataccttaga ccctcagtca tgccagtgcc tgctctgtgc ctgctctggg ccctggcaat        60
   ggtgacccgg cctgcctcag cggcccccat gggcggccca gaactggcac agcatgagga       120
   gctgaccctg ctcttccatg ggaccctgca gctgggccag gccctcaacg gtgtgtacag       180
   gaccacggag ggacggctga caaaggccag gaacagcctg ggtctctatg gccgcacaat       240
   agaactcctg gggcaggagg tcagccgggg ccgggatgca gcccaggaac ttcgggcaag       300
   cctgttggag actcagatgg aggaggatat tctgcagctg caggcagagg ccacagctga       360
   ggtgctgggg gaggtggccc aggcacagaa ggtgctacgg gacagcgtgc agcggctaga       420
   agtccagctg aggagcgcct ggctgggccc tgcctaccga gaatttgagg tcttaaaggc       480
   tcacgctgac aagcagagcc acatcctatg ggccctcaca ggccacgtgc agcggcagag       540
   gcgggagatg gtggcacagc agcatcggct gcgacagatc caggagagac tccacacagc       600
   ggcgctccca gcctgaatct gcctggatgg aactgaggac caatcatgct gcaaggaaca       660
   cttccacgcc ccgtgaggcc cctgtgcagg gaggagctgc ctgttcactg ggatcagcca       720
   gggcgccggg ccccacttct gagcacagag cagagacaga cgcaggcggg gacaaaggca       780
   gaggatgtag ccccattggg gaggggtgga ggaaggacat gtaccctttc atgcctacac       840
   acccctcatt aaagcagagt cgtggcatct caaaaaaaaa aaaaaaaa                     888
<210> 2
   <211> 198
   <212> **PRT**
   <213> Homo sapiens
<400> 2
   Met Pro Val Pro Ala Leu Cys Leu Leu Trp Ala Leu Ala Met Val Thr
   1               5                   10                  15
   Arg Pro Ala Ser Ala Ala Pro Met Gly Gly Pro Glu Leu Ala Gln His
               20                  25                  30
   Glu Glu Leu Thr Leu Leu Phe His Gly Thr Leu Gln Leu Gly Gln Ala
           35                  40                  45
   Leu Asn Gly Val Tyr Arg Thr Thr Glu Gly Arg Leu Thr Lys Ala Arg
       50                  55                  60
   Asn Ser Leu Gly Leu Tyr Gly Arg Thr Ile Glu Leu Leu Gly Gln Glu
   65                  70                  75                  80
   Val Ser Arg Gly Arg Asp Ala Ala Gln Glu Leu Arg Ala Ser Leu Leu
                   85                  90                  95
   Glu Thr Gln Met Glu Glu Asp Ile Leu Gln Leu Gln Ala Glu Ala Thr
               100                 105                 110
   Ala Glu Val Leu Gly Glu Val Ala Gln Ala Gln Lys Val Leu Arg Asp
           115                 120                 125
   Ser Val Gln Arg Leu Glu Val Gln Leu Arg Ser Ala Trp Leu Gly Pro
       130                 135                 140
   Ala Tyr Arg Glu Phe Glu Val Leu Lys Ala His Ala Asp Lys Gln Ser
   145                 150                 155                 160
   His Ile Leu Trp Ala Leu Thr Gly His Val Gln Arg Gln Arg Arg Glu
                   165                 170                 175
   Met Val Ala Gln Gln His Arg Leu Arg Gln Ile Gln Glu Arg Leu His
               180                 185                 190
   Thr Ala Ala Leu Pro Ala
           195

## Description

Aspects of the disclosure concern at least the fields of cell biology, molecular biology, biochemistry, pharmaceuticals, and medicine, including at least cardiac medicine.

### BACKGROUND

Heart failure (HF) is a common disease affecting almost 6 million Americans [Roger, *et al.,* 2012]. Traditional medical therapy for HF is directed toward relief of symptoms and blockade of neurohormonal activation. In patients with advanced stages of HF, heart transplant or left ventricular assist devices may be appropriate, but are expensive and have limitations and complications. The ideal goal for HF therapy is myocardial regeneration. It is currently known that cardiac myocytes are capable of regeneration, *via* various mechanisms, including self-replication of pre-existing adult cardiac muscle cells [Senyo, *et al.,* 2013; Eulalio, *et al.,* 2012], differentiation of resident cardiac progenitor cells [Smart, *et al.,* 2011; Bolli, *et al.,* 2011], dedifferentiation and proliferation of adult cardiac muscle cells [Beltrami, *et al.,* 2003; Jopling, *et al.,* 2010; Porrello, *et al.,* 2011] and transdifferentiation of fibroblast cells into cardiac muscle cells [Song, *et al.,* 2012; Qian, *et al.,* 2012]. However, the magnitude of myocardial regeneration triggered by such mechanisms is small and it remains unclear whether myocardial regeneration in heart failure is sufficient to reverse established cardiomyopathy. Multiple clinical trials of stem cell therapy have been attempted but without clear improvement in cardiac function [Nowbar, *et al.,* 2014]. Part of this problem may be the fact that patients with advanced HF often have lack of blood supply to the heart and extensive scar tissue due to ischemic cardiomyopathy or long-standing non-ischemic cardiomyopathy with extensive fibrosis.

The LIM homeodomain transcription factor (ISL-1 or Islet1) is a biomarker of cardiac progenitors or neural stem cells that give rise to the right ventricle, atria and outflow tract during cardiac development [Laugwitz, *et al.,* 2005]. These cells can differentiate to the different cell types that form an adult heart (cardiomyocytes, smooth muscle cells, and endothelial cells) [Moretti, *et al.,* 2006; Di Felice & Zummo, 2013], However ISL-1positive progenitor cells are usually quiescent during adult heart, being present in parasympathetic neurons, smooth muscle cells, a few cardiomyocytes within the proximal aorta and pulmonary artery and more abundantly in the sinoatrial node.

The lining of the adult heart consists of multi-potent epicardial mesothelial cells (EMCs) and is referred to as the epicardium, which is generated from the proepicardial organ during cardiac development. At a given time during development, a subpopulation of these epicardial cells, termed epicardium-derived cells (EPDCs), undergo epithelial-tomesenchymal transition (EMT), and hereby differentiate into all main cell lineages of the heart [Masters & Riley, 2014]. This mechanism may likely be conserved in the adult organ, and emerging evidence underscore that adult EMCs primarily undergo fibrogenic EMT upon cardiac stress, such as hypertension or infarction, to generate myofibroblast-like cells [Smart, *et al.,* 2012]. These cells can contribute to the development of cardiac disease, such as fibrosis, potentially leading to impaired cardiac performance and arrhythmias, including sudden death [Smart, *et al.,* 2011]. Yet, very little is known of the molecular and cellular aspects of these EMCs as well as associated factors, and their implication in EMT and cardiac fibrosis. Thus, more knowledge on the molecular circuits that regulate EMT of these EMCs will be invaluable in order to inhibit cardiac fibrosis and hereby prevent or treat certain heart diseases.

Angiopoietin-like proteins are a family of 8 member proteins that are structurally similar to the angiopoietins, all containing an amino-terminal coiled-coil domain, a linker region, and a carboxy-terminal fibrinogen-like domain except *ANGPTL8. ANGPTLs* have various functions in lipid metabolism, inflammation, cancer cell invasion, hematopoietic stem activity. *ANGPTL8* (also known as *C19orf80*) is a 596 bp cDNA with a short secret sequence in the N-terminus that is highly enriched in the liver and adipocytes [Zhang, 2012; Fu, *et al.,* 2013; Zhang, 2013; Ren, *et al.,* 2012; Quagliarini, *et al.,* 2012; Wang, *et al.,* 2013]. Yi et al [Yi, e al., 2013; Yi, *et al.,* 2014] found that a hepatic overexpression of *ANGPTL8* after a hydrodynamic injection of non-viral sleeping beauty transposon plasmids into the tail vein of adult mice had several effects: it stimulated the proliferation of pancreatic beta cells, increased insulin secretion, improved glucose tolerance and expanded the mass of the beta cell islets. Pancreatic beta cell proliferation induced by *ANGPTL8* was confirmed, but it was not able to reverse diabetes in rats with STZ- induced diabetes [Chen, *et al.,* 2015] or obese rhesus monkey with naturally occurring diabetes.

The present disclosure satisfies a long felt need in the art to provide efficacious therapy for cardiac medical conditions.

### BRIEF SUMMARY

The present invention provides Angiopoietin-like 8 (ANGPTL8) for use in a method of treating a cardiac-related medical condition by myocardial regeneration in an individual, comprising the step of providing an effective amount of said ANGPTL8 to the individual. The invention is further defined in the appended claims.

The present disclosure provides methods and/or compositions related to therapy and/or prevention of one or more cardiac-related medical conditions. Aspects of the present disclosure concern regeneration of tissue, including muscle tissue, such as myocardial tissue. Certain aspects relate to reversal of a cardiac-related medical condition (or improvement of at least one symptom thereof), including at least cardiac disease, cardiomyopathy (including drug-induced cardiomyopathy), cardiotoxicity, congestive heart failure, ischemic heart disease, acute myocardial infarction, atrial fibrillation, and arrhythmias.

Particular aspects of the disclosure concern delivery of a polynucleotide, protein, peptide, or mixture thereof to a certain tissue for proliferation and/or differentiation of certain cells in the tissue. The tissue may be of any kind, but in specific cases it is muscle tissue, including cardiac tissue. In particular aspects, methods and compositions of the disclosure allow for self-replication of pre-existing adult cardiac muscle cells, differentiation of adult resident cardiac progenitor cells, dedifferentiation and proliferation of adult cardiac muscle cells, and/or transdifferentiation of fibroblast cells into cardiac muscle cells.

In specific aspects, a polynucleotide, protein, peptide, or mixture thereof is targeted to a particular tissue of interest, including a muscle tissue, such as cardiac tissue, for example. The targeting may or may not include an ultrasound targeted microbubble destruction (UTMD) system for delivery of the polynucleotide or protein or peptide to the tissue of interest, including liver tissue (such as for ultimate cardiac location following movement into the circulation from the liver) or cardiac tissue, for example. In particular cases, the polynucleotide, protein, peptide, or mixture thereof localizes inside cells in the targeted tissue.

Aspects of the disclosure concern methods of treating a cardiac-related medical condition in an individual, comprising the step of providing an effective amount of an agonist of the rodent Paired immunoglobulin-like receptor B (PirB) receptor or human Leukocyte immunoglobulin-like receptor subfamily B member 2 (LilrB2) receptor to the individual. In specific aspects, the agonist is *ANGPTL1, ANGPTL2, ANGPTL3, ANGPTL4, ANGPTL5, ANGPTL6, ANGPTL7, ANGPTL8,* or a combination thereof.

Aspects of the disclosure include *ANGPTL1, ANGPTL2, ANGPTL3, ANGPTL4, ANGPTL5, ANGPTL6, ANGPTL7,* or *ANGPTL8* polynucleotides utilized for a therapeutic purpose, including for therapy for at least one cardiac-related medical condition. The polynucleotide may encompass part or all of *ANGPTL1, ANGPTL2, ANGPTL3, ANGPTL4, ANGPTL5, ANGPTL6, ANGPTL7,* or *ANGPTL8,* respectively, for example. In certain cases, the polynucleotide is or is on an expression vector.

Aspects of the disclosure include delivery of one or more polynucleotides that stimulate regeneration of cells (such as muscle cells, including cardiomyocytes) and/or tissue (including cardiac tissue). Particular aspects result in reversal (or treatment of at least one symptom) of one or more cardiac-related medical conditions. Certain aspects result in improvement of at least one symptom of a cardiac-related medical condition. In exemplary instances, the cardiac-related medical condition is heart failure. The heart failure may be the result of one or more causes, including heart failure following exposure to one or more drugs, including chemotherapy drugs, such as Adriamycin, for example.

Aspects of the disclosure include at least one of the following: indirect or direct targeted delivery of *ANGPTL8* polynucleotide viral or plasmid vectors to the heart and/or indirect or direct targeted delivery of therapeutic ANGPTL8 peptides or polypeptides to heart.

Particular but exemplary indications of aspects of the disclosure include at least applications for 1) congestive heart failure; 2) prevention from ventricular remodeling or aneurysm of myocardial infarction; and/or 3) cardiomyopathy. Other indications may also include coronary artery disease, ischemic heart disease, valvular heart disease, arrhythmias, *etc.* In specific aspects, methods and compositions of the disclosure provide cardiomyocyte regeneration that is sufficient to reverse established cardiomyopathy, reverse congestive heart failure, prevent ventricular remodeling or prevent aneurysm of myocardial infarction.

Microbubbles may be employed in particular delivery methods of ANGPTL8, in specific aspects. The microbubbles may comprise albumin, polymer shell, phospholipid, or a graphite shell, and the microbubbles may further comprise a gas, such as perfluoropropane, air, sulfur hexafluoride, perfluorobutane, perfluoropentane, and nitrogen.

The cells targeted in methods of the disclosure may be in an individual that has a cardiac-related medical condition, such as one selected from the group consisting of cardiac disease, cardiomyopathy, cardiotoxicity, congestive heart failure, myocardial infarction, cardiac ischemia, pericarditis, cardiac systolic dysfunction, and arrhythmia. In cases of cardiomyopathy, the condition may be induced by a drug, such as a chemotherapy drug (like Adriamycin) or a monoclonal antibody. Examples of drugs include those selected from the group consisting of anthracyclines; taxanes; fluoropyrimidine; cyclophosphamide; bevacizumab; trastuzumab; lapatinib; sorafenib; and sunitinib. The cardiomyopathy may be ischemic or non-ischemic cardiomyopathy. The cardiomyopathy may be caused by long-term high blood pressure, heart valve problems, heart tissue damage from a previous heart attack, chronic rapid heart rate, metabolic disorders, nutritional deficiencies, pregnancy, alcohol abuse, drug abuse, chemotherapy drugs, viral infection, hemochromatosis, genetic condition, elevated cholesterol levels, or a combination thereof.

In particular aspects, an individual is provided with an additional cardiac disease therapy, such as an additional cardiomyopathy therapy.

Aspects of the disclosure concern methods of treating a cardiac-related medical condition in an individual, comprising the step of providing an effective amount of an antagonist of the PirB receptor or LilrB2 receptor to the individual.

In one aspect, there is a method of treating a cardiac-related medical condition in an individual, comprising the steps of providing an effective amount of one or more agonists of the PirB receptor or LilrB2 receptor to the individual, and the agonist of the PirB receptor or LilrB2 receptor may be ANGPTL1, ANGPTL2, ANGPTL3, ANGPTL4, ANGPTL5, ANGPTL6, ANGPTL7, ANGPTL8, or a combination thereof. The cardiac-related medical condition may be selected from the group consisting of cardiac disease, cardiomyopathy, cardiotoxicity, congestive heart failure, myocardial infarction, cardiac ischemia, pericarditis, cardiac systolic dysfunction, and arrhythmia, in certain cases. The ANGPTL8 (or another one or more ANGPTLs) is provided to the individual as an ANGPTL8 polynucleotide or as an ANGPTL8 polypeptide, respectively. The ANGPTL8 polynucleotide or the ANGPTL8 polypeptide may be provided in a carrier, such as a liposome, nanoparticle, or a lipid-stabilized microbubble, for example.

In some aspects, when the ANGPTL8 polynucleotide(or another one or more ANGPTLs) or the ANGPTL8 polypeptide (or another one or more ANGPTLs) is provided in lipid-stabilized microbubbles, upon delivery to the individual the microbubbles are exposed to ultrasound conditions sufficient to deliver the ANGPTL8 polynucleotide or the ANGPTL8 polypeptide into the cells of the individual. The microbubbles may further comprise albumin, polymer shell, or a graphite shell. The microbubbles may further comprise a gas, such as one selected from the group consisting of perfluoropropane, air, sulfur hexafluoride, perfluorobutane, perfluoropentane, and nitrogen. In some cases, the ANGPTL8 is provided to the individual as an ANGPTL8 polynucleotide and the ANGPTL8 polynucleotide is comprised in a vector, such as one selected from the group consisting of a retroviral vector, lentiviral vector, adenoviral vector, adeno-associated vector, plasmid, or synthetic linear DNA strand. The plasmid may be a piggybac transposon gene delivery plasmid. The vector may be CRISPR-Cas9 gene delivery plasmids for long-term gene expression. In specific embodiments, the expression of the ANGPTL8 polynucleotide is regulated by CMV or a tissue-specific promoter, such as a cardiac muscle-specific promoter.

In situations wherein an individual has cardiomyopathy, the cardiomyopathy may be induced by a drug, such as a chemotherapy drug, including adriamycin. In some cases, the drug is a monoclonal antibody or a drug selected from the group consisting of anthracyclines; taxanes; fluoropyrimidine; cyclophosphamide; bevacizumab; trastuzumab; lapatinib; sorafenib; and sunitinib. In some cases, the cardiomyopathy is ischemic or non-ischemic cardiomyopathy. In certain aspects, the cardiomyopathy is caused by long-term high blood pressure, heart valve problems, heart tissue damage from a previous heart attack, chronic rapid heart rate, metabolic disorders, nutritional deficiencies, pregnancy, alcohol abuse, drug abuse, chemotherapy drugs, viral infection, hemochromatosis, genetic condition, or a combination thereof. In particular aspects, the individual is provided with an additional cardiac-related medical condition therapy, including an additional cardiomyopathy therapy.

In specific aspects, a providing step comprises injection, intravenous perfusion, intra-coronary artery myocardium perfusion, intra-artery organ perfusion by catheter, or coronary sinus perfusion catheter.

In aspects wherein microbubbles are employed, the microbubbles may be delivered to the liver, heart, brain, pancreas, or a combination thereof.

In certain aspects, there is a kit, housed in a suitable container, comprising a polynucleotide encoding at least part of ANGPTL8 and/or primers suitable to amplify at least part of an ANGPTL8 polynucleotide sequence and/or an ANGPTL8 peptide or polypeptide.

In specific aspects, the kit further comprises one or more reagents suitable for generating liposomes. The kit may further comprise an additional therapeutic compound, such as a cardiac-related medical condition therapy and/or device to provide such a therapy.

In one aspect, there is a method of treating a cardiac-related medical condition in an individual, comprising the steps of providing an effective amount of one or more antagonists of the PirB receptor or LilrB2 receptor to the individual.

### BRIEF DESCRIPTION OF THE DRAWINGS

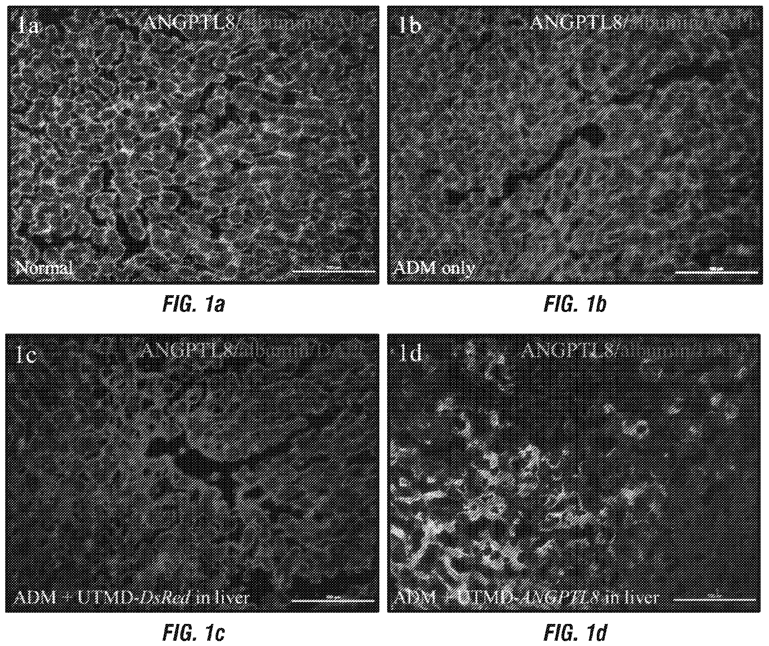

FIG. 1A-1M - UTMD delivered ANGPTL8 gene to liver. (a-d) triple staining with ANGPTL8/Albumin/DAPI for normal rat liver (a), liver of ADM only rat (b), ADM plus UTMD-pXL-BASII-CI-DsRed/hyPB targeted to rat liver (c), ADM plus UTMD-pXL-BASII-CI-*ANGPTL8*/pCI-*hyPB* targeted to liver (d). Scale bar is 100 µm. **(e)** a graphic of fasting plasma human *ANGPTL8* levels. In normal, ADM control and ADM plus UTMD-*DsRed* groups, human *ANGPTL8* was not detectable. Values are presented as mean ± SEM. *n* = 6 per group; ^{∗∗}P<0.001 vs control groups. **(f)** a graphic of RT-qPCR for human *ANGPTL8* mRNA, housekeeping gene is *actin* gene. Values are presented as mean ± SEM. *n* = 6 per group; ^{∗∗}P<0.001 vs control groups. **(g)** Schematic indicating time-course experiment. **(h-k)** Results of echocardiographic measurement of cardiac structure and function. M-mode images at 2D parasternal short axis of the left ventricle, **(h)** normal rat heart, **(i)** ADM control rat heart, **(j)** ADM plus UTMD-DsRed rat heart, **(k)** ADM plus UTMD-ANGPTL8 rat heart. **(l)** a graphic of fractional shortening, **(m)** a graphic of LV mass, Values are presented as mean±SEM. *n* = 6 per group; ^{∗∗}P<0.001 vs after ADM.

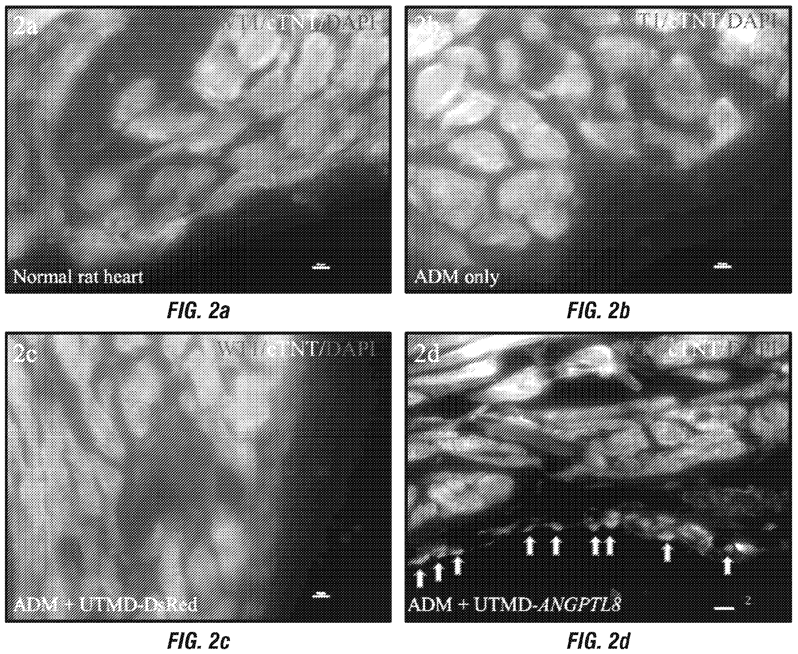

FIG. 2A-2H - ANGPTL8 induced the activation of WT1-positive progenitor cells in epicardial layer cells after ADM plus UTMD-ANGPTL8 gene delivery. Triple staining with WT1/cTNT/DAPI, **(a)** normal rat heart, **(b)** ADM control rat heart, **(c)** ADM plus UTMD-DsRed, **(d)** ADM plus UTMD-ANGPTL8. scale bar is 10 µm, WT1-positive progenitor cells also are positive of stem cells markers (OCT4, Nanog, Sox2) after ADM plus UTMD-ANGPTL8 gene delivery. **(e)** Triple staining with OCT4/cTNT/DAPI in ADM plus UTMD-ANGPTL8, **(f)** Nanog/cTNT/DAPI in ADM plus UTMD-ANGPTL8, **(g)** SOX2/cTNT/DAPI in ADM plus UTMD-ANGPTL8, scale bar is 10 µm, **(h)** a graphic of RT-qPCR for WT1, OCT4, Nanog and SOX2 mRNA, housekeeping gene is *actin* gene. Values are presented as mean ± SEM. *n* = 6 per group; ^{∗∗}P<0.001 vs control groups.

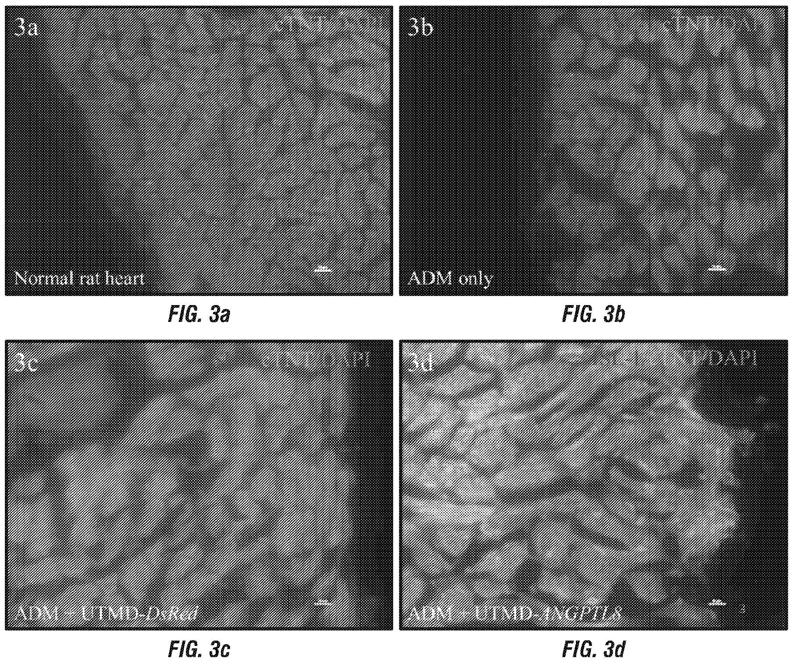

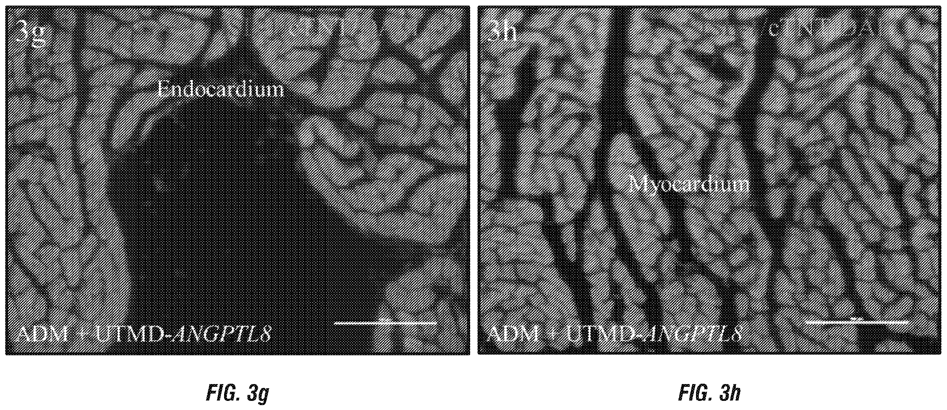

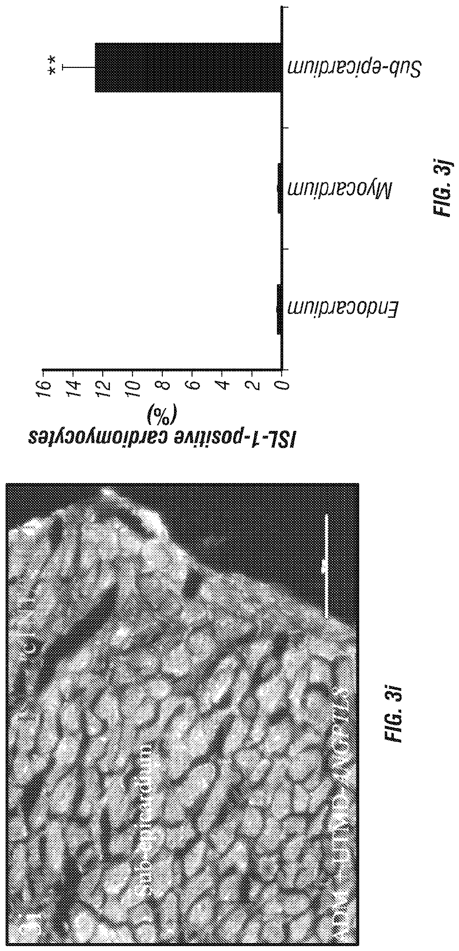

FIG. 3A-3J - The activation of ISL-1 (an early cardiac muscle differentiation marker) in epicardium and sub-epicardial layer after ADM plus UTMD-ANGPTL8. **(a)** normal rat heart, **(b)** ADM control rat heart, **(c)** ADM plus UTMD-DsRed, **(d)** ADM plus UTMD-ANGPTL8. scale bar is 10 µm. **(e)** a graphic of qRT-PCR for ISL-1 mRNA, housekeeping gene is *actin* gene. Values are presented as mean ± SEM. *n =* 6 per group; ^{∗∗}P<0.001 vs control groups. **(f)** a graphic for percentage of ISL-1-positive cardiomyctes cells. Values are presented as mean ± SEM. n=6 per group; ^{∗∗}p<0.001 vs control groups. The distribution of ISL-1-positive adult cardiac progenitor cells after ADM plus UTMD-ANGPTL8. **(g)** Endocardium, **(h)** Myocardium, **(i)** Sub-epicardium, at hearts of ADM plus UTMD-ANGPTL8 group. scale bar is 100 µm. **(j)** a graphic for percentage of ISL-1-positive cardiomyoctes cells. Values are presented as mean ± SEM. n=6 per group; ^{∗∗}*p*<0.001 vs endocardium and myocardium.
FIG. 4A-4F - A time-course experiment show that ANGPTL8 induced epicardial layer cells **(a)** into ISL-1-positive cardiac progenitor cells **(b)** and formed niches of cardiac progenitor cells in epicardium **(c)** proliferated and migrated into myocardium layer (d-e) and differentiate into new cardiac muscle cells **(f)**. Scale bar is 10 µm.

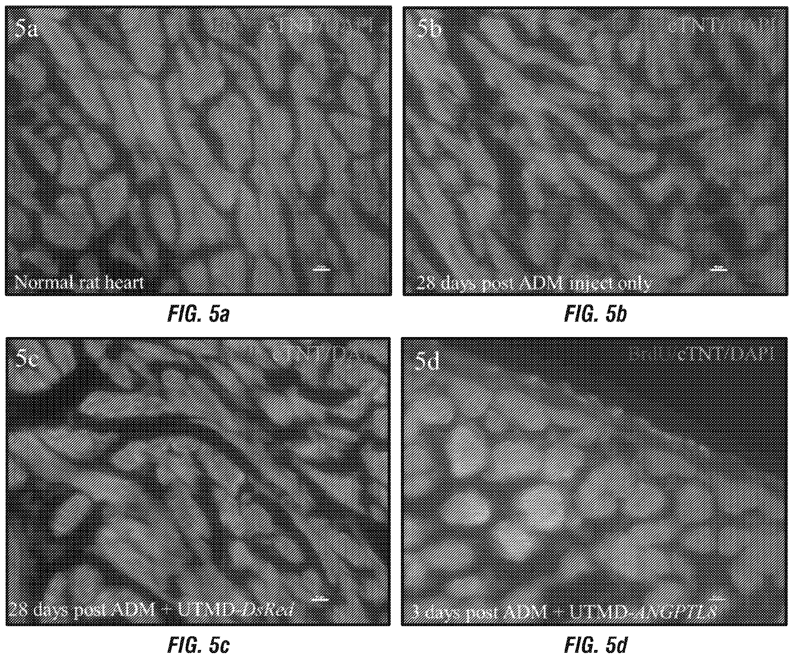

FIG. 5A-5G - BrdU staining shown some regenerating cardiac muscle cells are in proliferation. **(a)** normal rat heart, **(b)** 28 days post ADM inject, **(c)** 28 days post ADM plus UTMD-DsRed, **(d)** 3 days post ADM plus UTMD-ANGPTL8. **(e)** 14 days post ADM plus UTMD-ANGPTL8. **(f)** 28 days post ADM plus UTMD-ANGPTL8. scale bar is 10 µm, **(g)** Percentage of BrdU-positive cardiomyctes cells. Values are presented as mean ± SEM. *n=6* per group; ^{∗∗}p<0.001 vs control groups.

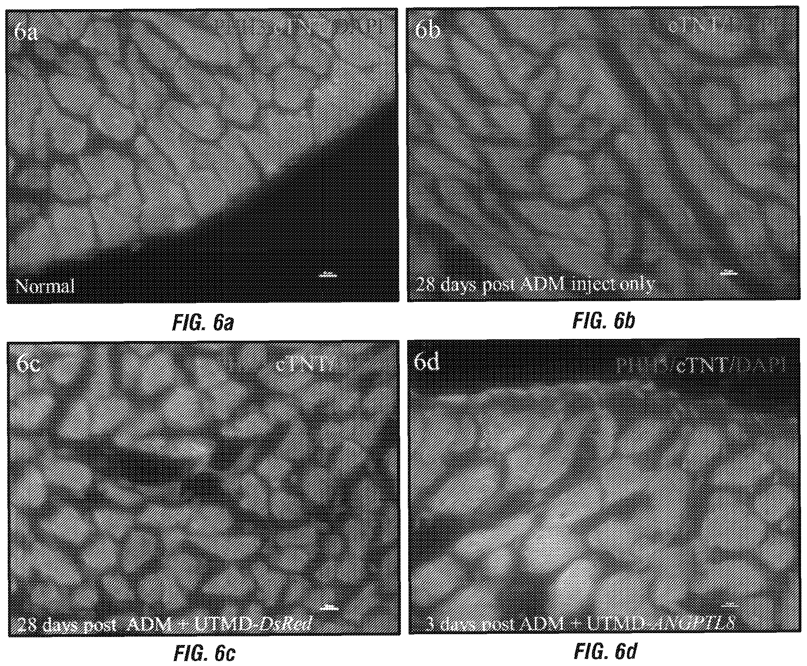

FIG. 6A-6G - Phospho-hi stone H3(PHH3) staining shown some regenerating cardiac muscle cells are in proliferation. **(a)** normal rat heart, **(b)** 28 days post ADM inject, **(c)** 28 days post ADM plus UTMD-DsRed, **(d)** 3 days post ADM plus UTMD-ANGPTL8. **(e)** 14 days post ADM plus UTMD-ANGPTL8. **(f)** 28 days post ADM plus UTMD-ANGPTL8. scale bar is 10 µm, **(g)** Percentage of PHH3-positive cardiomyctes cells. Values are presented as mean ± SEM. n=6 per group; ^{∗∗}p<0.001 vs control groups.

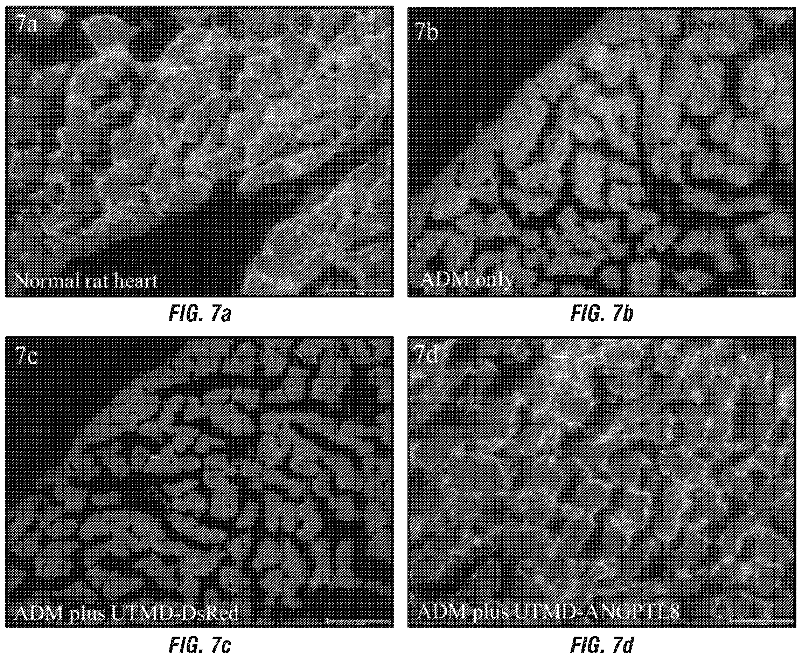

FIG. 7A-7H - Upper panel **(a-d)**: Paired immunoglobulin-like receptor B (PirB) distributed on membrane of cardiac muscle cells of adult rat. **(a)** Normal rat heart; **(b)** ADM only control heart; **(c)** ADM plus UTMD-DsRed heart; **(d)** ADM plus UTMD-ANGPTL8 heart; scale bar is 25 µm. Red color for PirB signal; green for cardiac troponin T signal; blue for nucleus identity. Down panel **(e-h)** are the culture atria muscle cell line (HL-1) treated with 0.5 µM ADM plus 2ng of ANGPTL8 peptide. Scale bar is 25 µm.

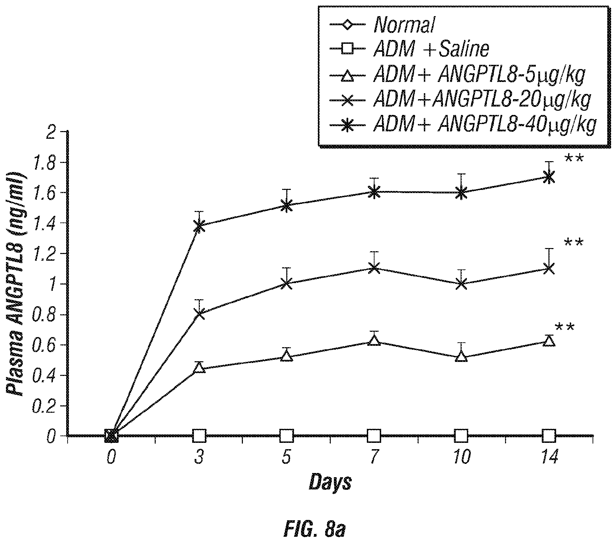

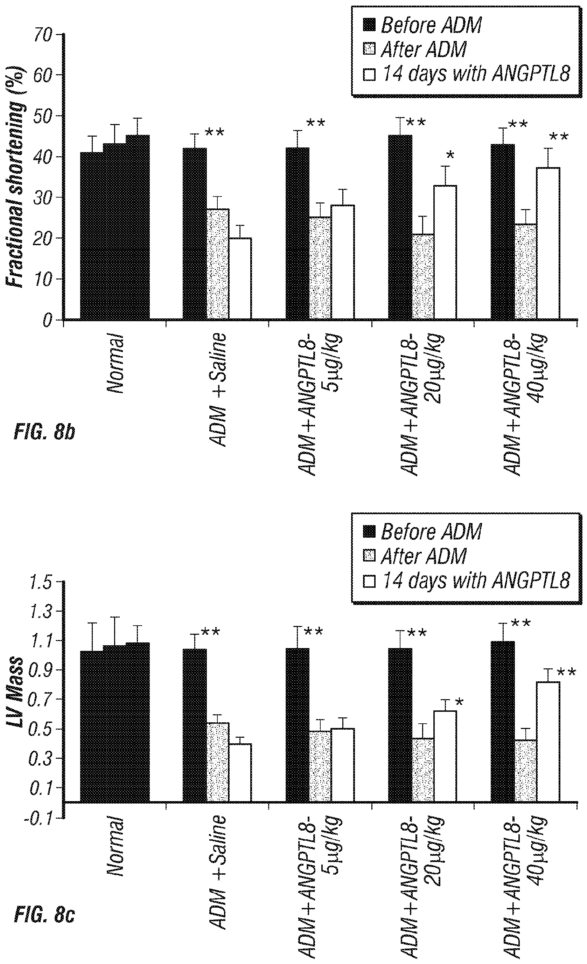

FIG. 8A-8C -ANGPTL8 protein intramuscular injection reversed established ADM cardiomyopathy. **(a)** a graphic of fasting plasma human *ANGPTL8* levels. In normal and ADM + saline inject, human *ANGPTL8* was not detectable. Values are presented as mean ± SEM. *n* =3 per group; ^{∗∗}P<0.001 vs control groups. ANGPTL8 protein injection at doses of 5µg-20µg-40µg/kg/day for 14 days; **(b)** a graphic of fractional shortening, **(c)** a graphic of LV mass, Values are presented as mean±SEM. *n* = 3 per group; ^{∗∗}P<0.001 and ^{∗}<0.05 vs after ADM.

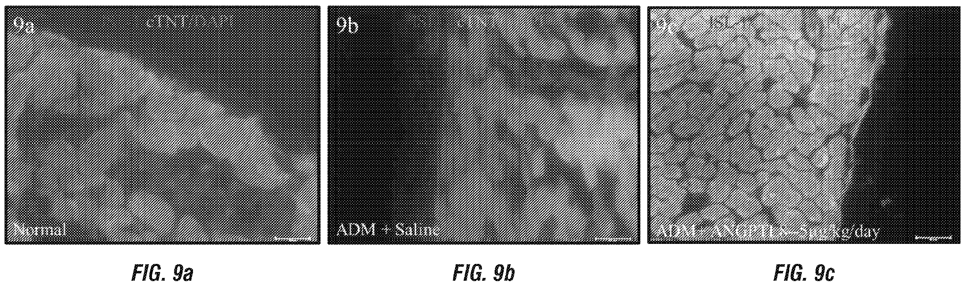

FIG. 9A-9E -ANGPTL8 protein therapy inducing the activation of ISL-1 (an early cardiac muscle differentiation marker) in epicardium and sub-epicardial layer. **(a)** normal rat heart, **(b)** ADM + Saline injection, **(c)** ADM plus ANGTPL8 protein 5 µg/kg/day for 14 days, **(d)** ADM plus ANGTPL8 protein 20 µg/kg/day for 14 days, **(e)** ADM plus ANGTPL8 protein 40 µg/kg/day for 14 days. scale bar is 25 µm.

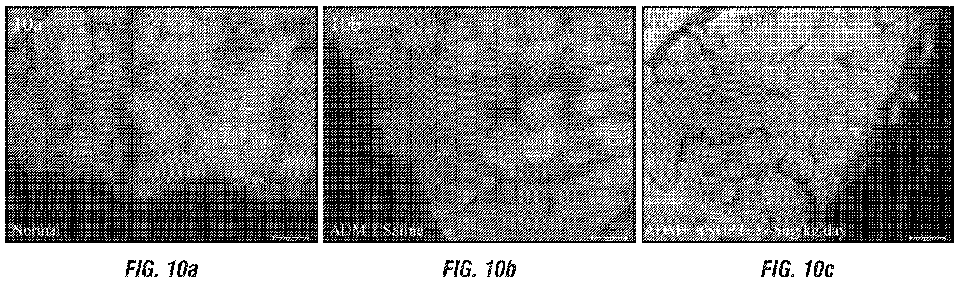

FIG. 10A-10E-Phospho-histone H3(PHH3) staining shown some epicardial cells and sub-epicardial cardiac muscle cells are in proliferation after ANGPTL8 protein therapy. **(a)** normal rat heart, **(b)** ADM + Saline injection, **(c)** ADM plus ANGTPL8 protein 5 µg/kg/day for 14 days, **(d)** ADM plus ANGTPL8 protein 20 µg/kg/day for 14 days, **(e)** ADM plus ANGTPL8 protein 40 µg/kg/day for 14 days. scale bar is 25 µm.

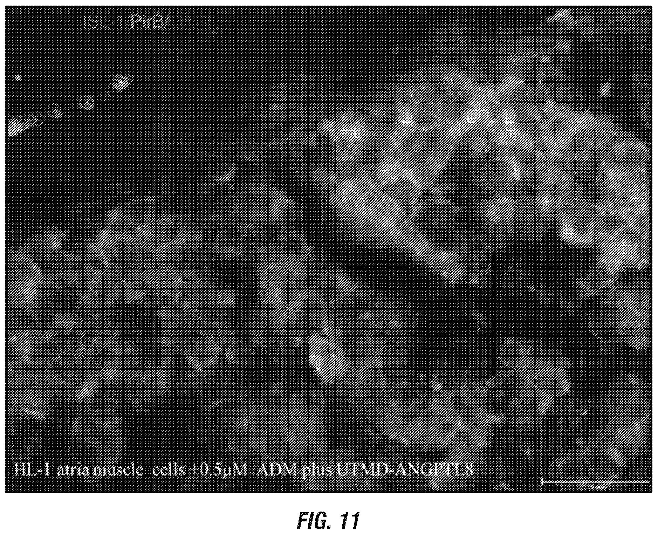

FIG. 11 - PirB distributed in membrane of ISL-1 positive cardiac progenitor cells. Scale bar is 25 µm.

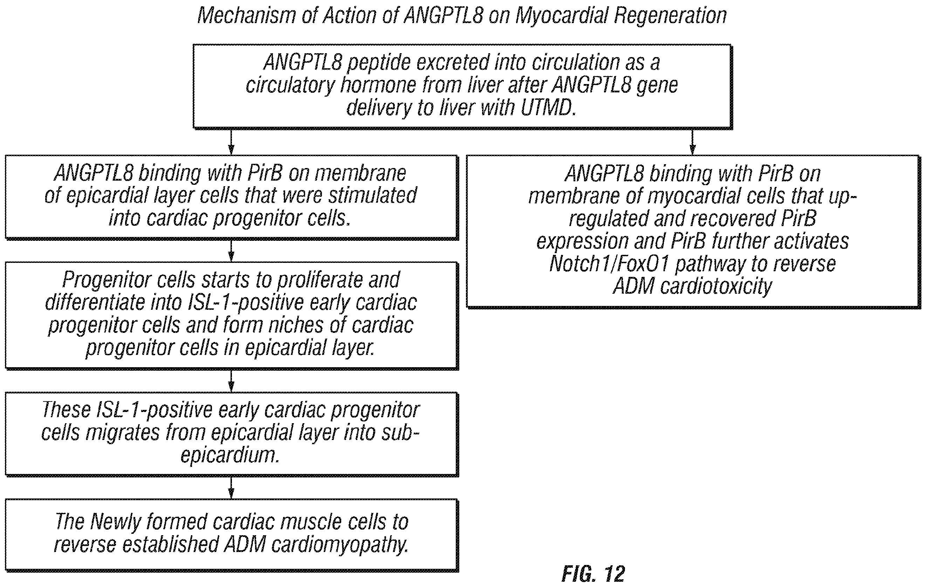

FIG. 12 -Schematic indicating mechanism of action of ANGPTL8 on myocardial regeneration.

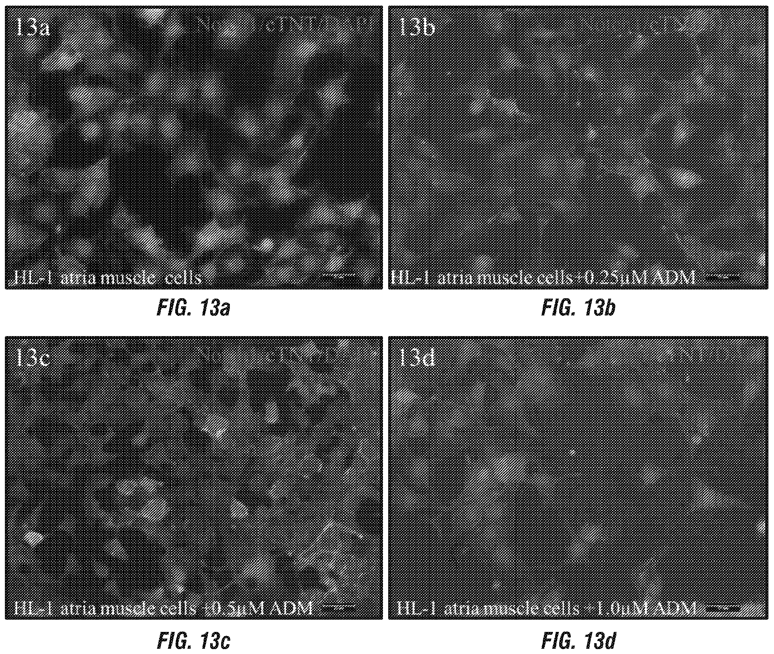

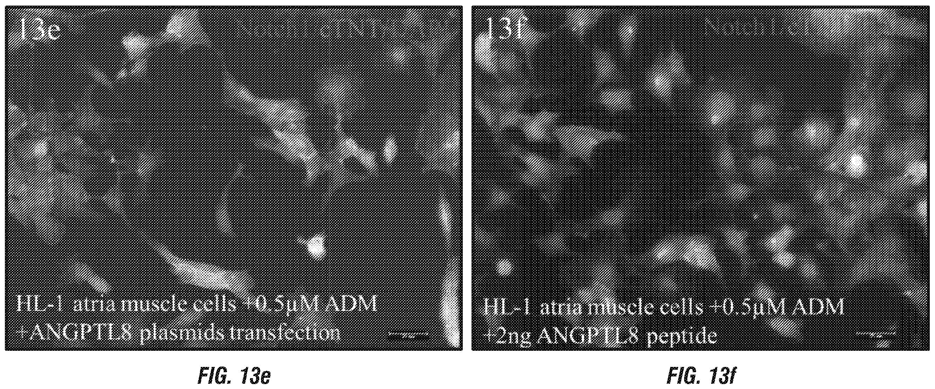

FIG. 13A-13F - ANGPTL8 removed the inhibition of Notch1 expression induced by ADM in HL-1 atria muscle cells in vitro. **(a)** HL-1 atria muscle cells; **(b)** HL-1 atria muscle cells plus 0.25µM ADM; **(c)** HL-1 atria muscle cells plus 0.5µM ADM; **(d)** HL-1 atria muscle cells plus 1.0 µM ADM; **(e)** HL-1 atria muscle cells plus 0.5µM ADM+ ANGPTL8 plasmids transfection; **(f)** HL-1 atria muscle cells plus 0.5µM ADM + 2 ng of ANGPTL8 peptide; Scale bar is 25 µm.0

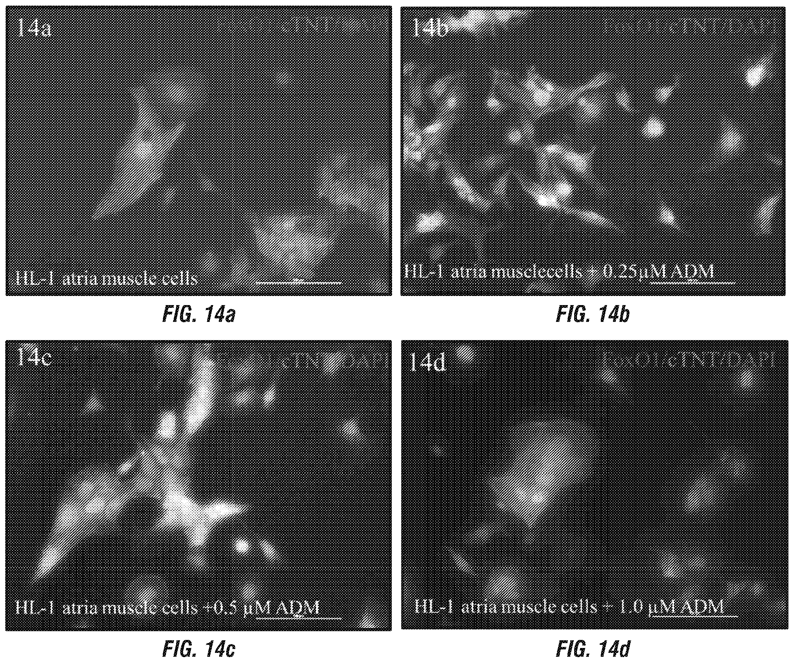

FIG. 14A-14F - ANGPTL8 reversed ADM induced FoxO1 nuclear expression of HL-1 atria muscle cells in vitro. **(a)** HL-1 atria muscle cells; **(b)** HL-1 atria muscle cells plus 0.25µM ADM; **(c)** HL-1 atria muscle cells plus 0.5µM ADM; **(d)** HL-1 atria muscle cells plus 1.0 µM ADM; **(e)** HL-1 atria muscle cells plus 0.5µM ADM+ ANGPTL8 plasmids transfection; **(f)** HL-1 atria muscle cells plus 0.5µM ADM + 2 ng of ANGPTL8 peptide; Scale bar is 25 µm.0.

### DETAILED DESCRIPTION OF THE INVENTION

The scope of the present application is not intended to be limited to the particular embodiments of the composition of matter, methods and steps described in the specification.

As used herein the specification, "a" or "an" may mean one or more. As used herein in the claim(s), when used in conjunction with the word "comprising", the words "a" or "an" may mean one or more than one. As used herein "another" may mean at least a second or more. In specific embodiments, aspects of the invention may "consist essentially of' or "consist of' one or more sequences of the invention, for example. Some embodiments of the invention may consist of or consist essentially of one or more elements, method steps, and/or methods of the invention. It is contemplated that any method or composition described herein can be implemented with respect to any other method or composition described herein.

The term "cardiac-related medical condition" as used herein refers to any medical condition that affects the heart, including that affects heart function.

### I. General Disclosure

The present disclosure provides methods and/or compositions for treatment and/or prevention of at least one cardiac-related medical condition. Such methods and compositions employ one or more agonists of PirB or LilrB2 receptor. In some cases, such methods and compositions employ at least part of ANGPTL8 polynucleotide and/or polypeptide and/or peptide, although ANGPTL1, ANGPTL2, ANGPTL3, ANGPTL4, ANGPTL5, ANGPTL6, ANGPTL7, ANGPTL8 polynucleotide and/or polypeptide and/or peptide may be used in addition or as alternative(s). The compositions may be provided to an individual with a cardiac-related medical condition with no carrier or the compositions may be provided with a carrier, and the carrier may be of any kind. In specific aspects, one or more ANGPTL8 (as an example) polynucleotides and/or polypeptides and/or peptides are delivered to an individual with a cardiac-related medical condition in an ultrasound-targeted microbubble destruction (UTMD) system. In certain cases the individual has cardiomyopathy, such as drug-induced cardiomyopathy.

The effects of *ANGPTL8* on myocardial regeneration in heart failure have not been reported. In this disclosure, UTMD is employed to deliver an *ANGPTL8* polynucleotide to the liver of rats with established cardiomyopathy induced by adriamycin. *ANGPTL8* can reverse established cardiomyopathy. The approach of gene therapy by targeting the delivery of non-viral DNA to the organs using ultrasound-targeted microbubble destruction (UTMD) has been demonstrated [Chen, *et al.,* 2006; Chen, *et al.,* 2008; Chai, *et al.,* 2009; Chen, *et al.,* 2010; Shimoda, *et al.,* 2010; Chen, *et al.,* 2012a; Chen *et al.,* 2012b].

In specific cases, ANGPTL8 is provided to an individual using the UTMD system, and the ANGPTL8-comprising microbubbles may be delivered to any organ in the individual. UTMD-ANGPTL8 gene delivery may be provided to any organs (although in specific aspects, not lung or bone) under ultrasound targeted microbubble destruction. ANGPTL8 is a circulatory hormone released into blood from original organs (liver or adipose tissue, for example) and has wide action on bone marrow stem cells, neurons cells, pancreatic cells, vascular endothelial cells, or plus cardiac muscle cells. In specific aspects, delivery of ANGPTL8-comprising microbubbles is targeted to the liver, heart, pancreas, or brain, for example. In particular aspects, release of ANGPTL8 in the liver results in entry into the circulation of an individual with a cardiac-related medical condition, thereby allowing it to act within the heart. Once in the heart, ANGPTL8 stimulates epicardial layer cells to become progenitor or stem-like cells. The progenitor or stem-like cells proliferate and differentiated into cardiac progenitor cells (such as ISL-1 positive cardiac progenitor cells or cardiac stem cells markers such as Wt1+, c-kit+, NKX2.5+, Tbx18+, SCA1+ or Gata4+) and form niches of progenitor cells in epicardial layer. Following this, the cardiac progenitor cells migrate from the epicardial layer into the sub-epicardium, facilitating reversal of the cardiac-related medical condition.

In alternative aspects, an effective amount of an antagonist of PirB or LilrB2 receptor is provided to an individual for the treatment of a cardiac-related medical condition.

### II. Cardiac-Related Medical Conditions and Treatment and/or Prevention Thereof

The cardiac-related medical condition may be selected from the group consisting of cardiac disease, cardiovascular disease, heart disease, cardiomyopathy, cardiotoxicity, myocardial infarction, cardiac ischemic disease, arrhythmias, coronary artery disease, and a combination thereof.

Particular types of cardiovascular disease may be treated or prevented, such as coronary artery disease (also known as coronary heart disease and ischaemic heart disease); cardiomyopathy (diseases of cardiac muscle); hypertensive heart disease; heart failure; cor pulmonale; cardiac dysrhythmias; inflammatory heart disease; endocarditis; inflammatory cardiomegaly; myocarditis; valvular heart disease; cerebrovascular disease; peripheral arterial disease; congenital heart disease; and rheumatic heart disease.

In particular aspects of the disclosure, cardiomyopathy is the cardiac-related medical condition. The cardiac-related medical condition (including, for example, cardiomyopathy) may be caused by one or more of a variety of characteristics, including, for example, long-term high blood pressure; heart valve problems; heart tissue damage (such as from a previous heart attack); chronic rapid heart rate; metabolic disorders, such as thyroid disease or diabetes; nutritional deficiencies of essential vitamins or minerals, such as thiamin (vitamin B-1), selenium, calcium and/or magnesium; pregnancy; alcohol abuse; drug abuse, including of narcotics or prescription drugs, such as cocaine or antidepressant medications, such as tricyclic antidepressants; use of some chemotherapy drugs to treat cancer (including Adriamycin); certain viral infections; hemochromatosis and/or an unknown cause or undetected cause. The cardiac-related medical condition may be directly or indirectly caused by cancer therapeutics, both small molecule drugs and biologics, that are associated with cardiotoxicity, for example. Examples include anthracyclines; taxanes; fluoropyrimidine; cyclophosphamide; bevacizumab; trastuzomab; lapatinib; sorafenib; and sunitinib. The drug may be an immunogenic composition, such as a monoclonal antibody, such as Herceptin, for example.

In some cases, methods and compositions of the present disclosure are employed for prevention of one or more cardiac-related medical conditions or delay of onset of one or more cardiac-related medical conditions or reduction of extent of one or more symptoms of one or more cardiac-related medical conditions. In particular cases, such prevention, delay or onset, or reduction of extent of one or more symptoms, occurs in an individual that is at risk for a cardiac-related medical condition. Exemplary risk factors include one or more of the following: age, gender (male, although it occurs in females), high blood pressure, high serum cholesterol levels, tobacco smoking, excessive alcohol consumption, sugar consumption, family history, obesity, lack of physical activity, psychosocial factors, diabetes mellitus, overweight, genetic predisposition, and/or exposure to air pollution.

### III. ANGPTL8 Compositions

Particular aspects of the disclosure concern agonists of PirB or LilrB2 receptor, and in specific cases the agonist is ANGPTL8. Certain embodiments of the present disclosure concern an ANGPTL8 polynucleotide, polypeptide, or peptide. In certain aspects, the ANGPTL8 polynucleotide, polypeptide, or peptide is a wild-type form or a mutant form. In cases when the ANGPTL8 polynucleotide, polypeptide, or peptide is a mutant form, the mutant is still functionally active to allow treatment of a cardiac medical condition. An exemplary human ANGPTL8 polynucleotide is at the National Center for Biotechnology Information's GenBank^{®} database at Accession Number NM_018687 (SEQ ID NO:1). An exemplary human ANGPTL8 polypeptide is at the National Center for Biotechnology Information's GenBank^{®} database at Accession Number NP_061157 (SEQ ID NO:2).

A functional fragment of ANGPTL8 may be utilized instead of the entire ANGPTL8 polynucleotide or entire ANGPTL8 polypeptide. A functional fragment of ANGPTL8 is one that is sufficient to allow regeneration of cells upon exposure to the fragment or that is sufficient to provide amelioration of at least one symptom of a cardiac-related medical condition. The functional fragment of ANGPTL8 nucleic acid may encode (or the peptide comprise) at least 197, 195, 190, 185, 180, 175, 170, 165, 160, 155, 150, 145, 140, 135, 130, 125, 120, 115, 110, 105, 100, 95, 90, 85, 80, 75, 70, 65, 60, 55, 50, 45, 40, 35, 30, 25, 15, or 10 amino acids of SEQ ID NO:2. The functional fragment of ANGPTL8 nucleic acid may encode a polypeptide or peptide no more than 197, 195, 190, 185, 180, 175, 170, 165, 160, 155, 150, 145, 140, 135, 130, 125, 120, 115, 110, 105, 100, 95, 90, 85, 80, 75, 70, 65, 60, 55, 50, 45, 40, 35, 30, 25, 15, or 10 amino acids of SEQ ID NO:2. The functional ANGPTL8 fragment may be 100%, 99%, 98%, 97%, 96%, 95%, 94%, 93%, 92%, 91%, 90%, 85%, 80%, 75%, or 70% identity to SEQ ID NO:2. An ANGPTL8 polynucleotide may be 100%, 99%, 98%, 97%, 96%, 95%, 94%, 93%, 92%, 91%, 90%, 85%, 80%, 75%, or 70% identity to SEQ ID NO: 1.

The term "nucleic acid" or "polynucleotide" is well known in the art. A " polynucleotide " as used herein will generally refer to a molecule *(i.e.,* a strand) of DNA, RNA or a derivative or analog thereof, comprising a nucleobase. A nucleobase includes, for example, a naturally occurring purine or pyrimidine base found in DNA (*e.g.,* an adenine "A," a guanine "G," a thymine "T" or a cytosine "C") or RNA (*e.g.,* an A, a G, an uracil "U" or a C). The term "nucleic acid" encompass the terms "oligonucleotide" and "polynucleotide," each as a subgenus of the term "nucleic acid." The term "oligonucleotide" refers to a molecule of between about 3 and about 100 nucleobases in length. The term "polynucleotide" refers to at least one molecule of greater than about 100 nucleobases in length.

These definitions generally refer to a single-stranded molecule, but in specific embodiments will also encompass an additional strand that is partially, substantially or fully complementary to the single-stranded molecule. Thus, a nucleic acid may encompass a double-stranded molecule or a triple-stranded molecule that comprises one or more complementary strand(s) or "complement(s)" of a particular sequence comprising a molecule. As used herein, a single stranded nucleic acid may be denoted by the prefix "ss," a double stranded nucleic acid by the prefix "ds," and a triple stranded nucleic acid by the prefix "ts."

### A. Nucleobases

As used herein a "nucleobase" refers to a heterocyclic base, such as for example a naturally occurring nucleobase *(i.e.,* an A, T, G, C or U) found in at least one naturally occurring nucleic acid *(i.e.,* DNA and RNA), and naturally or non-naturally occurring derivative(s) and analogs of such a nucleobase. A nucleobase generally can form one or more hydrogen bonds ("anneal" or "hybridize") with at least one naturally occurring nucleobase in manner that may substitute for naturally occurring nucleobase pairing *(e.g.,* the hydrogen bonding between A and T, G and C, and A and U).

"Purine" and/or "pyrimidine" nucleobase(s) encompass naturally occurring purine and/or pyrimidine nucleobases and also derivative(s) and analog(s) thereof, including but not limited to, those a purine or pyrimidine substituted by one or more of an alkyl, caboxyalkyl, amino, hydroxyl, halogen (*i.e.,* fluoro, chloro, bromo, or iodo), thiol or alkylthiol moiety. Preferred alkyl (*e.g.,* alkyl, caboxyalkyl, *etc.*) moeities comprise of from about 1, about 2, about 3, about 4, about 5, to about 6 carbon atoms. Other non-limiting examples of a purine or pyrimidine include a deazapurine, a 2,6-diaminopurine, a 5-fluorouracil, a xanthine, a hypoxanthine, a 8-bromoguanine, a 8-chloroguanine, a bromothymine, a 8-aminoguanine, a 8-hydroxyguanine, a 8-methylguanine, a 8-thioguanine, an azaguanine, a 2-aminopurine, a 5-ethylcytosine, a 5-methylcyosine, a 5-bromouracil, a 5-ethyluracil, a 5-iodouracil, a 5-chlorouracil, a 5-propyluracil, a thiouracil, a 2-methyladenine, a methylthioadenine, a N,N-diemethyladenine, an azaadenines, a 8-bromoadenine, a 8-hydroxyadenine, a 6-hydroxyaminopurine, a 6-thiopurine, a 4-(6-aminohexyl/cytosine), and the like.

A nucleobase may be comprised in a nucleoside or nucleotide, using any chemical or natural synthesis method described herein or known to one of ordinary skill in the art.

### B. Nucleosides

As used herein, a "nucleoside" refers to an individual chemical unit comprising a nucleobase covalently attached to a nucleobase linker moiety. A non-limiting example of a "nucleobase linker moiety" is a sugar comprising 5-carbon atoms *(i.e.,* a "5-carbon sugar"), including but not limited to a deoxyribose, a ribose, an arabinose, or a derivative or an analog of a 5-carbon sugar. Non-limiting examples of a derivative or an analog of a 5-carbon sugar include a 2'-fluoro-2'-deoxyribose or a carbocyclic sugar where a carbon is substituted for an oxygen atom in the sugar ring.

Different types of covalent attachment(s) of a nucleobase to a nucleobase linker moiety are known in the art. By way of non-limiting example, a nucleoside comprising a purine *(i.e.,* A or G) or a 7-deazapurine nucleobase typically covalently attaches the 9 position of a purine or a 7-deazapurine to the 1'-position of a 5-carbon sugar. In another non-limiting example, a nucleoside comprising a pyrimidine nucleobase *(i.e.,* C, T or U) typically covalently attaches a 1 position of a pyrimidine to a 1'-position of a 5-carbon sugar (Kornberg and Baker, 1992).

### C. Nucleotides

As used herein, a "nucleotide" refers to a nucleoside further comprising a "backbone moiety". A backbone moiety generally covalently attaches a nucleotide to another molecule comprising a nucleotide, or to another nucleotide to form a nucleic acid. The "backbone moiety" in naturally occurring nucleotides typically comprises a phosphorus moiety, which is covalently attached to a 5-carbon sugar. The attachment of the backbone moiety typically occurs at either the 3'- or 5'-position of the 5-carbon sugar. However, other types of attachments are known in the art, particularly when a nucleotide comprises derivatives or analogs of a naturally occurring 5-carbon sugar or phosphorus moiety.

### D. Nucleic Acid Analogs

A nucleic acid may comprise, or be composed entirely of, a derivative or analog of a nucleobase, a nucleobase linker moiety and/or backbone moiety that may be present in a naturally occurring nucleic acid. As used herein a "derivative" refers to a chemically modified or altered form of a naturally occurring molecule, while the terms "mimic" or "analog" refer to a molecule that may or may not structurally resemble a naturally occurring molecule or moiety, but possesses similar functions. As used herein, a "moiety" generally refers to a smaller chemical or molecular component of a larger chemical or molecular structure. Nucleobase, nucleoside and nucleotide analogs or derivatives are well known in the art, and have been described (see for example, Scheit, 1980).

Additional non-limiting examples of nucleosides, nucleotides or nucleic acids comprising 5-carbon sugar and/or backbone moiety derivatives or analogs, include those in U.S. Patent No. 5,681,947 which describes oligonucleotides comprising purine derivatives that form triple helixes with and/or prevent expression of dsDNA; U.S. Patents 5,652,099 and 5,763,167 which describe nucleic acids incorporating fluorescent analogs of nucleosides found in DNA or RNA, particularly for use as flourescent nucleic acids probes; U.S. Patent 5,614,617 which describes oligonucleotide analogs with substitutions on pyrimidine rings that possess enhanced nuclease stability; U.S. Patents 5,670,663, 5,872,232 and 5,859,221 which describe oligonucleotide analogs with modified 5-carbon sugars *(i.e.,* modified 2'-deoxyfuranosyl moieties) used in nucleic acid detection; U.S. Patent 5,446,137 which describes oligonucleotides comprising at least one 5-carbon sugar moiety substituted at the 4' position with a substituent other than hydrogen that can be used in hybridization assays; U.S. Patent 5,886,165 which describes oligonucleotides with both deoxyribonucleotides with 3'-5' internucleotide linkages and ribonucleotides with 2'-5' internucleotide linkages; U.S. Patent 5,714,606 which describes a modified internucleotide linkage wherein a 3'-position oxygen of the internucleotide linkage is replaced by a carbon to enhance the nuclease resistance of nucleic acids; U.S. Patent 5,672,697 which describes oligonucleotides containing one or more 5' methylene phosphonate internucleotide linkages that enhance nuclease resistance; U.S. Patents 5,466,786 and 5,792,847 which describe the linkage of a substituent moiety which may comprise a drug or label to the 2' carbon of an oligonucleotide to provide enhanced nuclease stability and ability to deliver drugs or detection moieties; U.S. Patent 5,223,618 which describes oligonucleotide analogs with a 2 or 3 carbon backbone linkage attaching the 4' position and 3' position of adjacent 5-carbon sugar moiety to enhanced cellular uptake, resistance to nucleases and hybridization to target RNA; U.S. Patent 5,470,967 which describes oligonucleotides comprising at least one sulfamate or sulfamide internucleotide linkage that are useful as nucleic acid hybridization probe; U.S. Patents 5,378,825, 5,777,092, 5,623,070, 5,610,289 and 5,602,240 which describe oligonucleotides with three or four atom linker moiety replacing phosphodiester backbone moiety used for improved nuclease resistance, cellular uptake and regulating RNA expression; U.S. Patent 5,858,988 which describes hydrophobic carrier agent attached to the 2'-O position of oligonuceotides to enhanced their membrane permeability and stability; U.S. Patent 5,214,136 which describes olignucleotides conjugated to anthraquinone at the 5' terminus that possess enhanced hybridization to DNA or RNA; enhanced stability to nucleases; U.S. Patent 5,700,922 which describes PNA-DNA-PNA chimeras wherein the DNA comprises 2'-deoxy-erythro-pentofuranosyl nucleotides for enhanced nuclease resistance, binding affinity, and ability to activate RNase H; and U.S. Patent 5,708,154 which describes RNA linked to a DNA to form a DNA-RNA hybrid.

### E. Polyether and Peptide Nucleic Acids

In certain aspects, it is contemplated that a nucleic acid comprising a derivative or analog of a nucleoside or nucleotide may be used in the methods and compositions of the disclosure. A non-limiting example is a "polyether nucleic acid", described in U.S. Patent Serial No. 5,908,845. In a polyether nucleic acid, one or more nucleobases are linked to chiral carbon atoms in a polyether backbone.

Another non-limiting example is a "peptide nucleic acid", also known as a "PNA", "peptide-based nucleic acid analog" or "PENAM", described in U.S. Patent Serial Nos. 5,786,461, 5891,625, 5,773,571, 5,766,855, 5,736,336, 5,719,262, 5,714,331, 5,539,082, and WO 92/20702. Peptide nucleic acids generally have enhanced sequence specificity, binding properties, and resistance to enzymatic degradation in comparison to molecules such as DNA and RNA (Egholm *et al.,* 1993; PCT/EP/01219). A peptide nucleic acid generally comprises one or more nucleotides or nucleosides that comprise a nucleobase moiety, a nucleobase linker moiety that is not a 5-carbon sugar, and/or a backbone moiety that is not a phosphate backbone moiety. Examples of nucleobase linker moieties described for PNAs include aza nitrogen atoms, amido and/or ureido tethers (see for example, U.S. Patent No. 5,539,082). Examples of backbone moieties described for PNAs include an aminoethylglycine, polyamide, polyethyl, polythioamide, polysulfinamide or polysulfonamide backbone moiety.

In certain aspects, a nucleic acid analogue such as a peptide nucleic acid may be used to inhibit nucleic acid amplification, such as in PCR, to reduce false positives and discriminate between single base mutants, as described in U.S. Patent Serial No. 5,891,625. Other modifications and uses of nucleic acid analogs are known in the art, and are encompassed herein. In a non-limiting example, U.S. Patent 5,786,461 describes PNAs with amino acid side chains attached to the PNA backbone to enhance solubility of the molecule. In another example, the cellular uptake property of PNAs is increased by attachment of a lipophilic group. U.S. application Ser. No. 117,363 describes several alkylamino moeities used to enhance cellular uptake of a PNA. Another example is described in U.S. Patent Nos. 5,766,855, 5,719,262, 5,714,331 and 5,736,336, which describe PNAs comprising naturally and non-naturally occurring nucleobases and alkylamine side chains that provide improvements in sequence specificity, solubility and/or binding affinity relative to a naturally occurring nucleic acid.

### F. Preparation of Nucleic Acids

A nucleic acid may be made by any technique known to one of ordinary skill in the art, such as for example, chemical synthesis, enzymatic production or biological production. Non-limiting examples of a synthetic nucleic acid *(e.g.,* a synthetic oligonucleotide), include a nucleic acid made by *in vitro* chemical synthesis using phosphotriester, phosphite or phosphoramidite chemistry and solid phase techniques such as described in EP 266,032, or *via* deoxynucleoside H-phosphonate intermediates as described by Froehler *et al.,* 1986 and U.S. Patent Serial No. 5,705,629. In the methods of the present disclosure, one or more oligonucleotide may be used. Various different mechanisms of oligonucleotide synthesis have been disclosed in for example, U.S. Patents. 4,659,774, 4,816,571, 5,141,813, 5,264,566, 4,959,463, 5,428,148, 5,554,744, 5,574,146, 5,602,244.

A non-limiting example of an enzymatically produced nucleic acid include one produced by enzymes in amplification reactions such as PCR^{™} (see for example, U.S.

Patent 4,683,202 and U.S. Patent 4,682,195), or the synthesis of an oligonucleotide described in U.S. Patent No. 5,645,897. A non-limiting example of a biologically produced nucleic acid includes a recombinant nucleic acid produced (*i.e*., replicated) in a living cell, such as a recombinant DNA vector replicated in bacteria (see for example, Sambrook *et al.* 1989).

### G. Purification of Nucleic Acids

A nucleic acid may be purified on polyacrylamide gels, cesium chloride centrifugation gradients, or by any other means known to one of ordinary skill in the art (see for example, Sambrook *et al.,* 1989).

In certain aspect, the present disclosure concerns a nucleic acid that is an isolated nucleic acid. As used herein, the term "isolated nucleic acid" refers to a nucleic acid molecule *(e.g.,* an RNA or DNA molecule) that has been isolated free of, or is otherwise free of, the bulk of the total genomic and transcribed nucleic acids of one or more cells. In certain instances, "isolated nucleic acid" refers to a nucleic acid that has been isolated free of, or is otherwise free of, bulk of cellular components or *in vitro* reaction components such as for example, macromolecules such as lipids or proteins, small biological molecules, and the like.

### H. Nucleic Acid Segments

The nucleic acid may be a nucleic acid segment. As used herein, the term "nucleic acid segment," are smaller fragments of a nucleic acid, such as for non-limiting example, those that encode only part of the ANGPTL8 peptide or polypeptide sequence. Thus, a "nucleic acid segment" may comprise any part of a gene sequence, of from about 2 nucleotides to the full length of the ANGPTL8 peptide or polypeptide encoding region.

Various nucleic acid segments may be designed based on a particular nucleic acid sequence, and may be of any length. By assigning numeric values to a sequence, for example, the first residue is 1, the second residue is 2, *etc.,* an algorithm defining all nucleic acid segments can be created:
n to n + y
where n is an integer from 1 to the last number of the sequence and y is the length of the nucleic acid segment minus one, where n + y does not exceed the last number of the sequence. Thus, for a 10-mer, the nucleic acid segments correspond to bases 1 to 10, 2 to 11, 3 to 12 ... and so on. For a 15-mer, the nucleic acid segments correspond to bases 1 to 15, 2 to 16, 3 to 17 ... and so on. For a 20-mer, the nucleic segments correspond to bases 1 to 20, 2 to 21, 3 to 22 ... and so on. In certain aspects, the nucleic acid segment may be a probe or primer. As used herein, a "probe" generally refers to a nucleic acid used in a detection method or composition. As used herein, a "primer" generally refers to a nucleic acid used in an extension or amplification method or composition.

### I. Nucleic Acid Complements

The present disclosure also encompasses a nucleic acid that is complementary to a ANGPTL8 nucleic acid. In particular aspects the disclosure encompasses a nucleic acid or a nucleic acid segment complementary to the ANGPTL8 encoding sequence. A nucleic acid "complement(s)" or is "complementary" to another nucleic acid when it is capable of base-pairing with another nucleic acid according to the standard Watson-Crick, Hoogsteen or reverse Hoogsteen binding complementarity rules. As used herein "another nucleic acid" may refer to a separate molecule or a spatial separated sequence of the same molecule.

As used herein, the term "complementary" or "complement(s)" also refers to a nucleic acid comprising a sequence of consecutive nucleobases or semiconsecutive nucleobases (*e.g*., one or more nucleobase moieties are not present in the molecule) capable of hybridizing to another nucleic acid strand or duplex even if less than all the nucleobases do not base pair with a counterpart nucleobase. In certain aspects, a "complementary" nucleic acid comprises a sequence in which about 70%, about 71%, about 72%, about 73%, about 74%, about 75%, about 76%, about 77%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, to about 100%, and any range derivable therein, of the nucleobase sequence is capable of base-pairing with a single or double stranded nucleic acid molecule during hybridization. In certain aspects, the term "complementary" refers to a nucleic acid that may hybridize to another nucleic acid strand or duplex in stringent conditions, as would be understood by one of ordinary skill in the art.

In certain aspects, a "partly complementary" nucleic acid comprises a sequence that may hybridize in low stringency conditions to a single or double stranded nucleic acid, or contains a sequence in which less than about 70% of the nucleobase sequence is capable of base-pairing with a single or double stranded nucleic acid molecule during hybridization.

### J. Hybridization

As used herein, "hybridization", "hybridizes" or "capable of hybridizing" is understood to mean the forming of a double or triple stranded molecule or a molecule with partial double or triple stranded nature. The term "anneal" as used herein is synonymous with "hybridize." The term "hybridization", "hybridize(s)" or "capable of hybridizing" encompasses the terms "stringent condition(s)" or "high stringency" and the terms "low stringency" or "low stringency condition(s)."

As used herein "stringent condition(s)" or "high stringency" are those conditions that allow hybridization between or within one or more nucleic acid strand(s) containing complementary sequence(s), but precludes hybridization of random sequences. Stringent conditions tolerate little, if any, mismatch between a nucleic acid and a target strand. Such conditions are well known to those of ordinary skill in the art, and are preferred for applications requiring high selectivity. Non-limiting applications include isolating a nucleic acid, such as a gene or a nucleic acid segment thereof, or detecting at least one specific mRNA transcript or a nucleic acid segment thereof, and the like.

Stringent conditions may comprise low salt and/or high temperature conditions, such as provided by about 0.02 M to about 0.15 M NaCl at temperatures of about 50°C to about 70°C. It is understood that the temperature and ionic strength of a desired stringency are determined in part by the length of the particular nucleic acid(s), the length and nucleobase content of the target sequence(s), the charge composition of the nucleic acid(s), and to the presence or concentration of formamide, tetramethylammonium chloride or other solvent(s) in a hybridization mixture.

It is also understood that these ranges, compositions and conditions for hybridization are mentioned by way of non-limiting examples only, and that the desired stringency for a particular hybridization reaction is often determined empirically by comparison to one or more positive or negative controls. Depending on the application envisioned it is preferred to employ varying conditions of hybridization to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions", and non-limiting examples of low stringency include hybridization performed at about 0.15 M to about 0.9 M NaCl at a temperature range of about 20°C to about 50°C. Of course, it is within the skill of one in the art to further modify the low or high stringency conditions to suite a particular application.

As used herein "wild-type" refers to the naturally occurring sequence of a nucleic acid at a genetic locus in the genome of an organism, or a sequence transcribed or translated from such a nucleic acid. Thus, the term "wild-type" also may refer to an amino acid sequence encoded by a nucleic acid. As a genetic locus may have more than one sequence or alleles in a population of individuals, the term "wild-type" encompasses all such naturally occurring allele(s). As used herein the term "polymorphic" means that variation exists *(i.e.,* two or more alleles exist) at a genetic locus in the individuals of a population. As used herein "mutant" refers to a change in the sequence of a nucleic acid or its encoded protein, polypeptide or peptide that is the result of the hand of man.

The present disclosure also concerns the isolation or creation of a recombinant construct or a recombinant host cell through the application of recombinant nucleic acid technology known to those of skill in the art or as described herein. A recombinant construct or host cell may comprise an ANGPTL8 polynucleotide, and may express an ANGPTL8 protein, peptide or peptide, or at least one biologically functional equivalent thereof.

Herein, in certain aspects, a "gene" refers to a nucleic acid that is transcribed. In certain aspects, the gene includes regulatory sequences involved in transcription, or message production or composition. In particular aspects, the gene comprises transcribed sequences that encode for a protein, polypeptide or peptide. As will be understood by those in the art, this function term "gene" includes both genomic sequences, RNA or cDNA sequences or smaller engineered nucleic acid segments, including nucleic acid segments of a non-transcribed part of a gene, including but not limited to the non-transcribed promoter or enhancer regions of a gene. Smaller engineered gene nucleic acid segments may express, or may be adapted to express using nucleic acid manipulation technology, proteins, polypeptides, domains, peptides, fusion proteins, mutants and/or such like.

"Isolated substantially away from other coding sequences" means that the gene of interest forms the significant part of the coding region of the nucleic acid, or that the nucleic acid does not contain large portions of naturally-occurring coding nucleic acids, such as large chromosomal fragments, other functional genes, RNA or cDNA coding regions. Of course, this refers to the nucleic acid as originally isolated, and does not exclude genes or coding regions later added to the nucleic acid by the hand of man.

The nucleic acid(s) of the present disclosure, regardless of the length of the sequence itself, may be combined with other nucleic acid sequences, including but not limited to, promoters, enhancers, polyadenylation signals, restriction enzyme sites, multiple cloning sites, coding segments, and the like, to create one or more nucleic acid construct(s). As used herein, a "nucleic acid construct" is a nucleic acid engineered or altered by the hand of man, and generally comprises one or more nucleic acid sequences organized by the hand of man.

In a non-limiting example, one or more nucleic acid constructs may be prepared that include a contiguous stretch of nucleotides identical to or complementary (at least in part) to SEQ ID NO: 1. A nucleic acid construct may be about 3, about 5, about 8, about 10 to about 14, or about 15, about 20, about 30, about 40, about 50, about 100, about 115, about 200, about 500, about 600, or about 650 nucleotides in length, as well as constructs of greater size, up to and including chromosomal sizes (including all intermediate lengths and intermediate ranges), given the advent of nucleic acids constructs such as a yeast artificial chromosome are known to those of ordinary skill in the art. It will be readily understood that "intermediate lengths" and "intermediate ranges", as used herein, means any length or range including or between the quoted values *(i.e.,* all integers including and between such values). Non-limiting examples of intermediate lengths include about 11, about 12, about 13, about 16, about 17, about 18, about 19, *etc.;* about 21, about 22, about 23, *etc.;* about 31, about 32, *etc.;* about 51, about 52, about 53, *etc.;* about 101, about 102, about 103, *etc.;* about 151, about 152, about 153, *etc.;* about 600, about 601, about 605, about 610, *etc.* Non-limiting examples of intermediate ranges include about 3 to about 32, about 150 to about 850, *etc.*

In certain embodiments, the nucleic acid construct is a recombinant vector. In particular embodiments, the disclosure concerns one or more recombinant vector(s) comprising nucleic acid sequences that encode an ANGPTL8 protein, polypeptide or peptide that includes within its amino acid sequence a contiguous amino acid sequence in accordance with, or essentially as set forth in, SEQ ID NO:2, corresponding to SEQ ID NO:1 nucleic acid. In particular aspects, the recombinant vectors are DNA vectors.

The term "biologically functional equivalent" is well understood in the art and is further defined in detail herein. Accordingly, a sequence that has 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of amino acids that are identical or functionally equivalent to the amino acids of SEQ ID NO:2, provided the biological activity of the protein, polypeptide or peptide is maintained.

In certain other embodiments, the disclosure concerns at least one recombinant vector that include within its sequence a polynucleotide sequence essentially as set forth in SEQ ID NO: 1. In particular embodiments, the recombinant vector comprises DNA sequences that encode protein(s), polypeptide(s) or peptide(s) exhibiting ANGPTL8 activity.

The term "functionally equivalent codon" is used herein to refer to codons that encode the same amino acid, such as the six codons for arginine and serine, and also refers to codons that encode biologically equivalent amino acids. Codon usage for various organisms and organelles can be found in the literature. Thus, it is contemplated that codon usage may be optimized for other animals, as well as other organisms such as a prokaryote *(e.g.,* an eubacteria, an archaea), an eukaryote *(e.g.,* a protist, a plant, a fungi, an animal), a virus and the like, as well as organelles that contain nucleic acids, such as mitochondria, chloroplasts and the like, based on the preferred codon usage as would be known to those of ordinary skill in the art.

It will also be understood that amino acid sequences or nucleic acid sequences may include additional residues, such as additional N- or C-terminal amino acids or 5' or 3' sequences, or various combinations thereof, and yet still be essentially as set forth in one of the sequences disclosed herein, so long as the sequence meets the criteria set forth above, including the maintenance of biological protein, polypeptide or peptide activity where expression of a proteinaceous composition is concerned. The addition of terminal sequences particularly applies to nucleic acid sequences that may, for example, include various non-coding sequences flanking either of the 5' and/or 3' portions of the coding region or may include various internal sequences, *i.e.,* introns, which are known to occur within genes.

Excepting intronic and flanking regions, and allowing for the degeneracy of the genetic code, nucleic acid sequences that have between about 70% and about 79%; or more preferably, between about 80% and about 89%; or even more particularly, between about 90% and about 99%; of nucleotides that are identical to the nucleotides of SEQ ID NO: 1 are encompassed in the disclosure.

Encompassed in the disclosure include sequences that are 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, or less percent identical to SEQ ID NO: 1 or SEQ ID NO:2. The sequences that are not 100 percent identical to SEQ ID NO: 1 or SEQ ID NO:2 may share their identity therewith at any region of the sequence, including the N-terminal or C-terminal ends, or inbetween thereof of SEQ ID NO:2 or the 5' or 3' ends or inbetween thereof SEQ ID NO: 1.

It will also be understood that this disclosure is not limited to the particular nucleic acid or amino acid sequences of SEQ ID NO:1 or SEQ ID NO:2, respectively. Recombinant vectors and isolated nucleic acid segments may therefore variously include these coding regions themselves, coding regions bearing selected alterations or modifications in the basic coding region, and they may encode larger polypeptides or peptides that nevertheless include such coding regions or may encode biologically functional equivalent proteins, polypeptide or peptides that have variant amino acids sequences.

The nucleic acids of the present disclosure encompass biologically functional equivalent ANGPTL8 proteins, polypeptides, or peptides. Such sequences may arise as a consequence of codon redundancy or functional equivalency that are known to occur naturally within nucleic acid sequences or the proteins, polypeptides or peptides thus encoded. Alternatively, functionally equivalent proteins, polypeptides or peptides may be created *via* the application of recombinant DNA technology, in which changes in the protein, polypeptide or peptide structure may be engineered, based on considerations of the properties of the amino acids being exchanged. Changes designed by man may be introduced, for example, through the application of site-directed mutagenesis techniques as discussed herein below, *e.g.,* to introduce improvements or alterations to the antigenicity of the protein, polypeptide or peptide, or to test mutants in order to examine ANGPTL8 protein, polypeptide or peptide activity at the molecular level.

### IV. Ultrasound Targeted Microbubble Destruction (UTMD) System

In one aspect, there are methods of delivering a bioactive agent to a target organ or tissue *in vivo* by using an ultrasound-targeted microbubble destruction (UTMD), using microbubbles loaded with nanosphere cationic liposomes containing the bioactive agent. Exemplary microbubbles comprise but are not limited to neutrally charged lipids, polymers, metals, or acrylic shells suitable for *in vivo* ultrasound-targeted microbubble destruction. In one aspect, the bioactive agent is first encapsulated within or attached to tiny cationic liposomes of nanoparticle size (10-60 nm) (hereinafter, nanosphere cationic liposomes either "loaded with" or "including" the bioactive agent refers to any bioactive agent encapsulated within or attached to the liposomes, e.g., cationic liposomes), and the liposomes are then attached to neutrally charged lipid-coated or albumin-coated microbubbles filled with a gas suitable for ultrasound microbubble destruction techniques, for example perfluoropropane. The liposomes may be attached to the outer surface of the microbubble shell, incorporated within the microbubble shell and/or encapsulated within the microbubble shell. In the present disclosure, one or more bioactive agents can be delivered either concomitantly or subsequently by ultrasound-targeted microbubble destruction using the neutrally charged lipid microbubbles loaded with bioactive agent-containing nanosphere cationic liposomes. In another aspect, the present disclosure is a method of treating a mammal in need of such treatment comprising administration of an effective amount of a composition comprising neutrally charged lipid microbubbles loaded with nanosphere cationic liposomes containing a bioactive agent *via* ultrasound-targeted microbubble destruction.

Examples of bioactive agents suitable for the present disclosure include one or more of polynucleotides (including ANGPTL8 polynucleotides) pharmaceuticals and drugs, bioactive synthetic organic molecules, proteins, peptides, polypeptides, vitamins, steroids, polyanionic agents, genetic material, and diagnostic agents. Bioactive vitamins, steroids, proteins, peptides and polypeptides can be of natural origin or synthetic. Exemplary polyanionic agents include but are not limited to sulphated polysaccharides, negatively charged serum albumin and milk proteins, synthetic sulphated polymers, polymerized anionic surfactants, and polyphosphates. Suitable diagnostic agents include but are not limited to dyes and contrast agents for use in connection with magnetic resonance imaging, ultrasound or computed tomography of a patient.

Suitable genetic material includes nucleic acids, nucleosides, nucleotides, and polynucleotides that can be either isolated genomic, synthetic or recombinant material; either single or double stranded; and either in the sense or antisense direction, with or without modifications to bases, carbohydrate residues or phosphodiester linkages. Exemplary sources for the genetic material include but are not limited to deoxyribonucleic acids (DNA), ribonucleic acids (RNA), complementary DNA (cDNA), messenger RNA (mRNA), ribosomal RNA (rRNA), short interfering RNA (siRNA), ribozymes, and mixed duplexes and triplexes of RNA and DNA.

Genetic materials are genes carried on expression vectors including but not limited to helper viruses, plasmids, phagemids, cosmids, and yeast artificial chromosomes. The genetic material suitable for the present disclosure is capable of coding for at least a portion of a therapeutic, regulatory, and/or diagnostic protein. Moreover, genetic materials can preferably code for more than one type of protein. For example, a bioactive agent may comprise plasmid DNA comprising genetic material encoding therapeutic protein and a selectable or diagnostic marker to monitor the delivery of the plasmid DNA, *e.g.,* pDsRedhuman insulin promoter. Such proteins include but are not limited to histocompatibility antigens, cell adhesion molecules, growth factors, coagulation factors, hormones, insulin, cytokines, chemokines, antibodies, antibody fragments, cell receptors, intracellular enzymes, transcriptional factors, toxic peptides capable of eliminating diseased or malignant cells. Other genetic materials that could be delivered by this technique included adenovirus, adeno-associated virus, retrovirus, lentivirus, RNA, siRNA, or chemicals that selectively turn on or off specific genes, such as polyamides or peptide fragments. Modifications to wild-type proteins resulting in agonists or antagonists of the wild type variant fall in the scope of this disclosure. The genetic material may also comprise a tissue-specific promoter or expression control sequences such as a transcriptional promoter, an enhancer, a transcriptional terminator, an operator or other control sequences.

Examples of other agents that may be used with the polynucleotides of the present disclosure include one or more of the following therapeutics pre-loaded into a liposome and associated with microbubbles including, but are not limited to, hormone products such as, vasopressin and oxytocin and their derivatives, glucagon and thyroid agents as iodine products and anti-thyroid agents; cardiovascular products as chelating agents and mercurial diuretics and cardiac glycosides; respiratory products as xanthine derivatives (theophylline and aminophylline); anti-infectives as aminoglycosides, antifungals (*e.g*., amphotericin), penicillin and cephalosporin antibiotics, antiviral agents (*e.g*., Zidovudine, Ribavirin, Amantadine, Vidarabine and Acyclovir), antihelmintics, antimalarials, and antituberculous drugs; biologicals such as antibodies (*e.g*., antitoxins and antivenins), vaccine antigens (*e.g.,* bacterial vaccines, viral vaccines, toxoids); antineoplastics (*e.g.,* nitrosoureas, nitrogen mustards, antimetabolites (fluorouracil, hormones, progestins and estrogens agonists and/or antagonists); mitotic inhibitors (*e.g*., Etoposide and/or Vinca alkaloids), radiopharmaceuticals (*e.g*., radioactive iodine and phosphorus products); and Interferon, hydroxyurea, procarbazine, Dacarbazine, Mitotane, Asparaginase and cyclosporins, including mixtures and combinations thereof. Other suitable therapeutics include, but are not limited to: thrombolytic agents such as urokinase; coagulants such as thrombin; antineoplastic agents, such as platinum compounds (*e.g*., spiroplatin, cisplatin, and carboplatin), methotrexate, adriamycin, taxol, mitomycin, ansamitocin, bleomycin, cytosine arabinoside, arabinosyl adsnine, mercaptopolylysine, vincristine, busulfan, chlorambucil, melphalan (*e.g*., PAM, L-PAM or phenylalanine mustard), mercaptopurine, mitotane, procarbazine hydrochloride dactinomycin (actinomycin D), daunorubicinhydrochloride, doxorubicin hydrochloride, mitomycin, plicamycin (mithramycin), aminoglutethimide, estramustine phosphate sodium, flutamide, leuprolide acetate, megestrol acetate, tamoxifen citrate, testolactone, trilostane, amsacrine (m-AMSA), asparaginase (L-asparaginase), Erwinaasparaginase, etoposide (VP-16), interferon alpha-2a, interferon alpha-2b, teniposide (VM-26), vinblastine sulfate (VLB), vincristine sulfate, bleomycin, bleomycin sulfate, methotrexate, adriamycin, and arabinosyl; blood products such as parenteral iron, hemin; biological response modifiers such as muramyldipeptide, muramyltripeptide, microbial cell wall components, lymphokines (*e.g.*, bacterial endotoxin such as lipopolysaccharide, macrophage activation factor), sub-units of bacteria (such as Mycobacteria, Corynebacteria), the synthetic dipeptide N-acetyl-muramyl-L-alanyl-D-isog-lutamine; anti-fungalagents such as ketoconazole, nystatin, griseofulvin, flucytosine (5-fc), miconazole, amphotericin B, ricin, and beta-lactam antibiotics (*e.g*., penicillin, ampicillin, sulfazecin); hormones such as growth hormone, PDGF, EGF, CSF, GM-CSF, melanocyte stimulating hormone, estradiol, beclomethasone dipropionate, betamethasone, betamethasone acetate and betamethasone sodium phosphate, vetamethasonedisodiumphosphate, vetamethasone sodium phosphate, cortisone acetate, dexamethasone, dexamethasone acetate, dexamethasone sodium phosphate, flunsolide, hydrocortisone, hydrocortisone acetate, hydrocortisone cypionate, hydrocortisone sodium phosphate, hydrocortisone sodium succinate, methylprednisolone, methylprednisolone acetate, methylprednisolone sodium succinate, paramethasone acetate, prednisolone, prednisoloneacetate, prednisolone sodium phosphate, prednisolone rebutate, prednisone, triamcinolone, triamcinolone acetonide, triamcinolone diacetate, triamcinolone hexacetonide and fludrocortisone acetate; vitamins such vitamin C, E, A, K, ascyanocobalamin, neinoic acid, retinoids and derivatives such as retinolpalmitate, and alpha-tocopherol(s); peptides (*e.g.,* T cell epitopes such as MAGE, GAGE, DAGE, *etc.*)*;* proteins, such as manganese super oxide dimutase, alcohol dehydrogenase, nitric oxide synthase; enzymes such as alkaline phosphatase; anti-allergic agents such as amelexanox; anti-coagulation agents such as phenprocoumon and heparin; circulatory drugs such as propranolol; metabolic potentiators such asglutathione; antituberculars such as para-aminosalicylic acid, isoniazid, capreomycin sulfate cycloserine, ethambutol hydrochloride ethionamide, pyrazinamide, rifampin, and streptomycin sulfate; antivirals such as acyclovir, amantadine azidothymidine (AZT or Zidovudine), Ribavirin andvidarabine monohydrate (adenine arabinoside, ara-A); antianginals such asdiltiazem, nifedipine, verapamil, erythrityl tetranitrate, isosorbidedinitrate, nitroglycerin (glyceryl trinitrate) and pentaerythritoltetranitrate; anticoagulants such as phenprocoumon, heparin; antibiotics such as dapsone, chloramphenicol, neomycin, cefaclor, cefadroxil, cephalexin, cephradine erythromycin, clindamycin, lincomycin, amoxicillin, ampicillin, bacampicillin, carbenicillin, dicloxacillin, cyclacillin, picloxacillin, hetacillin, methicillin, nafcillin, oxacillin, penicillin G, penicillin V, ticarcillin rifampin and tetracycline; antiinflammatories such as difunisal, ibuprofen, indomethacin, meclofenamate, mefenamic acid, naproxen, oxyphenbutazone, phenylbutazone, piroxicam, sulindac, tolmetin, aspirin and salicylates; antiprotozoans such as chloroquine, hydroxychloroquine, metronidazole, quinine and meglumine antimonate; antirheumatics such as penicillamine; narcotics such as paregoric; opiates such as codeine, heroin, methadone, morphine and opium; cardiac glycosides such as deslanoside, digitoxin, digoxin, digitalin and digitalis; neuromuscular blockers such as atracurium besylate, gallamine triethiodide, hexafluorenium bromide, metocurine iodide, pancuronium bromide, succinylcholine chloride (suxamethonium chloride), tubocurarine chloride and vecuronium bromide; sedatives (hypnotics) such as amobarbital, amobarbital sodium, aprobarbital, butabarbital sodium, chloral hydrate, ethchlorvynol, ethinamate, flurazepam hydrochloride, glutethimide, methotrimeprazine hydrochloride, methyprylon, midazolam hydrochloride, paraldehyde, pentobarbital, pentobarbital sodium, phenobarbital sodium, secobarbital sodium, talbutal, temazepam and triazolam; local anesthetics such as bupivacaine hydrochloride, chloroprocaine hydrochloride, etidocainehydrochloride, lidocaine hydrochloride, mepivacaine hydrochloride, procainehydrochloride and tetracaine hydrochloride; general anesthetics such asdroperidol, etomidate, fentanyl citrate with droperidol, ketaminehydrochloride, methohexital sodium and thiopental sodium; and radioactive particles or ions such as strontium, iodide rhenium and yttrium, and combinations and mixtures thereof.

In addition to the polynucleotides of the disclosure, prodrugs may be pre-loaded into the liposomes prior to attachment to the microbubbles. Prodrugs are well known in the art and may include inactive drug precursors that are metabolized to form active drugs. The skilled artisan will recognize suitable prodrugs (and if necessary their salt forms) as described by, *e.g.,* in Sinkula, *et al.,* 1975. Prodrugs, for example, may include inactive forms of the active drugs wherein a chemical group is present on the prodrug which renders it inactive and/or confers solubility or some other property to the drug. In this form, the prodrugs are generally inactive, but once the chemical group has been cleaved from the prodrug, by heat, cavitation, pressure, and/or by enzymes in the surrounding environment or otherwise, the active drug is generated. Such prodrugs are well described in the art, and comprise a wide variety of drugs bound to chemical groups through bonds such as esters to short, medium or long chain aliphatic carbonates, hemiesters of organic phosphate, pyrophosphate, sulfate, amides, amino acids, azo bonds, carbamate, phosphamide, glucosiduronate, N-acetylglucosamine and beta-glucoside. Examples of drugs with the parent molecule and the reversible modification or linkage are as follows: convallatoxin with ketals, hydantoin with alkyl esters, chlorphenesin with glycine or alanins esters, acetaminophen with caffeine complex, acetylsalicylic acid with THAM salt, acetylsalicylic acid with acetamidophenyl ester, naloxone with sulfateester, 15-methylprostaglandin F sub 2 with methyl ester, procaine with polyethylene glycol, erythromycin with alkyl esters, clindamycin with alkylesters or phosphate esters, tetracycline with betains salts, 7-acylaminocephalosporins with ring-substituted acyloxybenzyl esters, nandrolone with phenylproprionate decanoate esters, estradiol with enolether acetal, methylprednisolone with acetate esters, testosterone with n-acetylglucosaminide glucosiduronate (trimethylsilyl) ether, cortisol or prednisolone or dexamethasone with 21-phosphate esters. Prodrugs may also be designed as reversible drug derivatives and used as modifiers to enhance drug transport to site-specific tissues. Examples of carrier molecules with reversible modifications or linkages to influence transport to a site specific tissue and for enhanced therapeutic effect include isocyanate with haloalkyl nitrosurea, testosterone with propionateester, methotrexate (3-5'-dichloromethotrexat-e) with dialkyl esters, cytosine arabinoside with 5'-acylate, nitrogen mustard (2,2'-dichloro-N-methyldiethylamine), nitrogen mustard with aminomethyltetracycline, nitrogen mustard with cholesterol or estradiol ordehydroepiandrosterone esters and nitrogen mustard with azobenzene.

The skilled artisan will recognize that a particular chemical group may be modified in any given agent may be selected to influence the partitioning of the drug into either the shell or the interior of the microbubbles. The bond selected to link the chemical group to the drug may be selected to have the desired rate of metabolism, e.g., hydrolysis in the case of ester bonds in the presence of serum esterases after release from the microbubbles. Additionally, the particular chemical group may be selected to influence the biodistribution of the drug employed in the microbubbles, e.g., N,N-bis(2-chloroethyl)-phosphorodiamidic acid with cyclic phosphoramide. Additionally, the prodrugs employed within the microbubbles may be designed to contain reversible derivatives that are used as modifiers of duration of activity to provide, prolong or depot action effects.

For example, nicotinic acid may be modified with dextran and carboxymethlydextran esters, streptomycin with alginic acid salt, dihydrostreptomycin with pamoate salt, cytarabine (ara-C) with 5'-adamantoats ester, ara-adenosine (ara-A) with 5-palmirate and 5'-benzoate esters, amphotericin B with methyl esters, testosterone with 17-beta-alkyl esters, estradiol with formate ester, prostaglandin with 2-(4-imidazolyl) ethylamine salt, dopamine with amino acid amides, chloramphenicol with mono- and bis(trimethylsilyl) ethers, and cycloguanil with pamoate salt. In this form, a depot or reservoir of long-acting drug may be released *in vivo* from the prodrug bearing microbubbles. The particular chemical structure of the therapeutics may be selected or modified to achieve a desired solubility such that the therapeutic is loaded into a liposome prior to attaching or loading in, to, at or about a microbubble. Similarly, other therapeutics may be formulated with a hydrophobic group which is aromatic or sterol in structure to incorporate into the surface of the microbubble.

Cationic liposomes suitable for use in the present disclosure comprise one or more monocationic or polycationic lipids, optionally combined with one or more neutral or helper lipids. The cationic lipids suitable for the present disclosure can be obtained commercially or made by methods known in the art. Cationic lipids suitable for the formation of cationic liposomes are well known in the art and include but are not limited to any phospholipid-related materials, such as lecithin, phosphatidylethanolamine, lysolecithin, lysophosphatidylethanolamine, phosphatidylserine, phosphatidylinositol, sphingomyelin, cephalin, cardiolipin, phosphatidic acid, cerebrosides, dicetylphosphate, dioleoylphosphatidylcholine (DOPC), dipalmitoylphosphatidylcholine (DPPC), dioleoylphosphatidylglycerol (DOPG), dipalmitoylphosphatidylglycerol (DPPG), dipalmitoylphosphatidylethanolamine 5-carboxyspermylamide (DPPES), dioleoylphosphatidylethanolamine (DOPE), palmitoyloleoylphosphatidylcholine (POPC), palmitoyloleoylphosphatidylethanolamine (POPE) and dioleoylphosphatidyl-ethanolamine 4-(N-maleimidomethyl)cyclohexane-1-carboxylate (DOPE-mal). Additional non-phosphorous containing lipids include but are not limited to stearylamine, dodecylamine, hexadecylamine, acetyl palmitate, glycerol ricinoleate, hexadecyl stearate, isopropyl myristate, amphoteric acrylic polymers, triethanolamine-lauryl sulfate, alkyl-aryl sulfate polyethyloxylated fatty acid amides, dioctadecyldimethyl ammonium bromide and steroids such as cholesterol, ergosterol, ergosterol B1, B2 and B3, androsterone, cholic acid, desoxycholic acid, chenodesoxycholic acid, lithocholic acid, N-[1-(2,3-dioleoyloxy)propyl]-N,N,N-trimethylammonium chloride (DOTMA), 1,2-bis(oleoyloxy)-3-3-(trimethylammonia)propane (DOTAP), and 5-carboxyspermylglycine dioctadecylamide (DOGS). A preferred liposome formulation comprises the polycationic lipid 2,3-dioleyloxy-N-[2-(sperminecarboxaido)ethyl]-N,N-dimethyl-1-propanaminu- m trifluoroacetate (DOSPA) and the neutral lipid dioleoyl phosphatidylethanolamine (DOPE) at (3:1, w/w), and mixtures and combinations thereof.

In the method of the present disclosure, the cationic liposomes are loaded with the polynucleotide, at least. In one aspect, a cationic lipid formulation of one or more lipids dissolved in one or more organic solvents is first dried or lyophilized to remove the organic solvent(s), resulting in a lipid film. Just prior to use, the lipid film is mixed with a polynucleotide suitable for the present disclosure suspended in a suitable aqueous medium for forming liposomes from the dried lipid film. For example, water, an aqueous buffer solution, or a tissue culture media can be used for rehydration of the lipid film. A suitable buffer is phosphate buffered saline, *i.e.,* 10 mM potassium phosphate having a pH of 7.4 in 0.9% NaCl solution. In another aspect, the dried lipid film is rehydrated with a suitable aqueous medium to form liposomes before the addition of the bioactive agent. This method is preferred when the bioactive agent comprises genetic material. The incorporation of the bioactive agent into the cationic liposomes is often performed at a temperature within the range of about 0 to 30° C., *e.g.,* room temperature, in about 5, 10-20 minutes.

In the methods of the present disclosure, the cationic liposomes with attached bioactive agent(s) (such as a polynucleotide) are then loaded onto neutrally charged microbubbles. In a preferred aspect, this is accomplished by adding to the cationic liposomes with attached bioactive agent(s) a lipid composition suitable for making the microbubble shell, mixing well, and then adding an appropriate gas for encapsulation by the microbubble shell, followed by vigorous shaking for about 5 to 60 seconds, preferably for about 20 seconds. In a preferred method, the lipid composition is kept at about 0 to 30° C. before the addition of the cationic liposomes with attached bioactive agent(s).

To form the microbubble shell, any biocompatible lipid of natural or synthetic origin known to be useful in ultrasound-targeted microbubble destruction are contemplated as part of the present disclosure. Exemplary lipids can be found in International Application No. WO 2000/45856 and include but are not limited to fatty acids, phosphatides, glycolipids, glycosphingolipids, sphingolipids, aliphatic alcohols, aliphatic waxes, terpenes, sesquiterpenes, and steroids. Examples of lipids are phosphocholines, phosphatidylcholines, phosphatidylethanolamines, phosphatidylserines, phosphatidylglycerols, and phosphatidylinositol. A particular lipid is 1,2-palmitoyl-sn-glycero-3-phosphocholine or 1,2-palmitoyl-sn-glycero-phosphatidylethanolamine. The specific lipid is L-1,2-palmitoyl-snglycero-3-phosphocholine and L-1,2-palmitoyl-sn-glycero-phosphatidylethanolamine.

Gases suitable for the present disclosure are generally inert and biocompatible, including but not limited to air; carbon dioxide; nitrogen; oxygen; fluorine; noble gases such as helium, neon, argon, and xenon; sulfur-based gases; fluorinated gases; and mixtures thereof. The gas may be a perfluoropropane, e.g., octafluoropropane.

As is well known to those versed in the art, targeting ligands can also be attached to the microbubbles to confer additional tissue specificity. Such ligands could include monoclonal antibodies, peptides, polypeptides, proteins, glycoproteins, hormones or hormone analogues, monosaccharides, polysaccharides, steroids or steroid analogues, vitamins, cytokines, or nucleotides.

The delivery methods of the present disclosure comprising neutrally charged microbubbles loaded with nanosphere cationic liposomes containing one or more bioactive agents provide all the advantages of an ultrasound-targeted microbubble delivery system combined with all the advantages of a liposome delivery system. The ultrasound-targeted microbubble delivery system allows for delivery of a drug/gene bioactive agent to a specific organ or tissue while minimizing the exposure of other organs or tissues to the bioactive agent. During delivery, the bioactive agent(s) remain within the protective cationic liposome, which shields the bioactive agent(s) from proteases, nucleases, lipases, carbohydrate-cleaving enzymes, free radicals, or other chemical alterations. This method increases the delivery of the bioactive agent and its bioavailability to the target tissue. For example, in the delivery of neutrally charged microbubbles loaded with nanosphere cationic liposomes containing plasmid DNA, the level of gene expression at the target site is increased over the level of expression possible with either a microbubble delivery or a liposome delivery of the same plasmid DNA.

In one aspect, the present disclosure is a method of treating a mammal in need of such treatment comprising administration of an effective amount of a composition comprising neutrally charged lipid microbubbles loaded with nanosphere cationic liposomes containing a bioactive agent (such as an ANGPTL8 polynucleotide) *via* ultrasound-targeted microbubble destruction. Administration of the composition comprising neutrally charged lipid microbubbles loaded with nanosphere cationic liposomes containing a bioactive agent and the ultrasound-targeted microbubble destruction of these microbubbles to release the bioactive agent can be accomplished by any means known in the art. Repeat administration of the microbubbles is possible, particularly to prolong the duration of the therapeutic effect. For example, repeated transfection of cardiomyocytes by ultrasound targeted microbubble destruction has been shown to extend the peak duration of luciferase activity in the heart from 4 days to 12 days (Bekeredjian et al, 2003). This potentially allows for the duration of gene or drug delivery to be tailored to the specific biological or medical need.

### V. Nucleic Acid-Based Expression Systems

ANGPTL8 may be provided as a polynucleotide in the UTMD system to a target tissue, such as the heart. The ANGPTL8 polynucleotide may be provided in an expression vector for use in the UTMD system. The polynucleotide in the UTMD system may be provided to a target tissue, such as the liver (for indirect delivery to the heart) or the heart or the pancreas or brain.

An expression vector for use in the UTMD system may comprise one or more suitable restriction enzyme digestion sequences, start codons, stop codons, nuclear localization signals, protease cutting codons, selectable markers, origins of replication, regulatory regions, multiple cloning sites, and a combination thereof. Such moieties may be positioned in the expression vector in any suitable order.

### A. Vectors

The term "vector" is used to refer to a carrier nucleic acid molecule into which an ANGPTL8 nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (*e.g*., YACs). One of skill in the art would be well equipped to construct a vector through standard recombinant techniques (see, for example, Maniatis *et al.,* 1988 and Ausubel *et al.,* 1994).

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host cell. In addition to control sequences that govern transcription and translation, vectors and expression vectors may contain nucleic acid sequences that serve other functions as well and are described *infra.*

### 1. Promoters and Enhancers

A "promoter" is a control sequence that is a region of a nucleic acid sequence at which initiation and rate of transcription are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control," and "under transcriptional control" mean that a promoter is in a correct functional location and/or orientation in relation to a nucleic acid sequence to control transcriptional initiation and/or expression of that sequence.

In embodiments of the invention, a CMV promoter or a tissue-specific promoter may be employed. The tissue-specific promoter may be a cardiac tissue specific promoter. Examples of cardiac tissue specific promoters include ventricle-specific myosin light chain-2 (mlc-2v); alpha-myosin heavy chain (a-MHC). Another example of a promoter that may be employed includes the insulin promoter, including the rat insulin promoter.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of *(i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cisacting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e.,* containing different elements of different transcriptional regulatory regions, and/or mutations that alter expression. For example, promoters that are most commonly used in recombinant DNA construction include the β-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR^{™}, in connection with the compositions disclosed herein (see U.S. Patent Nos. 4,683,202 and 5,928,906). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression, (see, for example Sambrook *et al.* 1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination (as per, for example, the Eukaryotic Promoter Data Base EPDB, http://www.epd.isb-sib.ch/) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

### 2. Initiation Signals and Internal Ribosome Binding Sites

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain aspects of the disclosure, the use of internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819).

### 3. Multiple Cloning Sites

Vectors can include a multiple cloning site (MCS), which is a nucleic acid region that contains multiple restriction enzyme sites, any of which can be used in conjunction with standard recombinant technology to digest the vector (see, for example, Carbonelli *et al.,* 1999, Levenson *et al.,* 1998, and Cocea, 1997) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### 4. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression (see, for example, Chandler *et al.,* 1997)

### 5. Termination Signals

The vectors or constructs of the present disclosure will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message. The terminator and/or polyadenylation site elements can serve to enhance message levels and to minimize read through from the cassette into other sequences.

Terminators contemplated for use in the disclosure include any known terminator of transcription described herein or known to one of ordinary skill in the art, including but not limited to, for example, the termination sequences of genes, such as for example the bovine growth hormone terminator or viral termination sequences, such as for example the SV40 terminator. In certain embodiments, the termination signal may be a lack of transcribable or translatable sequence, such as due to a sequence truncation.

### 6. Polyadenylation Signals

In expression, particularly eukaryotic expression, one will typically include a polyadenylation signal to effect proper polyadenylation of the transcript. The nature of the polyadenylation signal is not believed to be crucial to the successful practice of the disclosure, and any such sequence may be employed. Preferred embodiments include the SV40 polyadenylation signal or the bovine growth hormone polyadenylation signal, convenient and known to function well in various target cells. Polyadenylation may increase the stability of the transcript or may facilitate cytoplasmic transport.

### 7. Origins of Replication

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

### 8. Selectable and Screenable Markers

In certain embodiments of the disclosure, cells containing a nucleic acid construct of the present disclosure may be identified *in vitro* or *in vivo* by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase (*tk*) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

### 9. Plasmid Vectors

In certain embodiments, a plasmid vector is contemplated for use to transform a host cell. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, E. coli is often transformed using derivatives of pBR322, a plasmid derived from an E. coli species. pBR322 contains genes for ampicillin and tetracycline resistance and thus provides easy means for identifying transformed cells. The pBR plasmid, or other microbial plasmid or phage must also contain, or be modified to contain, for example, promoters which can be used by the microbial organism for expression of its own proteins.

In addition, phage vectors containing replicon and control sequences that are compatible with the host microorganism can be used as transforming vectors in connection with these hosts. For example, the phage lambda GEM^{™}-11 may be utilized in making a recombinant phage vector which can be used to transform host cells, such as, for example, E. coli LE392.

Genomic integrated plasmids, such as piggybac or sleeping beauty transposon gene delivery plasmids, may be employed for long term transgenic expression of ANGPTL8 gene in heart or other organ.

Further useful plasmid vectors include pIN vectors (Inouye *et al.,* 1985); and pGEX vectors, for use in generating glutathione S-transferase (GST) soluble fusion proteins for later purification and separation or cleavage. Other suitable fusion proteins are those with β-galactosidase, ubiquitin, and the like.

Bacterial host cells, for example, E. coli, comprising the expression vector, are grown in any of a number of suitable media, for example, LB. The expression of the recombinant protein in certain vectors may be induced, as would be understood by those of skill in the art, by contacting a host cell with an agent specific for certain promoters, e.g., by adding IPTG to the media or by switching incubation to a higher temperature. After culturing the bacteria for a further period, generally of between 2 and 24 h, the cells are collected by centrifugation and washed to remove residual media.

### 10. Viral Vectors

The ability of certain viruses to infect cells or enter cells *via* receptor-mediated endocytosis, and to integrate into host cell genome and express viral genes stably and efficiently have made them attractive candidates for the transfer of foreign nucleic acids into cells (*e.g*., mammalian cells). Non-limiting examples of virus vectors that may be used to deliver a nucleic acid of the present disclosure are described below.

### a. Adenoviral Vectors

A particular method for delivery of the nucleic acid involves the use of an adenovirus expression vector. Although adenovirus vectors are known to have a low capacity for integration into genomic DNA, this feature is counterbalanced by the high efficiency of gene transfer afforded by these vectors. "Adenovirus expression vector" is meant to include those constructs containing adenovirus sequences sufficient to (a) support packaging of the construct and (b) to ultimately express a tissue or cell-specific construct that has been cloned therein. Knowledge of the genetic organization or adenovirus, a 36 kb, linear, double-stranded DNA virus, allows substitution of large pieces of adenoviral DNA with foreign sequences up to 7 kb (Grunhaus and Horwitz, 1992).

### b. AAV Vectors

The nucleic acid may be introduced into the cell using adenovirus assisted transfection. Increased transfection efficiencies have been reported in cell systems using adenovirus coupled systems (Kelleher and Vos, 1994; Cotten *et al.,* 1992; Curiel, 1994). Adeno-associated virus (AAV) is an attractive vector system for use in embodiments of the present disclosure as it has a high frequency of integration and it can infect nondividing cells, thus making it useful for delivery of genes into mammalian cells, for example, in tissue culture (Muzyczka, 1992) or *in vivo.* AAV has a broad host range for infectivity (Tratschin *et al.,* 1984; Laughlin *et al.,* 1986; Lebkowski *et al.,* 1988; McLaughlin *et al.,* 1988). Details concerning the generation and use of rAAV vectors are described in U.S. Patent Nos. 5,139,941 and 4,797,368.

### c. Retroviral Vectors

Retroviruses have promise as delivery vectors due to their ability to integrate their genes into the host genome, transferring a large amount of foreign genetic material, infecting a broad spectrum of species and cell types and of being packaged in special cell-lines (Miller, 1992).

In order to construct a retroviral vector, a nucleic acid is inserted into the viral genome in the place of certain viral sequences to produce a virus that is replication-defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into a special cell line *(e.g.,* by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

Lentiviruses are complex retroviruses, which, in addition to the common retroviral genes *gag, pol,* and *env,* contain other genes with regulatory or structural function. Lentiviral vectors are well known in the art (see, for example, Naldini *et al.,* 1996; Zufferey *et al.,* 1997; Blomer *et al.,* 1997; U.S. Pat. Nos. 6,013,516 and 5,994,136). Some examples of lentivirus include the Human Immunodeficiency Viruses: HIV-1, HIV-2 and the Simian Immunodeficiency Virus: SIV. Lentiviral vectors have been generated by multiply attenuating the HIV virulence genes, for example, the genes *env, vif, vpr, vpu* and *nef* are deleted making the vector biologically safe.

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136. One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific.

### d. Other Viral Vectors

Other viral vectors may be employed as vaccine constructs in the present disclosure. Vectors derived from viruses such as vaccinia virus (Ridgeway, 1988; Baichwal and Sugden, 1986; Coupar *et al.,* 1988), sindbis virus, cytomegalovirus and herpes simplex virus may be employed. They offer several attractive features for various mammalian cells (Friedmann, 1989; Ridgeway, 1988; Baichwal and Sugden, 1986; Coupar *et al.,* 1988; Horwich *et al.,* 1990).

### 11. Delivery Using Modified Viruses

A nucleic acid to be delivered may be housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. This modification can permit the specific infection of hepatocytes *via* sialoglycoprotein receptors.

Another approach to targeting of recombinant retroviruses was designed in which biotinylated antibodies against a retroviral envelope protein and against a specific cell receptor were used. The antibodies were coupled *via* the biotin components by using streptavidin (Roux *et al.,* 1989). Using antibodies against major histocompatibility complex class I and class II antigens, they demonstrated the infection of a variety of human cells that bore those surface antigens with an ecotropic virus *in vitro* (Roux *et al.,* 1989).

### B. Vector Delivery and Cell Transformation

Suitable methods for nucleic acid delivery for transformation of an organelle, a cell, a tissue or an organism for use with the current disclosure are believed to include virtually any method by which a nucleic acid *(e.g.,* DNA) can be introduced into an organelle, a cell, a tissue or an organism, as described herein or as would be known to one of ordinary skill in the art. Such methods include, but are not limited to, direct delivery of DNA such as by *ex vivo* transfection (Wilson *et al.,* 1989, Nabel *et al,* 1989), by injection (U.S. Patent Nos. 5,994,624, 5,981,274, 5,945,100, 5,780,448, 5,736,524, 5,702,932, 5,656,610, 5,589,466 and 5,580,859), including microinjection (Harlan and Weintraub, 1985; U.S. Patent No. 5,789,215); by electroporation (U.S. Patent No. 5,384,253; Tur-Kaspa *et al.,* 1986; Potter *et al.,* 1984); by calcium phosphate precipitation (Graham and Van Der Eb, 1973; Chen and Okayama, 1987; Rippe *et al.,* 1990); by using DEAE-dextran followed by polyethylene glycol (Gopal, 1985); by direct sonic loading (Fechheimer *et al.,* 1987); by liposome mediated transfection (Nicolau and Sene, 1982; Fraley *et al.,* 1979; Nicolau *et al.,* 1987; Wong *et al.,* 1980; Kaneda *et al.,* 1989; Kato *et al.,* 1991) and receptor-mediated transfection (Wu and Wu, 1987; Wu and Wu, 1988); by microprojectile bombardment (PCT Application Nos. WO 94/09699 and 95/06128; U.S. Patent Nos. 5,610,042; 5,322,783 5,563,055, 5,550,318, 5,538,877 and 5,538,880); by agitation with silicon carbide fibers (Kaeppler *et al.,* 1990; U.S. Patent Nos. 5,302,523 and 5,464,765); by *Agrobacterium-mediated* transformation (U.S. Patent Nos. 5,591,616 and 5,563,055); by PEG-mediated transformation of protoplasts (Omirulleh *et al.,* 1993; U.S. Patent Nos. 4,684,611 and 4,952,500); by desiccation/inhibition-mediated DNA uptake (Potrykus *et al.,* 1985), and any combination of such methods. Through the application of techniques such as these, organelle(s), cell(s), tissue(s) or organism(s) may be stably or transiently transformed.

### 1. Ex vivo Transformation

Methods for transfecting vascular cells and tissues removed from an organism in an *ex vivo* setting are known to those of skill in the art. For example, canine endothelial cells have been genetically altered by retroviral gene transfer *in vitro* and transplanted into a canine (Wilson *et al.,* 1989). In another example, yucatan minipig endothelial cells were transfected by retrovirus *in vitro* and transplanted into an artery using a double-balloon catheter (Nabel *et al.,* 1989). Thus, it is contemplated that cells or tissues may be removed and transfected *ex vivo* using the nucleic acids of the present disclosure. In particular aspects, the transplanted cells or tissues may be placed into an organism. In preferred facets, a nucleic acid is expressed in the transplated cells or tissues.

### 2. Injection

In certain aspects, a nucleic acid may be delivered to an organelle, a cell, a tissue or an organism *via* one or more injections (*i.e.,* a needle injection), such as, for example, subcutaneously, intradermally, intramuscularly, intervenously, intraperitoneally, *etc.* Methods of injection of vaccines are well known to those of ordinary skill in the art (*e.g.,* injection of a composition comprising a saline solution). Further aspects of the present disclosure include the introduction of a nucleic acid by direct microinjection. Direct microinjection has been used to introduce nucleic acid constructs into *Xenopus* oocytes (Harland and Weintraub, 1985).

### 3. Electroporation

In certain aspects of the present disclosure, a nucleic acid is introduced into an organelle, a cell, a tissue or an organism *via* electroporation. Electroporation involves the exposure of a suspension of cells and DNA to a high-voltage electric discharge. In some variants of this method, certain cell wall-degrading enzymes, such as pectin-degrading enzymes, are employed to render the target recipient cells more susceptible to transformation by electroporation than untreated cells (U.S. Patent No. 5,384,253). Alternatively, recipient cells can be made more susceptible to transformation by mechanical wounding.

Transfection of eukaryotic cells using electroporation has been quite successful. Mouse pre-B lymphocytes have been transfected with human kappa-immunoglobulin genes (Potter *et al.,* 1984), and rat hepatocytes have been transfected with the chloramphenicol acetyltransferase gene (Tur-Kaspa *et al.,* 1986) in this manner.

To effect transformation by electroporation in cells such as, for example, plant cells, one may employ either friable tissues, such as a suspension culture of cells or embryogenic callus or alternatively one may transform immature embryos or other organized tissue directly. In this technique, one would partially degrade the cell walls of the chosen cells by exposing them to pectin-degrading enzymes (pectolyases) or mechanically wounding in a controlled manner. Examples of some species which have been transformed by electroporation of intact cells include maize (U.S. Patent No. 5,384,253; Rhodes *et al.,* 1995; D'Halluin *et al.,* 1992), wheat (Zhou *et al.,* 1993), tomato (Hou and Lin, 1996), soybean (Christou *et al.,* 1987) and tobacco (Lee *et al.,* 1989).

One also may employ protoplasts for electroporation transformation of plant cells (Bates, 1994; Lazzeri, 1995). For example, the generation of transgenic soybean plants by electroporation of cotyledon-derived protoplasts is described by Dhir and Widholm in International Patent Application No. WO 9217598. Other examples of species for which protoplast transformation has been described include barley (Lazerri, 1995), sorghum (Battraw *et al.,* 1991), maize (Bhattacharjee *et al.,* 1997), wheat (He *et al.,* 1994) and tomato (Tsukada, 1989).

### 4. Calcium Phosphate

In other aspects of the present disclosure, a nucleic acid is introduced to the cells using calcium phosphate precipitation. Human KB cells have been transfected with adenovirus 5 DNA (Graham and Van Der Eb, 1973) using this technique. Also in this manner, mouse L(A9), mouse C127, CHO, CV-1, BHK, NIH3T3 and HeLa cells were transfected with a neomycin marker gene (Chen and Okayama, 1987), and rat hepatocytes were transfected with a variety of marker genes (Rippe *et al.,* 1990).

### 5. DEAE-Dextran

In another aspects, a nucleic acid is delivered into a cell using DEAE-dextran followed by polyethylene glycol. In this manner, reporter plasmids were introduced into mouse myeloma and erythroleukemia cells (Gopal, 1985).

### 6. Sonication Loading

Additional aspects of the present disclosure include the introduction of a nucleic acid by direct sonic loading. LTK- fibroblasts have been transfected with the thymidine kinase gene by sonication loading (Fechheimer *et al.,* 1987).

### 7. Liposome-Mediated Transfection

In a further embodiment of the disclosure, a nucleic acid may be entrapped in a lipid complex such as, for example, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated is an nucleic acid complexed with Lipofectamine (Gibco BRL) or Superfect (Qiagen).

Liposome-mediated nucleic acid delivery and expression of foreign DNA *in vitro* has been very successful (Nicolau and Sene, 1982; Fraley *et al.,* 1979; Nicolau *et al.,* 1987). The feasibility of liposome-mediated delivery and expression of foreign DNA in cultured chick embryo, HeLa and hepatoma cells has also been demonstrated (Wong *et al.,* 1980).

In certain embodiments of the disclosure, a liposome may be complexed with a hemagglutinating virus (HVJ). This has been shown to facilitate fusion with the cell membrane and promote cell entry of liposome-encapsulated DNA (Kaneda *et al.,* 1989). In other embodiments, a liposome may be complexed or employed in conjunction with nuclear non-histone chromosomal proteins (HMG-1) (Kato *et al.,* 1991). In yet further embodiments, a liposome may be complexed or employed in conjunction with both HVJ and HMG-1. In other embodiments, a delivery vehicle may comprise a ligand and a liposome.

### 8. Receptor Mediated Transfection

Still further, a nucleic acid may be delivered to a target cell *via* receptor-mediated delivery vehicles. These take advantage of the selective uptake of macromolecules by receptor-mediated endocytosis that will be occurring in a target cell. In view of the cell type-specific distribution of various receptors, this delivery method adds another degree of specificity to the present disclosure.

Certain receptor-mediated gene targeting vehicles comprise a cell receptor-specific ligand and a nucleic acid-binding agent. Others comprise a cell receptor-specific ligand to which the nucleic acid to be delivered has been operatively attached. Several ligands have been used for receptor-mediated gene transfer (Wu and Wu, 1987; Wagner *et al.,* 1990; Perales *et al.,* 1994; Myers, EPO 0273085), which establishes the operability of the technique. Specific delivery in the context of another mammalian cell type has been described (Wu and Wu, 1993). In certain aspects of the present disclosure, a ligand will be chosen to correspond to a receptor specifically expressed on the target cell population.

In other embodiments, a nucleic acid delivery vehicle component of a cell-specific nucleic acid targeting vehicle may comprise a specific binding ligand in combination with a liposome. The nucleic acid(s) to be delivered are housed within the liposome and the specific binding ligand is functionally incorporated into the liposome membrane. The liposome will thus specifically bind to the receptor(s) of a target cell and deliver the contents to a cell. Such systems have been shown to be functional using systems in which, for example, epidermal growth factor (EGF) is used in the receptor-mediated delivery of a nucleic acid to cells that exhibit upregulation of the EGF receptor.

In still further embodiments, the nucleic acid delivery vehicle component of a targeted delivery vehicle may be a liposome itself, which will preferably comprise one or more lipids or glycoproteins that direct cell-specific binding. For example, lactosyl-ceramide, a galactose-terminal asialganglioside, have been incorporated into liposomes and observed an increase in the uptake of the insulin gene by hepatocytes (Nicolau *et al.,* 1987). It is contemplated that the tissue-specific transforming constructs of the present disclosure can be specifically delivered into a target cell in a similar manner.

### 9. Microprojectile Bombardment

Microprojectile bombardment techniques can be used to introduce a nucleic acid into at least one, organelle, cell, tissue or organism (U.S. Patent No. 5,550,318; U.S. Patent No. 5,538,880; U.S. Patent No. 5,610,042; and PCT Application WO 94/09699). This method depends on the ability to accelerate DNA-coated microprojectiles to a high velocity allowing them to pierce cell membranes and enter cells without killing them (Klein *et al.,* 1987). There are a wide variety of microprojectile bombardment techniques known in the art, many of which are applicable to the disclosure.

Microprojectile bombardment may be used to transform various cell(s), tissue(s) or organism(s), such as for example any plant species. Examples of species which have been transformed by microprojectile bombardment include monocot species such as maize (PCT Application WO 95/06128), barley (Ritala *et al.,* 1994; Hensgens *et al.,* 1993), wheat (U.S. Patent No. 5,563,055), rice (Hensgens *et al.,* 1993), oat (Torbet *et al.,* 1995; Torbet *et al.,* 1998), rye (Hensgens *et al.,* 1993), sugarcane (Bower *et al.,* 1992), and sorghum (Casas *et al.,* 1993; Hagio *et al.,* 1991); as well as a number of dicots including tobacco (Tomes *et al.,* 1990; Buising and Benbow, 1994), soybean (U.S. Patent No. 5,322,783), sunflower (Knittel *et al.* 1994), peanut (Singsit *et al.,* 1997), cotton (McCabe and Martinell, 1993), tomato (VanEck *et al.* 1995), and legumes in general (U.S. Patent No. 5,563,055).

In this microprojectile bombardment, one or more particles may be coated with at least one nucleic acid and delivered into cells by a propelling force. Several devices for accelerating small particles have been developed. One such device relies on a high voltage discharge to generate an electrical current, which in turn provides the motive force (Yang *et al.,* 1990). The microprojectiles used have consisted of biologically inert substances such as tungsten or gold particles or beads. Exemplary particles include those comprised of tungsten, platinum, and preferably, gold. It is contemplated that in some instances DNA precipitation onto metal particles would not be necessary for DNA delivery to a recipient cell using microprojectile bombardment. However, it is contemplated that particles may contain DNA rather than be coated with DNA. DNA-coated particles may increase the level of DNA delivery *via* particle bombardment but are not, in and of themselves, necessary.

For the bombardment, cells in suspension are concentrated on filters or solid culture medium. Alternatively, immature embryos or other target cells may be arranged on solid culture medium. The cells to be bombarded are positioned at an appropriate distance below the macroprojectile stopping plate.

An illustrative example of a method for delivering DNA into a cell *(e.g.,* a plant cell) by acceleration is the Biolistics Particle Delivery System, which can be used to propel particles coated with DNA or cells through a screen, such as a stainless steel or Nytex screen, onto a filter surface covered with cells, such as for example, a monocot plant cells cultured in suspension. The screen disperses the particles so that they are not delivered to the recipient cells in large aggregates. It is believed that a screen intervening between the projectile apparatus and the cells to be bombarded reduces the size of projectiles aggregate and may contribute to a higher frequency of transformation by reducing the damage inflicted on the recipient cells by projectiles that are too large.

### C. Host Cells

As used herein, the terms "cell," "cell line," and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, "host cell" refers to a prokaryotic or eukaryotic cell, and it includes any transformable organism that is capable of replicating a vector and/or expressing a heterologous gene encoded by a vector. A host cell can, and has been, used as a recipient for vectors. A host cell may be "transfected" or "transformed," which refers to a process by which exogenous nucleic acid is transferred or introduced into the host cell. A transformed cell includes the primary subject cell and its progeny. As used herein, the terms "engineered" and "recombinant" cells or host cells are intended to refer to a cell into which an exogenous nucleic acid sequence, such as, for example, a vector, has been introduced. Therefore, recombinant cells are distinguishable from naturally occurring cells which do not contain a recombinantly introduced nucleic acid.

In certain aspects, it is contemplated that RNAs or proteinaceous sequences may be co-expressed with other selected RNAs or proteinaceous sequences in the same host cell. Co-expression may be achieved by co-transfecting the host cell with two or more distinct recombinant vectors. Alternatively, a single recombinant vector may be constructed to include multiple distinct coding regions for RNAs, which could then be expressed in host cells transfected with the single vector.

A tissue may comprise a host cell or cells to be transformed with a polynucleotide encoding part or all of ANGPTL8. The tissue may be part or separated from an organism. In certain aspects, a tissue may comprise, but is not limited to, myocytes, adipocytes, alveolar, ameloblasts, axon, basal cells, blood (*e.g*., lymphocytes), blood vessel, bone, bone marrow, brain, breast, cartilage, cervix, colon, cornea, embryonic, endometrium, endothelial, epithelial, esophagus, facia, fibroblast, follicular, ganglion cells, glial cells, goblet cells, kidney, liver, lung, lymph node, muscle, neuron, ovaries, pancreas, peripheral blood, prostate, skin, skin, small intestine, spleen, stem cells, stomach, testes, anthers, ascite tissue, cobs, ears, flowers, husks, kernels, leaves, meristematic cells, pollen, root tips, roots, silk, stalks, and all cancers thereof.

In certain aspects, the host cell or tissue may be comprised in at least one organism. In certain aspects, the organism may be, but is not limited to, a prokayote (*e.g.*, a eubacteria, an archaea) or an eukaryote, as would be understood by one of ordinary skill in the art (see, for example, webpage http://phylogeny.arizona.edu/tree/phylogeny.html).

Numerous cell lines and cultures are available for use as a host cell, and they can be obtained through the American Type Culture Collection (ATCC), which is an organization that serves as an archive for living cultures and genetic materials (www.atcc.org). An appropriate host can be determined by one of skill in the art based on the vector backbone and the desired result. A plasmid or cosmid, for example, can be introduced into a prokaryote host cell for replication of many vectors. Cell types available for vector replication and/or expression include, but are not limited to, bacteria, such as *E. coli* (*e.g., E. coli* strain RR1, *E. coli* LE392, *E. coli* B, *E. coli* X 1776 (ATCC No. 31537) as well as *E*. *coli* W3110 (F-, lambda-, prototrophic, ATCC No. 273325), DH5α, JM109, and KC8, bacilli such as *Bacillus subtilis;* and other enterobacteriaceae such as *Salmonella typhimurium, Serratia marcescens,* various *Pseudomonas* specie, as well as a number of commercially available bacterial hosts such as SURE^{®} Competent Cells and Solopack^{™} Gold Cells (Stratagene^{®}, La Jolla). In certain aspects, bacterial cells such as *E. coli* LE392 are particularly contemplated as host cells for phage viruses.

Examples of eukaryotic host cells for replication and/or expression of a vector include, but are not limited to, HeLa, NIH3T3, Jurkat, 293, Cos, CHO, Saos, and PC12. Many host cells from various cell types and organisms are available and would be known to one of skill in the art. Similarly, a viral vector may be used in conjunction with either a eukaryotic or prokaryotic host cell, particularly one that is permissive for replication or expression of the vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

### D. Expression Systems

Numerous expression systems exist that comprise at least a part or all of the compositions discussed above. Prokaryote- and/or eukaryote-based systems can be employed for use with the present disclosure to produce nucleic acid sequences, or their cognate polypeptides, proteins and peptides. Many such systems are commercially and widely available.

The insect cell/baculovirus system can produce a high level of protein expression of a heterologous nucleic acid segment, such as described in U.S. Patent No. 5,871,986, 4,879,236, and which can be bought, for example, under the name MaxBac^{®} 2.0 from Invitrogen^{®} and BacPack^{™} Baculovirus Expression System From Clontech^{®}.

Other examples of expression systems include Stratagene^{®}'s Complete Control^{™} Inducible Mammalian Expression System, which involves a synthetic ecdysoneinducible receptor, or its pET Expression System, an *E. coli* expression system. Another example of an inducible expression system is available from Invitrogen^{®}, which carries the T-Rex^{™} (tetracycline-regulated expression) System, an inducible mammalian expression system that uses the full-length CMV promoter. Invitrogen^{®} also provides a yeast expression system called the *Pichia methanolica* Expression System, which is designed for high-level production of recombinant proteins in the methylotrophic yeast *Pichia methanolica.* One of skill in the art would know how to express a vector, such as an expression construct, to produce a nucleic acid sequence or its cognate polypeptide, protein, or peptide.

It is contemplated that the proteins, polypeptides or peptides produced by the methods of the disclosure may be "overexpressed", *i.e.,* expressed in increased levels relative to its natural expression in cells. Such overexpression may be assessed by a variety of methods, including radio-labeling and/or protein purification. However, simple and direct methods are preferred, for example, those involving SDS/PAGE and protein staining or western blotting, followed by quantitative analyses, such as densitometric scanning of the resultant gel or blot. A specific increase in the level of the recombinant protein, polypeptide or peptide in comparison to the level in natural cells is indicative of overexpression, as is a relative abundance of the specific protein, polypeptides or peptides in relation to the other proteins produced by the host cell and, e.g., visible on a gel.

In some aspects, the expressed proteinaceous sequence forms an inclusion body in the host cell, the host cells are lysed, for example, by disruption in a cell homogenizer, washed and/or centrifuged to separate the dense inclusion bodies and cell membranes from the soluble cell components. This centrifugation can be performed under conditions whereby the dense inclusion bodies are selectively enriched by incorporation of sugars, such as sucrose, into the buffer and centrifugation at a selective speed. Inclusion bodies may be solubilized in solutions containing high concentrations of urea *(e.g.* 8M) or chaotropic agents such as guanidine hydrochloride in the presence of reducing agents, such as β-mercaptoethanol or DTT (dithiothreitol), and refolded into a more desirable conformation, as would be known to one of ordinary skill in the art.

### E. Proteins, Polypeptides, and Peptides

In some cases, embodiments may utilize purified ANGPTL8 proteins, polypeptides, or peptides. The term "purified proteins, polypeptides, or peptides" as used herein, is intended to refer to an proteinaceous composition, isolatable from mammalian cells or recombinant host cells, wherein the at least one protein, polypeptide, or peptide is purified to any degree relative to its naturally-obtainable state, *i.e.,* relative to its purity within a cellular extract. A purified protein, polypeptide, or peptide therefore also refers to a wild-type or mutant protein, polypeptide, or peptide free from the environment in which it naturally occurs.

The nucleotide and protein, polypeptide and peptide sequences for various genes have been previously disclosed, and may be found at computerized databases known to those of ordinary skill in the art. One such database is the National Center for Biotechnology Information's GenBank^{®} and GenPept^{®} databases. The coding regions for these known genes may be amplified and/or expressed using the techniques disclosed herein or by any technique that would be known to those of ordinary skill in the art. Additionally, peptide sequences may be sythesized by methods known to those of ordinary skill in the art, such as peptide synthesis using automated peptide synthesis machines, such as those available from Applied Biosystems (Foster City, CA).

Generally, "purified" will refer to a specific protein, polypeptide, or peptide composition that has been subjected to fractionation to remove various other proteins, polypeptides, or peptides, and which composition substantially retains its activity, as may be assessed, for example, by the protein assays, as described herein below, or as would be known to one of ordinary skill in the art for the desired protein, polypeptide or peptide.

Where the term "substantially purified" is used, this will refer to a composition in which the specific protein, polypeptide, or peptide forms the major component of the composition, such as constituting about 50% of the proteins in the composition or more. In preferred embodiments, a substantially purified protein will constitute more than 60%, 70%, 80%, 90%, 95%, 99% or even more of the proteins in the composition.

A peptide, polypeptide or protein that is "purified to homogeneity," as applied to the present disclosure, means that the peptide, polypeptide or protein has a level of purity where the peptide, polypeptide or protein is substantially free from other proteins and biological components. For example, a purified peptide, polypeptide or protein will often be sufficiently free of other protein components so that degradative sequencing may be performed successfully.

Various methods for quantifying the degree of purification of proteins, polypeptides, or peptides will be known to those of skill in the art in light of the present disclosure. These include, for example, determining the specific protein activity of a fraction, or assessing the number of polypeptides within a fraction by gel electrophoresis.

To purify a desired protein, polypeptide, or peptide a natural or recombinant composition comprising at least some specific proteins, polypeptides, or peptides will be subjected to fractionation to remove various other components from the composition. In addition to those techniques described in detail herein below, various other techniques suitable for use in protein purification will be well known to those of skill in the art. These include, for example, precipitation with ammonium sulfate, PEG, antibodies and the like or by heat denaturation, followed by centrifugation; chromatography steps such as ion exchange, gel filtration, reverse phase, hydroxylapatite, lectin affinity and other affinity chromatography steps; isoelectric focusing; gel electrophoresis; and combinations of such and other techniques.

Another example is the purification of a specific fusion protein using a specific binding partner. Such purification methods are routine in the art. As the present disclosure provides DNA sequences for the specific proteins, any fusion protein purification method can now be practiced. This is exemplified by the generation of an specific protein-glutathione S-transferase fusion protein, expression in *E. coli,* and isolation to homogeneity using affinity chromatography on glutathione-agarose or the generation of a polyhistidine tag on the N- or C-terminus of the protein, and subsequent purification using Ni-affinity chromatography. However, given many DNA and proteins are known, or may be identified and amplified using the methods described herein, any purification method can now be employed.

Although preferred for use in certain embodiments, there is no general requirement that the protein, polypeptide, or peptide always be provided in their most purified state. Indeed, it is contemplated that less substantially purified protein, polypeptide or peptide, which are nonetheless enriched in the desired protein compositions, relative to the natural state, will have utility in certain embodiments.

Methods exhibiting a lower degree of relative purification may have advantages in total recovery of protein product, or in maintaining the activity of an expressed protein. Inactive products also have utility in certain embodiments, such as, *e.g.,* in determining antigenicity *via* antibody generation.

### VI. Combination Therapy

In certain cases, the ANGPTL8 therapy of the present disclosure may be utilized in conjunction with one or more other therapies for a cardiac-related medical condition. ANGPTL8 may be used in combination with other genes or gene products in peptide, protein, or nucleic acid form, or the other agent may be a small molecule, for example. The one or more other therapies may be directly or indirectly related to the cardiac-related medical condition (examples of indirectly related therapies include those for pain or infection).

The ANGPTL8 therapy may precede or follow the other agent treatment by intervals ranging from minutes to hours to days to weeks or months. When the other agent and the ANGPTL8 therapy are applied separately to the individual, one would generally ensure that a significant period of time did not expire between the time of each delivery, such that the agent and ANGPTL8 therapy would still be able to exert an advantageously combined effect on the cell. In such instances, it is contemplated that one may contact the individual with both modalities simultaneously or within minutes of each other or within about 1-12, 6-12, or 12-24 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

Yet further, ANGPTL8 may also include other nucleic acids or peptides. In such instances, ANGPTL8 therapy may also be used in combination may precede or follow the other agent treatment by intervals ranging from minutes to weeks or months. When the other agent and the ANGPTL8 therapy are applied separately to the individual, one would generally ensure that a significant period of time did not expire between the time of each delivery, such that the agent and ANGPTL8 therapy would still be able to exert an advantageously combined effect on the cell. In such instances, it is contemplated that one may contact the individual with both modalities simultaneously or within minutes of each other or within about 1-12, 6-12, or 12-24 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In specific aspects, ANGPTL8 therapy and another agent are provided at the same time or at different times. The ANGPTL8 therapy and the other agent may be within the same plurality of microbubbles or they may be comprised in separate pluralities of microbubbles. In cases wherein the ANGPTL8 therapy and the other agent are provided at different times, they may be separated by any suitable range in times, such as minutes, hours, days, or weeks. In cases wherein they are provided separately, the order of delivery of ANGPTL8 therapy and the other therapy may be of any suitable order, including delivery of ANGPTL8 prior to or subsequent to the other therapy. In cases wherein ANGPTL8 therapy and the other therapy are provided to an individual in need thereof, they may be provided with yet another therapy for a cardiac-related medical condition.

Examples of other treatments to be employed with the ANGPTL8 and/or another therapy includes one or more of the following: ACE Inhibitors, Aldosterone Inhibitor, Angiotensin II Receptor Blocker (ARBs); Beta-Blockers, Calcium Channel Blockers, Cholesterol-Lowering Drugs, Digoxin, Diuretics, Inotropic Therapy, Potassium or Magnesium, Vasodilators, anticoagulant medication, aspirin, or a combination thereof.

### VII. Kits of the Disclosure

Any of the compositions described herein may be comprised in a kit. In a non-limiting example, ANGPTL8 (or ANGPTL1, ANGPTL2, ANGPTL3, ANGPTL4, ANGPTL5, ANGPTL6, and/orANGPTL7) polynucleotide or primers for amplification of it may be comprised in a kit. In specific aspects, the kit comprises ANGPTL8 peptides or polypeptides. The kit may alternatively or additionally comprise reagents for generating liposomes and/or microbubbles and optionally may have additional agents for therapy of a cardiac-related medical condition.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the one or more compositions in close confinement for commercial sale. Such containers may include injection or blow-molded plastic containers into which the desired vials are retained.

The composition may be formulated into a syringeable composition. In which case, the container means may itself be a syringe, pipette, and/or other such like apparatus, from which the formulation may be applied to an infected area of the body, injected into an animal, and/or even applied to and/or mixed with the other components of the kit. However, the components of the kit may be provided as dried powder(s). When reagents and/or components are provided as a dry powder, the powder can be reconstituted by the addition of a suitable solvent. It is envisioned that the solvent may also be provided in another container means.

The kits of the present disclosure will also typically include a means for containing the vials in close confinement for commercial sale, such as, e.g., injection and/or blow-molded plastic containers into which the desired vials are retained.

In particular aspects, the kit comprises reagents and/or tools for determining that an individual has a cardiac-related medical condition. In some aspects, the kit comprises one or more additional therapies for a cardiac-related medical condition, such as one or more of ACE Inhibitor, aldosterone inhibitor, angiotensin II receptor blocker (ARBs); beta-blocker, calcium channel blocker, cholesterol-lowering drug, digoxin, diuretics, inotropic therapy, potassium, magnesium, vasodilator, anticoagulant medication, aspirin, and a combination thereof.

### EXAMPLES

The following examples are included to demonstrate preferred aspects of the invention.

### EXAMPLE 1

### EXAMPLES OF METHODS

### Animal protocols

Animal studies were performed according to National Institutes of Health (NIH) recommendations and approved by our institutional animal research committee. Adult male Sprague-Dawley rats were purchased from Harlan Laboratories (Indianapolis, IN, USA).

The protocol was planned to test the hypothesis that delivery of *ANGPTL8* by UTMD could reverse established adriamycin (ADM) cardiomyopathy defined as a fractional shortening < 30% by echocardiography after injection of ADM (Iliskovic, *et al.,* 1997) at total dose of 15 mg/kg/ip, 2.5mg/kg/ip 6 times over 2 weeks.

**Protocol-1 for *ANGTPL8* gene therapy:** There were 120 rats divided into three control groups of 30 rats each and a treatment group of 30 rats: (1) normal control rats; (2) ADM injection only; (3) ADM plus UTMD with a DsRed reporter gene (pXL-BASII-CI-DsRed/pCI-hyPB); (4) ADM plus UTMD with *ANGPTL8* (pXL-BASII-CI-*ANGTL8*/pCIhyPB). All rats were euthanized at 3 days, 7 days, 14 days, 21 days and 28 days after UTMD The thymidine analog, 5-bromo-2-deoxyuridine (BrdU) (100mg/kg) was injected intraperitoneally 6 hours prior to euthanizing.

Male Sprague-Dawley rats (230-270g) were anesthetized with intraperitoneal ketamine (60 mg/kg) and xylazine (5 mg/kg), and a polyethylene tube (PE 50, Becton Dickinson, Franklin Lakes, TN, USA) was inserted into the right internal jugular vein by cut-down. Piggybac transposon donor plasmids and helper plasmids ratio (pXL-BASII-CI-*ANGPTL8*/pCI-hyPB) was 5:1. Microbubble or control solutions (0.5 ml diluted with 0.5 ml phosphate-buffered solution (PBS)) were infused over 1 min 30 seconds via pump (Genie, Kent Scientific, Torrington, CT). During the infusion, ultrasound was directed to the liver using a commercially available ultrasound transducer (S3, Sonos 5500, Philips Ultrasound, Bothell, WA). Ultrasound was then applied in ultraharmonic mode (transmit 1.3 MHz/receive 3.6 MHz) at a mechanical index of 1.2. Four bursts of ultrasound were triggered to every fourth end-systole by electrocardiogram using a delay of 45-70 ms after the peak of the R wave. These settings have shown to be optimal for plasmid delivery by UTMD using this instrument (Chen *et al.,* 2003). Bubble destruction was visually apparent in all rats. After UTMD, the jugular vein was tied off, the skin closed, and the animals allowed to recover. All of rats were euthanized using an overdose of sodium pentobarbital (120 mg/kg).

**Protocol-2 for *ANGTPL8* protein therapy:** 15 rats divided into (1) normal control rats; (2) ADM plus Saline inject rats; (3) ADM plus *ANGPTL8* protein at dose of 5µg/kg/day intramuscular inject for 14 days; (4) ADM plus *ANGPTL8* protein at dose of 20µg/kg/day intramuscular inject for 14 days; (5) ADM plus *ANGPTL8* protein at dose of 40µg/kg/day intramuscular inject for 14 days; All rats were euthanized at 14 days after starting ANGTPL8 protein inject. The thymidine analog, 5-bromo-2-deoxyuridine (BrdU) (100mg/kg) was injected intraperitoneally 6 hours prior to euthanasia. Human recombinant protein ANGTPL8 protein was purchased from Phoenix Pharmaceuticals (Cat # 056-61, Burlingame, CA, USA).

### Manufacture of plasmid-containing lipid-stabilized microbubbles

Lipid-stabilized microbubbles were made as previously described [Iliskovic, *et al.,* 1997]. Briefly, 250 mg of 1,2-dipalmitoyl-*sn*-glycero-3-phosphocholine, 50 mg of 1,2-dipalmitoyl-*sn*-glycero-3-phosphoethanolamine and 10% glucose were mixed with PBS and boiled in water until fully dissolved. Next 2 mg of plasmid DNA was mixed with 0.5 ml of ethyl alcohol and centrifuged at 10,000 g for 5 min. The supernatant fraction was removed, and the DNA pellet was placed in an incubator at 37°C for 5 min to remove any remaining ethyl alcohol. The DNA was then added to 50 µl of Lipofectamine 2000 (1 mg/ml; Invitrogen, Carlsbad, CA, USA) and mixed for 20 min. This mixture was added to 250 µl of liposome solution, 5 µl of 10% albumin and 50 µl of glycerol (10 mg/ml) in 1.5 ml vials, and placed on ice. The headspace of the vials was filled with perfluoropropane gas, and the vials were then shaken for 30 seconds at 4°C. The mean diameter and concentration of the microbubbles were 1.9 ±0.2 µm and 5.2 ± 0.3 × 10⁹ bubbles per ml, respectively. The concentration of plasmid carried by the microbubbles was 250±10 µg/ml.

### Plasmid constructs

Human *ANGPTL8* cDNA with Sal1/Not1 cutting sites (Integrated DNA Technologies, Coralville, IA, USA) was subcloned into PiggyBac transposon plasmids (pXL-BSII donor plasmid) provided by Dr Fraser at the University of Notre Dame (Notre Dame, IN, USA) [36], and hyperactive piggyBac transposase helper plasmid was provided by Dr Bradley at Wellcome Trust Sanger Institute (Cambridge, UK) (Yusa, *et al.,* 2011). Cloning, isolation and purification of the plasmids were performed by standard procedures, and the PCR products were sequenced to confirm that no artefactual mutations were present.

### Immunohistochemistry

Tissue samples were fixed in 10% formalin for 24 hours and transferred into 70% alcohol for paraffin embedding and 4% paraformaldehyde and 20% sucrose overnight at 4 °C for frozen sections. Cryostat sections 5-8 µm in thickness were further fixed with acetone (-20°C) for 5 min and quenched for 5-20 min with 10 mM glycine in PBS. Sections were then rinsed in PBS 3 times, and permeabilized with 0.5% Triton X-100 in PBS for 15 min. The slides that needed further nuclear protein retrieval were subjected to boiling citrate buffer solution with tween 20 at pH 6.0 for 5 minutes. Sections were blocked with Aquablock solution (EastCoast Bio, North Berwick, ME) at room temperature for 1hr and washed with PBS 1 time. The primary antibodies rat anti-PirB, 1: 200 dilution (BD biosciences, San Jose, CA ) , rabbit anti-Notchl, 1:200, rabbit anti- FoxoO1, 1:250, and rabbit anti-ISL-1, 1: 500 dilution, and mouse anti-cardiac troponin T, 1: 250 dilution, rabbit anti-BrdU, 1:200 dilution, rabbit anti-human *ANGPTL8* at 1:100 dilution, rabbit anti-OCT4, 1:250 dilution, rabbit anti-Nanog, 1:250 dilution, rabbit anti-SOX2, 1:500 dilution, rabbit anti-albumin, 1:250 dilution (Abcam Inc, Cambridge, MA), anit-rabbit Smooth muscle actin-alpha , 1:500 dilution (Sigma, St. Louis, MO), anti-rabbit von Willebrand Factor, 1:200 dilution (Dako, Carpinteria, CA), mouse anti-human *ANGPTL8* 1:200 dilution (Phoenix Pharmaceuticals, Burlingame, CA, USA), were added and incubated for 2 hrs at RT or overnight at 4 °C. After washing with PBS three times for 5 min, the secondary antibody (Sigma, St; Louis, MO) anti-mouse lgG conjugated with FITC; anti-rabbit IgG-conjugated with Texas Red, or anti-donkey lgG conjugated with Cy5) (1:250 dilution in block solution) were added and incubated for 1 hr at RT. Sections were rinsed with PBS for 10 min, 3 times, and incubated with Dapi (Invitrogen, Carlsbad, CA), 1: 5000 dilution for 5 min and washed 3 times with PBST, then mounted. A confocal microscope was used to take pictures. BrdU staining included with incubating in HCl (IN) for 10 minutes on ice to break open the DNA structure of the labeled cells and then followed by HCl (2N) for 10 minutes at room temperature before moving them to an incubator for 20 minutes at 37°C, Immediately after the acid washes, Borate buffer (0.1M) is added to buffer the cells for 12 minutes.

### Culture of HL-1 Atria Muscle Cells line.

The HL-1 cell line was a generous gift from Dr. William C. Claycomb (Claycomb, *et al.,* 1998) in Louisiana State University Medical Center, New Orleans, LA. The cells were maintained in Claycomb basal medium (Sigma) supplemented with 10% fetal bovine serum, 0.1 mM norepinephrine and 2 mM L-glutamine. HL-1 cells were treated with adriamycin at 0 µM, 0.25 µM, 0.50 µM, or 1.00 µM for 48 hrs in complete growth medium, or add ANGPTL8 gene plasmids transfection with lipofectamin2000, or 2ng of ANGPTL8 peptide to a dish and were subjected to immunofluorescent staining.

### ELISA for detecting plasma ANGPTL8

Fasting venous blood was collected in EDTA tubes from the tail vein, and plasma lipids were measured using standard enzymatic assays. Human *ANGTPL8* was measured using ELISA (Phoenix Pharmaceuticals).

### RNA isolation and quantitative RT-PCR analysis

Total RNA was isolated from 100 mg of pancreas, liver and left quadratic skeletal muscle using the RNeasy mini kit (Qiagen). Real-time quantitative RT-PCR (qRT-PCR) analysis was performed on an ABI 7700 Sequence Detector (Applied Biosystems, Grand Island, NY, USA) using SYBR Green (RT2 SYBR Green qPCR Kit; Qiagen, Boston, MA, USA). Data were normalized to the expression of housekeeping genes (as an endogenous control). Changes in gene expression were normalized to control liver and cardiac muscle samples.

### Western blotting

Total protein extracts from pancreatic, liver and muscle tissue were evaluated with a Cytoplasmic Extraction Kit (Thermo Scientific, Rockford, IL, USA). Protein concentrations were determined using the BCA-200 Protein Assay kit (Pierce, Grand Island, NY, USA); equal amounts of protein were separated by SDS-PAGE to nitrocellulose membranes and incubated with primary antibodies ANGPTL8 (1:1,000 dilutions) and antiactin (1: 2,000 dilutions). Horseradish peroxidase secondary antibodies were used, and chemiluminescence was determined using the SuperSignal West Dura detection system (Pierce); a cytoplasmic marker (actin) was used to confirm equal loading. All Western blots were performed in duplicate.

### Data analysis

Data were analyzed using Statview software (SAS, Cary, NC, USA). The values are presented as mean ± SEM. Differences were analyzed by repeated measures ANOVA with Fisher's post hoc test and were considered significant at p<0.05.

### EXAMPLE 2

### UTMD TARGETING OF HUMAN ANGPTL8 GENE TO LIVER OF RATS

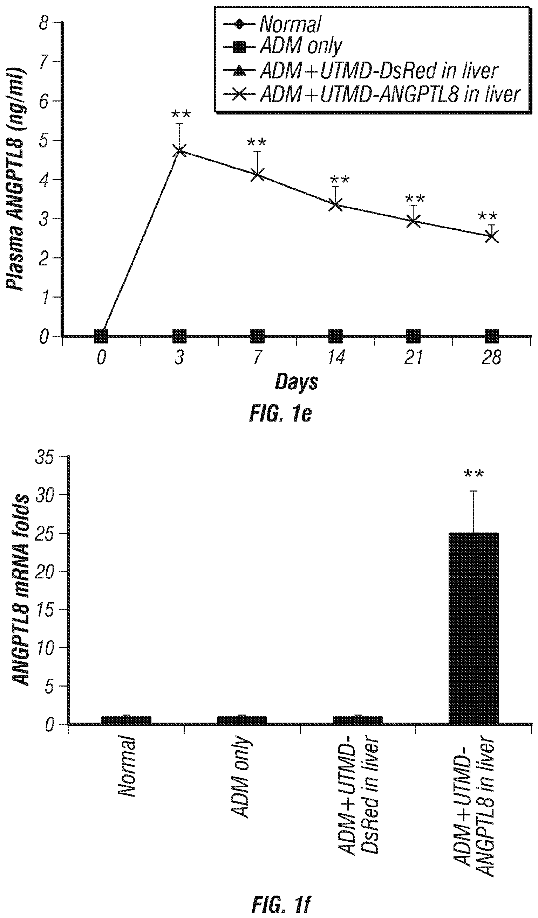

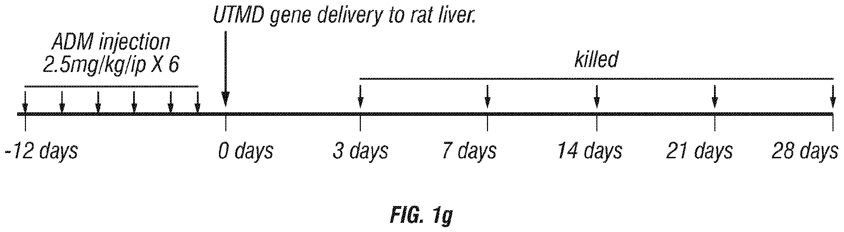

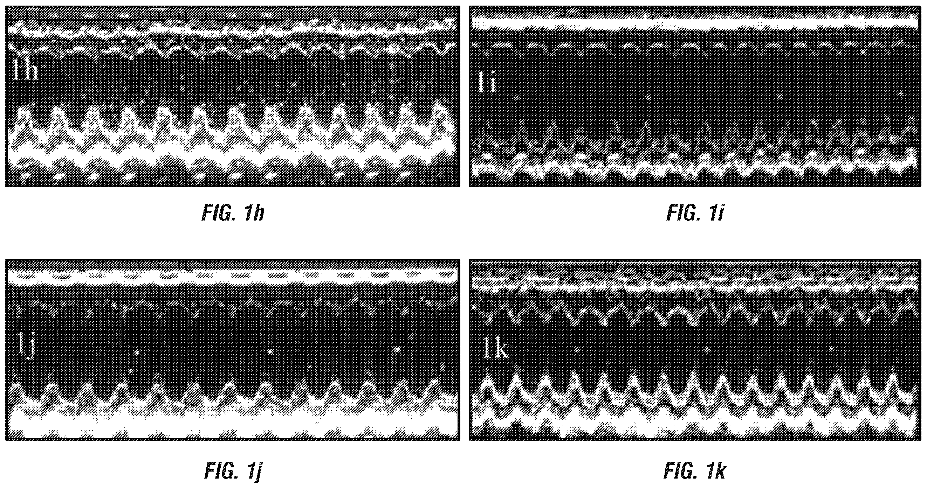

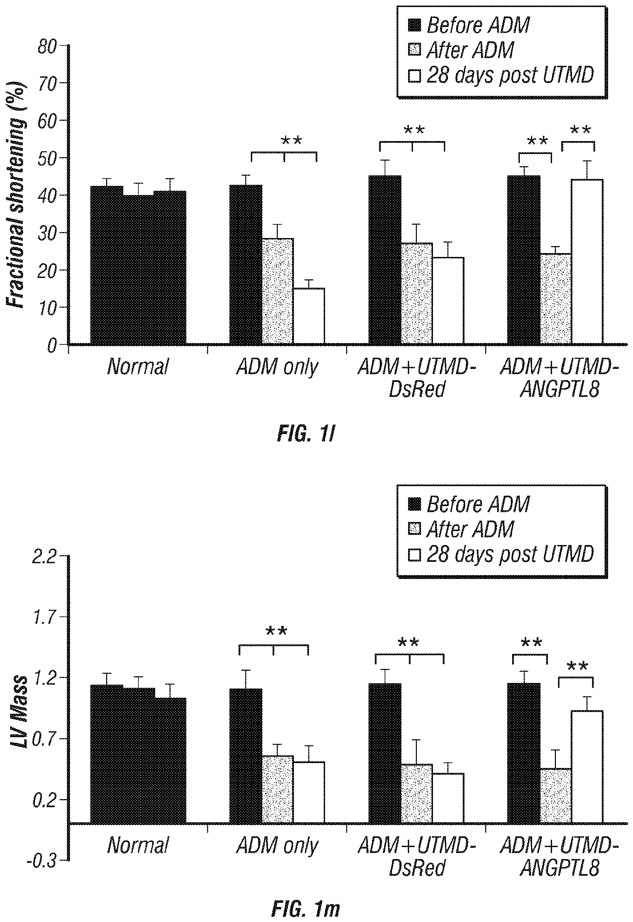

FIG. 1 shows that human *ANGPTL8* signal was detected in liver cells after UTMD-pXL-BASII-CI- *ANGPTL8*/pCI-hyPB delivered to the liver (FIG. 1d) but was not detected in normal rat liver, nor in the controls treated with Adriamycin (ADM) only and ADM plus UTMD-DsRed reporter gene (FIG. 1a-c). Human *ANGPTL8* signal was detected in the cytoplasm of liver cells of rats from 3 days to 4 weeks after delivery of the *ANGPTL8* gene to liver by UTMD. FIG. 1e shows fasting plasma levels of human *ANGPTL8* in rats. In the normal and *DsRed* reporter gene control rat groups, no human *ANGPTL8* was detected in fasting plasma. However, in the treatment groups, human *ANGPTL8* was detected in fasting plasma from day 1 to day 28 after UTMD. Human *ANGPTL8* mRNA levels were further evaluated using qRT-PCR. The results (FIG. If) showed that human *ANGPTL8* mRNA levels after *UTMD-ANGPTL8* gene delivery were 25± 8 fold greater, respectively, than for the normal, ADM only and ADM plus *UTMD-DsRed* control groups (p<0.001). Western blotting was employed to detect human *ANGPTL8* from liver protein extracts, and the results showed that the human *ANGPTL8* signal existed in the liver protein extracts after *UTMD-ANGPTL8* gene delivery.

### EXAMPLE 3

### REVERSAL OF ESTABLISHED ADRIAMYCIN CARDIOMYOPATHY AFTER UTMD-ANGPTL8 GENE THERAPY

Pharmacological effects of *ANGPTL8* on rat hearts with established adriamycin cardiomyopathy was characterized. Echocardiography was utilized to evaluate heart structure and function in all groups. FIG. 1h-k demonstrated M-mode images derived from 2D parasternal short axis views of the left ventricle showing decreased LV fractional shortening and LV mass in adriamycin cardiomyopathy with restoration to normal values by UTMD- *ANGPTL8* gene therapy (1 and m).

### EXAMPLE 4

### ANGPTL8 INDUCED THE ACTIVATION OF CARDIAC PROGENITOR CELLS IN EPICARDIAL LAYER CELLS

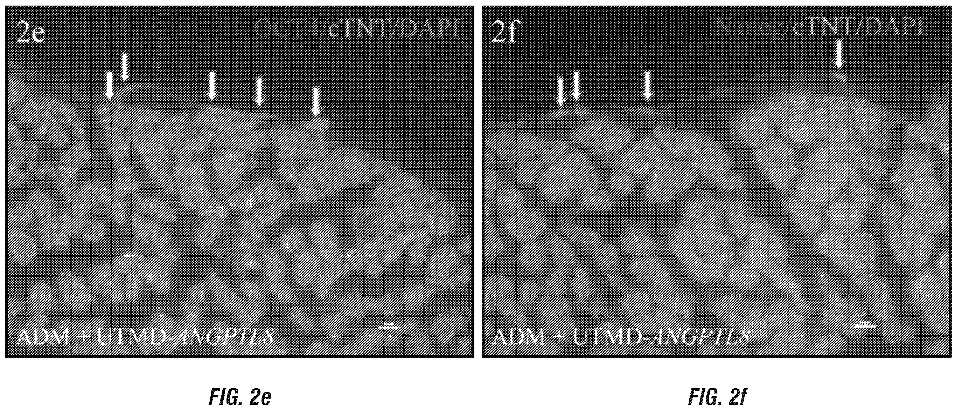

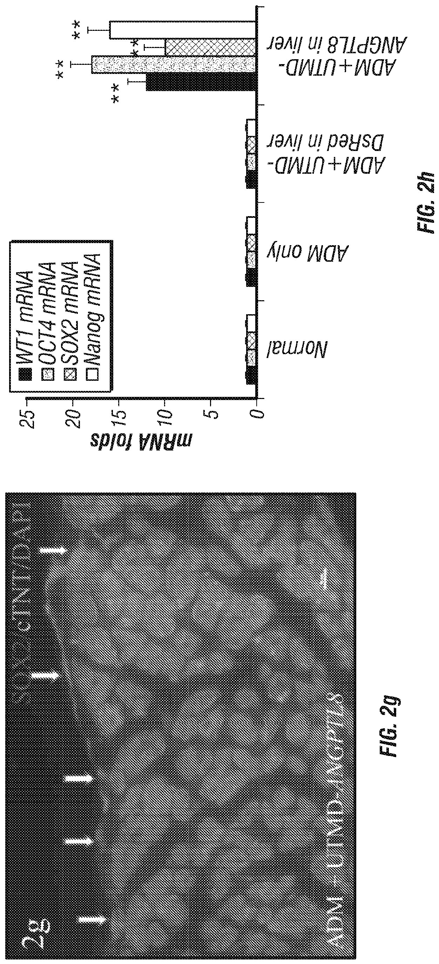

The epicardium is a single layer of quiescent epithelial cells located at the surface of the myocardium layer that does not express Wt1. Previous studies have shown that the activation of epicardium-derived cells (EPDCs) undergo epithelial to mesenchymal transformation to form WT1-positive cardiac progenitor cells that then migrate into the myocardium prior to differentiation. FIG. 2 shows that WT1 signal from the nucleus of epicardial layer cells was observed, suggesting that exogenous human *ANGPTL8* activated the rat WT1gene, which is not normally expressed in epicardium of adult rat hearts. There was also activation of OCT4, Nanog and Sox2 genes and biomarkers of stem cells in epicardial layer cells after ADM plus UTMD- *ANGPTL8* gene delivery. WT1, OCT4, Nanog, and Sox2 mRNA level in UTMD- *ANGPTL8* group was 12, 18, 10 and 16-fold higher than in normal control, ADM only and ADM plus UTMD-DsRed groups (P<0.001) (FIG. 2h).

### EXAMPLE 5

### ANGPTL8 SIGNIFICANTLY ACTIVATED ISL-1 POSITIVE CARDIAC PROGENITOR CELLS IN EPICARDIAL LAYER

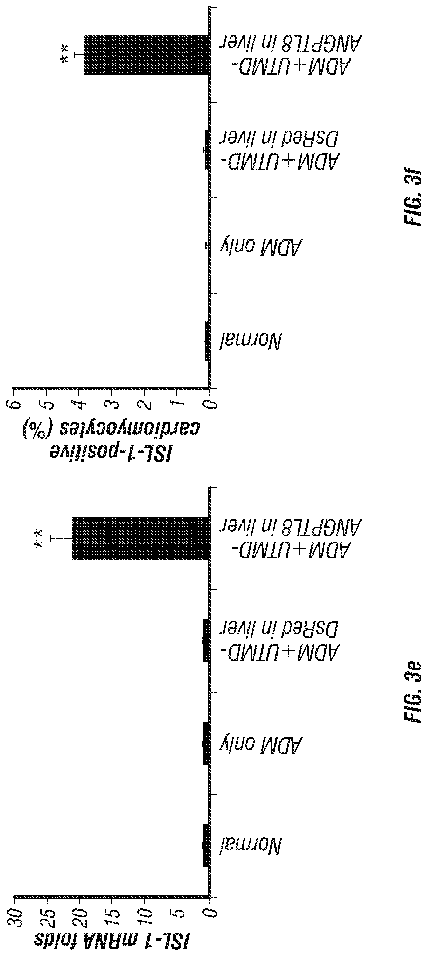

FIG. 3d shown that ISL-1 positive signals were seen in nucleus of cardiac muscle cells 28 days after ADM plus UTMD- *ANGPTL8* but not in other groups (FIG. 3a-c). The percentage of ISL-1 positive cardiac muscle cells was 3.8±1.2% in ADM plus UTMD-*ANGPTL8* group (p<0.001 vs controlling groups) (FIG. 3f). qRT-PCR shown that mRNA level of ISL-1 is 21-fold higher in ADM plus UTMD- *ANGPTL8* compared to controls (p<0.001) (FIG. 3e). Finally, the location of ISL-1 positive cells was confined to the epicardium and sub-epicardium (FIG. 3i) and not seen in endocardium and myocardium (FIG. 3g-j) (P<0.001).

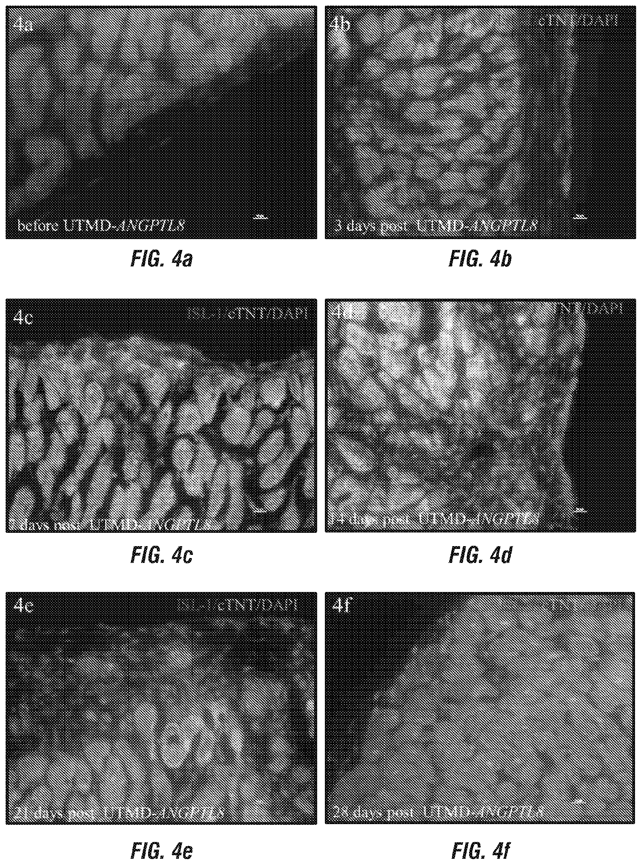

The effect of *ANGPTL8* on ISL-1 positive cardiac progenitor cells was investigated with a time-course experiment. FIG. 4 shows that no ISL-1 signal was seen in epicardial layer cells before UTMD- *ANGPTL8* gene delivery (FIG. 4a), but was observed 3 days post UTMD- *ANGPTL8* treatment (FIG. 4b). ISL-1 positive cells appeared to cluster into epicardial niches of cardiac progenitor cells 7 days post UTMD- *ANGPTL8* gene delivery (FIG. 4c), further proliferating and migrating into sub-epicardial myocardium (FIG. 4d-e) and finally differentiating into new cardiac muscle cells (FIG. 4f).

### EXAMPLE 6

### REGENERATING CARDIAC MUSCLE CELLS ARE IN PROLIFERATION

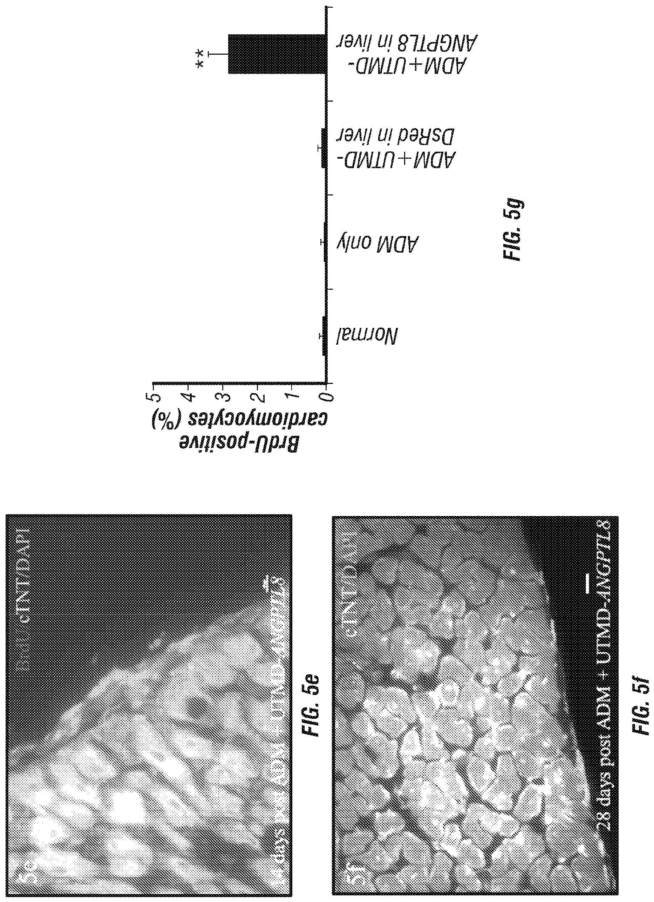

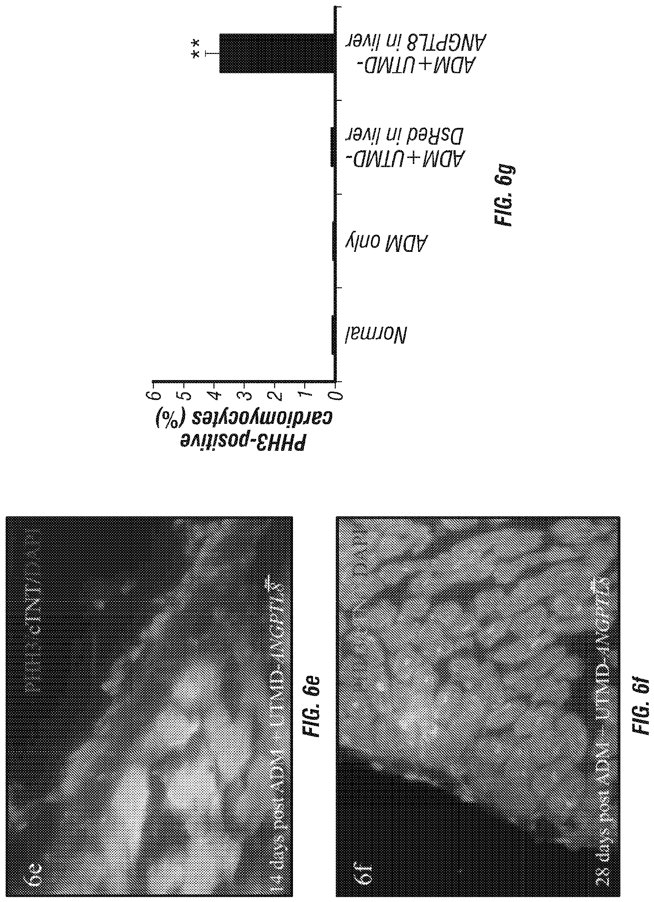

A proliferation marker (anti-BrdU ) and mitotic marker (anti-phosphohistone H3 (Ser10) (PHH3)) were utilized to demonstrate if regenerating cardiac muscle cells were proliferating. The percentage of BrdU and PHH3 positive cardiomyocytes was calculated by counting stained nuclei (pink color) from 1000 cTnT positive cardiomyocytes cells in the sub-epicardium using serial sections through each rat heart (n=6 each group). FIG. 5 shows that BrdU signal was observed within the nuclear positive cells by confocal microscopy in ADM plus UTMD- *ANGPTL8groups.* The percentage of BrdU positive sub-epicardial muscle cells in the rats treated with UTMD- *ANGPTL8* gene therapy 28 days post-UTMD (FIG. 5f) was 2.83±0.53% (p<0.001 vs controls). FIG. 5d-e shows evidence of cell proliferation (BrdU expression) confined to the epicardial layer. FIG. 6 shows another marker of proliferation (PHH3) in the epicardial layer. The percentage of PHH3 positive cardiac muscle cells at sub-epicardial area in the rats treated with UTMD- *ANGPTL8* at 28 days post UTMD (FIG. 6g) was 3.8±0.60% (p<0.001 vs controls).

### EXAMPLE 7

### ANGPTL8 SIGNIFICANTLY UPREGULATED PIRB EXPRESSION ON MEMBRANE OF CARDIAC MUSCLE CELLS AND ACTIVATED NOTCH1 SIGNAL PATHWAY

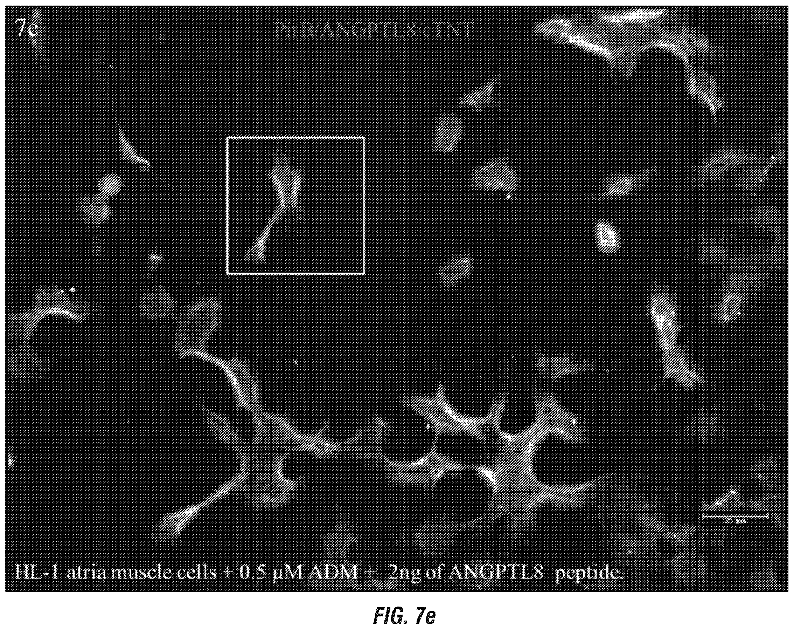

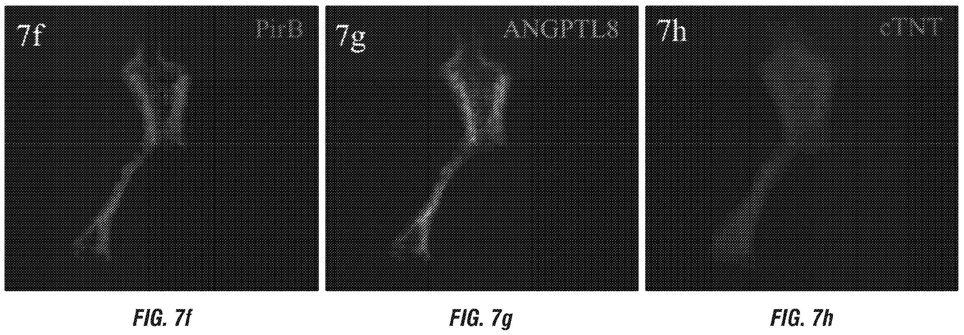

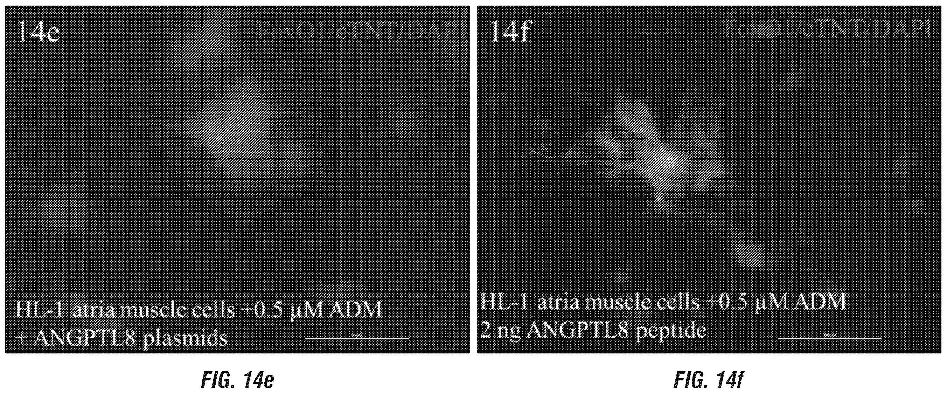

Zheng, et al (2012) reported that Paired immunoglobulin-like receptor B (PirB) in rodents or LILRB2 in human is a specific receptor of *ANGPTLs* in membrane of hematopoietic stem cells. *ANGPTLs* stimulate expansion of hematopoietic stem cells *ex vivo* by activating PirB. It also is an inhibitory receptor in neurons that inhibited regeneration of adult neuron cells [Atwal, *et al.,* 2008]. However, there are no reports of PirB in adult cardiac muscle cells. FIG. 7 shows robust presence of PirB on the surface of cTNT positive cardiac muscle cells (FIG. 7a). However, under established cardiomyopathy induced by ADM, PirB was rarely seen (FIG. 7b-c), after reversal of ADM cardiomyopathy by *ANGPTL8* gene liver delivery, PirB was again seen on the surface of membrane of cardiac muscle cells (FIG. 7d). Cultured mouse HL-1 atrial muscle cells were utilized to confirm what was found in the *in vivo* experiment, FIG. 7e shown that PirB clearly localized on the membrane of HL-1 cells as a membrane receptor, and ANGPTL8 peptide are binding with PirB (FIG.7f-h). Lin, et al (Lin, *et al.,* 2015) reported that *ANGPTLs* stimulate hematopoietic stem cells development through its receptor LILRB2 via the Notch 1 pathway. Some reports suggested that the cardiotoxicity of some anti-cancer drugs is associated with inhibition of the Notch1 pathway in heart (Rizzo, *et al.,* 2015). Notch1 signal is present in cytoplasm or nucleus of HL-1 atrial muscle cells (FIG. 13a). ADM significantly inhibited Notch1 expression (FIG. 13b-d), which appears to be restored after *ANGPTL8* gene transfection or ANGPTL8 peptide treatment (FIG. 13e-f). ADM induced cardiomyopathy is associated with FoxO1 nuclear expression of cardiac muscle cells (Chen, *et al.,* 2015), here it is further confirmed in cultured HL-1 atrial muscle cells that ANGPTL8 removed the FoxO1 nuclear expression induced by ADM (FIG. 14).

### EXAMPLE 8

### ANGPTL8 PROTEIN THERAPY ALSO REVERSED ESTABLISHED ADM CARDIOMYOPATHY

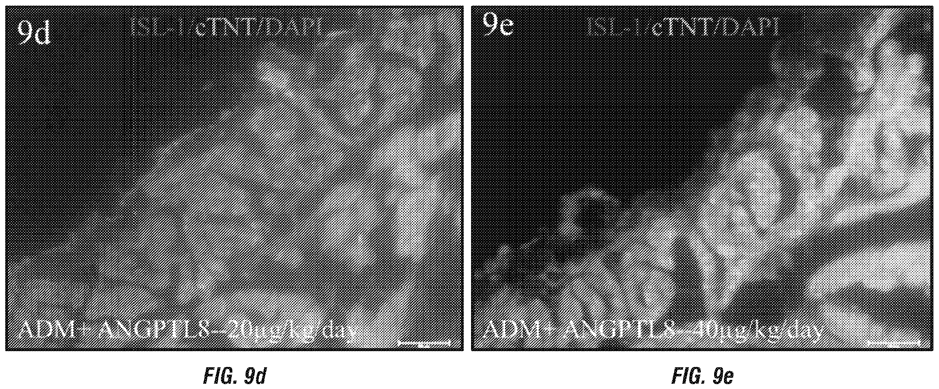

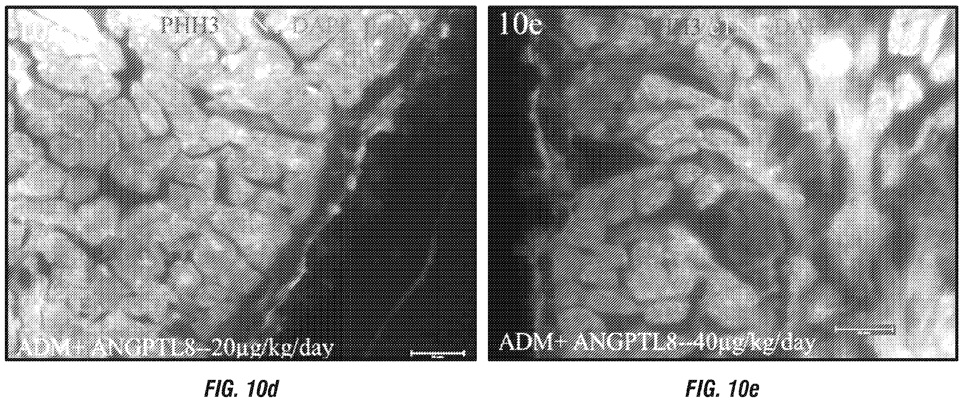

It was considered whether exogenous *ANGPTL8* protein delivery has the same effects as its gene liver delivery. Three different dosages of *ANGPTL8* protein were set for intramuscular injection daily for 14 days, FIG. 8a shows fasting plasma levels of human *ANGPTL8* protein in rats. No human *ANGPTL8* was detected in fasting plasma in ADM plus saline injection, in the three different dosages groups. However, human *ANGPTL8* was detected in fasting plasma from day 3, 7, 10 and 14 during daily injection of *ANGPTL8* protein for consistently 14 days. FIG. 8b-8c demonstrated M-mode images derived from 2D parasternal short-axis views of the left ventricle, compared with normal rat heart, ADM plus saline injection control rat heart, ADM plus *ANGPTL8*-5µg/kg rat heart, ADM plus *ANGPTL8-*20µg/kg rat heart and ADM plus *ANGPTL8*-40µg/kg rat heart. There was decreased LV fractional shortening and LV mass in adriamycin cardiomyopathy with some degree of restoration by *ANGPTL8* protein therapy. FIG. 9 shows that ISL-1 positive cells in epicardial layer were activated by *ANGPTL8* protein injection with a dose dependent manner after 14 days treatment. FIG. 10 displayed that some epicardial cells and sub-epicardial cardiac muscle cells are in proliferation after *ANGPTL8* protein therapy.

### Significance of Certain Aspects

Balmer, *et al.* (2014) reported that cardiac progenitor cells in epicardial layer of adult heart are a resource for myocardial regeneration and they are able to differentiate into cardiac muscle cells, coronary artery muscle cells and vascular endothelial cells. They appear to originate from migration of hematopoietic stem cells from bone marrow. Under some pathological states, these cells may differentiate into fibroblasts, forming scar tissue in heart. It would be useful to understand how to drive these cardiac progenitor cells to differentiate into muscle cells but not fibroblasts. There is some evidence that *ANGPTL1-7* expands hematopoietic stem cells *ex vivo* and stimulates the formation of niches of hematopoietic cells in bone marrow. This disclosure demonstrates that *ANGPTL8* can stimulate adult myocardial regeneration by proliferation of cardiac progenitor cells located at epicardium. Time-course experiments suggest that *ANGPTL8* drove the differentiation of cardiac progenitor/stem cells into ISL-1 positive cardiac progenitor cells that formed niches and migrated into sub-epicardial myocardium. A molecular mechanism by which ANGPTL8 activated these quiescent adult cardiac progenitor cells in adult hearts with established ADM cardiomyopathy may be determined.

Deng, *et al.* (Zheng, *et al.,* 2012; Deng, *et al.,* 2014; Zhang, *et al.,* 2008; Zhang, *et al.,* 2006) showed that the human immune inhibitory receptor leukocyte immunoglobulin-like receptor B2 (LILRB2) and its mouse ortholog paired immunoglobulin-like receptor (PirB) are receptors for several angiopoietin-like proteins. LILRB2 and PirB are expressed on human and mouse hematopoietic stem cells, respectively, and the binding of *ANGPTLs* to these receptors supported *ex vivo* expansion of hematopoietic stem cells. In mouse transplantation acute myeloid leukemia models, a deficiency in intracellular signaling of PirB resulted in increased differentiation of leukemia cells, revealing that PirB supports leukemia development. Their study indicated an unexpected functional significance of classical immune inhibitory receptors in maintenance of normal adult stem cells and in support of cancer development. FIG. 1 shows that PirB existed in the surface membrane of ISL-1 positive cardiac progenitor cells. This disclosure suggests that in certain aspects *ANGPTL8* expands cardiac progenitor cells in epicardium by the activation of PirB signal pathway. Although the data indicates that this inhibitory receptor signal is related to the Notch1 / FoxO1control pathway (Kitamura, *et al.,* 2007; Chen, *et al.,* 2015), one can decipher the role of PirB in adult cardiac muscle cells under physiological or pathological states. Nevertheless, the finding that *ANGPTL8* reverses established ADM induced cardiomyopathy provides a useful agent for myocardial regeneration in adult animals.

### REFERENCES

All patents and publications mentioned in this specification are indicative of the level of those skilled in the art to which the invention pertains.
Atwal JK, Pinkston-Gosse J, Syken J, Stawicki S, Wu Y, Shatz C, Tessier-Lavigne M. ( 2008) PirB is a functional receptor for myelin inhibitors of axonal regeneration. Science; 322:967-970
Balmer GM, Bollini S, Dubé KN, Martinez-Barbera JP, Williams O, Riley PR. (2014) Dynamic haematopoietic cell contribution to the developing and adult epicardium. Nat Commun. 5: 4054.
Beltrami AP, et al. (2003) Adult cardiac stem cells are multipotent and support myocardial regeneration. Cell 114(6):763-776.
Bolli R, et al. (2011) Cardiac stem cells in patients with ischaemic cardiomyopathy (SCIPIO): initial results of a randomised phase 1 trial. Lancet 378(9806):1847-1857.
Cary LC, Goebel M, Corsaro BG, Wang HG, Rosen E, Fraser MJ (1989) Transposon mutagenesis of baculoviruses: analysis of Trichoplusia ni transposon IFP2 insertions within the FP-locus of nuclear polyhedrosis viruses. Virology 172:156-169
Chai RJ, Chen S, Ding J, Grayburn P (2009) Efficient, glucose responsive, and islet-specific transgene expression by a modified rat insulin promoter. Gene Therapy 16: 1202-1209
Chen J, Chen S, Huang P, Meng XL, Clayton S, Shen JS, Grayburn PA. In vivo targeted delivery of ANGPTL8 gene for beta cell regeneration in rats. Diabetologia. 2015; 58:1036-44
Chen S, Ding JH, Bekeredjian R et al (2006) Efficient gene delivery to pancreatic islets with ultrasonic microbubble destruction technology. Proc Natl Acad Sci U S A 103: 8469-8474
Chen S, Ding JH, Grayburn PA (2007) Reversal of streptozotocin-induced diabetes in rats by gene therapy with betacellulin and pancreatic duodenal homeobox-1. Gene therapy 14: 1102-1110
Chen S, Shimoda M, Chen J, Matsumodo S, Grayburn PA (2012) Transient overexpression of cyclin D2/CDK4/GLP1 genes induces proliferation and differentiation of adult pancreatic progenitors and mediates islet regeneration. Cell Cycle 11: 695-705
Chen S, Shimoda M, Chen J, Matsumoto S, Grayburn PA (2012) Ectopic transgenic expression of NKX2.2 induced differentiation of adult pancreatic progenitors and mediates islet regeneration. Cell Cycle 11: 1544-1553
Chen S, Shimoda M, Wang MY et al (2010) Regeneration of pancreatic islets in vivo by ultrasound-targeted gene therapy. Gene Therapy 17: 1411-1420
Chen S, Shohet RV, Bekeredjian R, Frenkel P, Grayburn PA. Optimization of ultrasound parameters for cardiac gene delivery of adenoviral or plasmid deoxyribonucleic acid by ultrasound-targeted microbubble destruction. J Am Coll Cardiol 2003; 42(2):301-308.
Deng M, Lu Z, Zheng J, Wan X, Chen X, Hirayasu K, Sun H, Lam Y, Chen L, Wang Q, Song C, Huang N, Gao GF, Jiang Y, Arase H, Zhang CC. (2014) A motif in LILRB2 critical for Angptl2 binding and activation. Blood. 124:924-35.
Di Felice V, Zummo G. (2013) Stem cell populations in the heart and the role of Isll positive cells. Eur J Histochem. 57(2):e14
Eulalio A, et al. (2012) Functional screening identifies miRNAs inducing cardiac regeneration. Nature 492(7429):376-381.
Fu Z, Yao F, Abou-Samra AB, Zhang R (2013) Lipasin, thermoregulated in brown fat, is a novel but atypical member of the Angiopoietin-like protein family. Biochem. Biophys. Res. Commun. 430: 1126-1131
Iliskovic N, Singal PK. Lipid Lowering: An Important Factor in Preventing Adriamycin-Induced Heart Failure. American Journal of Pathology. 1997; 150: 727-734.
Jopling C, et al. (2010) Zebrafish heart regeneration occurs by cardiomyocyte dedifferentiation and proliferation. Nature 464(7288):606-609.
Kitamura T, Kitamura YI, Funahashi Y, Shawber CJ, Castrillon DH, Kollipara R, DePinho RA, Kitajewski J, Accili D. (2007) A Foxo/Notch pathway controls myogenic differentiation and fiber type specification. J Clin Invest. 117:2477-85.
Laugwitz KL, Moretti A, Lam J, Gruber P, Chen Y, Woodard S, Lin LZ, Cai CL, Lu MM, Reth M, Platoshyn O, Yuan JX, Evans S, Chien KR. Postnatal isl1+ cardioblasts enter fully differentiated cardiomyocyte lineages. Nature. 2005;433:647-653.
Lin MI, Price EN, Boatman S, Hagedorn EJ, Trompouki E, Satishchandran S, Carspecken CW, Uong A, DiBiase A, Yang S, Canver MC, Dahlberg A, Lu Z, Zhang CC, Orkin SH, Bernstein ID, Aster JC, White RM, Zon LI.(2015) Angiopoietin-like proteins stimulate HSPC development through interaction with notch receptor signaling. Elife.4. doi: 10.7554/eLife.05544.
Masters M, Riley PR. (2014) The epicardium signals the way towards heart regeneration. Stem Cell Res. 13(3): 683-692.
Moretti A, Caron L, Nakano A, Lam JT, Bernshausen A, Chen Y, Qyang Y, Bu L, Sasaki M, Martin-Puig S, Sun Y, Evans SM, Laugwitz KL, Chien KR. Multipotent embryonic isl1+ progenitor cells lead to cardiac, smooth muscle, and endothelial cell diversification. Cell. 2006;127:1151-1165.
Nowbar AN, et al. (2014) Discrepancies in autologous bone marrow stem cell trials and enhancement of ejection fraction (DAMASCENE): weighted regression and meta-analysis. BMJ 348:g2688.
Porrello ER, et al. (2011) Transient regenerative potential of the neonatal mouse heart. Science 331(6020):1078-1080.
Qian L, et al. (2012) In vivo reprogramming of murine cardiac fibroblasts into induced cardiomyocytes. Nature 485(7400):593-598.
Quagliarini F, Wang Y, Kozlitina J et al (2012) Atypical Angiopoietin-like protein that regulates ANGPTL3. Proc. Natl. Acad. Sci. USA 109: 19751-19756
Ren G, Kim JY, Smas CM (2012) Identification of RIFL, a novel adipocyte-enriched insulin target gene with a role in lipid metabolism. Am. J. Physiol. Endocrinol. Metab. 303: E334-E351
Rizzo P, Mele D, Caliceti C, Pannella M, Fortini C, Clementz AG, Morelli MB, Aquila G, Ameri P, Ferrari R. (2015); The role of notch in the cardiovascular system: potential adverse effects of investigational notch inhibitors; Front Oncol. 4:384
Roger VL, et al. (2012) American Heart Association Statistics Committee and Stroke Statistics Subcommittee. Heart Disease and Stroke Statistics--2012 Update: A Report From the American Heart Association. Circulation 125(2):e2-e220.
Senyo SE, et al. (2013) Mammalian heart renewal by pre-existing cardiomyocytes Nature 493(7432):433-436.
Shimoda M, Chen S, Noguchi H, Matsumoto S, Grayburn PA (2010) In vivo non-viral gene delivery of human vascular endothelial growth factor improves revascularization and restoration of euglycaemia after human islet transplantation into mouse liver. Diabetologia 53: 1669-1679
Smart N, Bollini N, et al. (2011) De novo cardiomyocytes from within the activated adult heart after injury Nature. 474(7353): 640-644.
Smart N, et al. (2011) De novo cardiomyocytes from within the activated adult heart after injury. Nature 474(7353):640-644.
Smart N, Paul R. Riley PR. (2012) The epicardium as a candidate for heart regeneration Future Cardiol. 8(1): 53-69
Song K, et al. (2012) Heart repair by reprogramming non-myocytes with cardiac transcription factors. Nature 485(7400):599-604.
Wang Y, Quagliarini F, Gusarova V et al (2013) Mice lacking ANGPTL8 (Betatrophin) manifest disrupted triglyceride metabolism without impaired glucose homeostasis. Proc Natl Acad Sci U S A 110: 16109-16114
Yi P, Park JS, Melton DA (2013) Betatrophin: a hormone that controls pancreatic beta cell proliferation. Cell 153: 747-758.
Yi P, Park JS, Melton DA (2014) Perspectives on the activities of ANGPTL8/betatrophin. Cell 159:467-468
Yusa K, Zhou L, Li MA, Bradley A, Craig NL (2011) A hyperactive piggyBac transposase for mammalian applications. Proc Natl Acad Sci U S A 108:1531-1536
Zhang CC, et al. (2006) Angiopoietin-like proteins stimulate ex vivo expansion of hematopoietic stem cells. Nat Med. 12:240-245.
Zhang CC, Kaba M, Iizuka S, Huynh H, Lodish HF. (2008) Angiopoietin-like 5 and IGFBP2 stimulate ex vivo expansion of human cord blood hematopoietic stem cells as assayed by NOD/SCID transplantation. Blood. 111:3415-3423.
Zhang R (2012) Lipasin, a novel nutritionally-regulated liver-enriched factor that regulates serum triglyceride levels. Biochem. Biophys. Res. Commun. 424: 786-792
Zhang R, Abou-Samra AB (2013) Emerging roles of Lipasin as a critical lipid regulator. Biochem Biophys Res Commun 432: 401-405
Zheng J, Umikawa M, Cui C, Li J, Chen X, Zhang C, Huynh H, Kang X, Silvany R, Wan X, Ye J, Cantó AP, Chen SH, Wang HY, Ward ES, Zhang CC. (2012) Inhibitory receptors bind ANGPTLs and support blood stem cells and leukaemia development. Nature.485:656-660.

## Claims

1. Angiopoietin-like 8 (ANGPTL8) for use in a method of treating a cardiac-related medical condition by myocardial regeneration in an individual, comprising the step of providing an effective amount of said ANGPTL8 to the individual.

2. The ANGPTL8 for the use of claim 1, wherein the method is for treating a cardiac-related medical condition selected from cardiac disease, cardiomyopathy, cardiotoxicity, congestive heart failure, myocardial infarction, cardiac ischemia, pericarditis, cardiac systolic dysfunction, and arryhthmia.

3. The ANGPTL8 for the use of claim 1 or 2, in combination with ANGPTL1, ANGPTL2, ANGPTL3, ANGPTL4, ANGPTL5, ANGPTL6, or ANGPTL7.

4. The ANGPTL8 for the use of any one of claims 1 to 3, wherein the ANGPTL8 is provided to the individual as an ANGPTL8 polynucleotide or as an ANGPTL8 polypeptide, optionally
wherein the ANGPTL8 polynucleotide or the ANGPTL8 polypeptide is provided in a carrier.

5. The ANGPTL8 for the use of claim 4, wherein the ANGPTL8 polynucleotide or the ANGPTL8 polypeptide is provided in a carrier, the carrier is a liposome, nanoparticle, or a lipid-stabilized microbubble.

6. The ANGPTL8 for the use of claim 5, wherein when the ANGPTL8 polynucleotide or the ANGPTL8 polypeptide is provided in lipid-stabilized microbubbles, upon delivery to the individual the microbubbles are exposed to ultrasound conditions sufficient to deliver the ANGPTL8 polynucleotide or the ANGPTL8 polypeptide into the cells of the individual.

7. The ANGPTL for the use of claim 5 or 6, wherein:
(i) the microbubbles further comprise albumin, polymer shell, or a graphite shell; or
(ii) the microbubbles further comprise a gas, optionally
wherein the gas is selected from the group consisting of perfluoropropane, air, sulfur hexafluoride, perfluorobutane, perfluoropentane, and nitrogen.

8. The ANGPTL8 for the use of any one of the preceding claims, wherein the ANGPTL8 is provided to the individual as an ANGPTL8 polynucleotide and the ANGPTL8 polynucleotide is comprised in a vector.

9. The ANGPTL8 for the use of claim 8, wherein the vector is selected from the group consisting of a retroviral vector, lentiviral vector, adenoviral vector, adeno-associated vector, plasmid, or synthetic linear DNA strand, optionally wherein the plasmid is a piggybac transposon gene delivery plasmid.

10. The ANGPTL8 for the use of claim 8 or 9, wherein the expression of the ANGPTL8 polynucleotide is regulated by CMV or a tissue-specific promoter, optionally wherein the tissue-specific promoter is cardiac muscle-specific.

11. The ANGPTL8 for the use of any one of the preceding claims, wherein the method is for treating cardiomyopathy that is induced by a drug, optionally wherein:
(a) the drug is a chemotherapy drug, further optionally
wherein the drug is adriamycin; or
(b) the drug is a monoclonal antibody; or
(c) the drug is selected from the group consisting of anthracyclines; taxanes; fluoropyrimidine; cyclophosphamide; bevacizumab; trastuzomab; lapatinib; sorafenib; and sunitinib.

12. The ANGPTL8 for the use of any one of the preceding claims, wherein the method is for treating cardiomyopathy, and the cardiomyopathy is:
(a) ischemic or non-ischemic cardiomyopathy; and/or
(b) is caused by long-term high blood pressure, heart valve problems, heart tissue damage from a previous heart attack, chronic rapid heart rate, metabolic disorders, nutritional deficiencies, pregnancy, alcohol abuse, drug abuse, chemotherapy drugs, viral infection, hemochromatosis, genetic condition, or a combination thereof.

13. The ANGPTL8 for the use of any one of the preceding claims, wherein the individual is provided with an additional therapy for a cardiac-related medical condition, selected from ACE Inhibitors, Aldosterone Inhibitor, Angiotensin II Receptor Blocker (ARBs); Beta-Blockers, Calcium Channel Blockers, Cholesterol-Lowering Drugs, Digoxin, Diuretics, Inotropic Therapy, Potassium or Magnesium, Vasodilators, anticoagulant medication, aspirin, or a combination thereof.

14. The ANGPTL8 for the use of any one of the preceding claims, wherein the providing step comprises injection, intravenous perfusion, intra-coronary artery myocardium perfusion, intra-artery organ perfusion by catheter, or coronary sinus perfusion catheter.

15. The ANGPTL8 for the use of any one of claims 5-14, wherein the microbubbles are delivered to the liver, heart, brain, pancreas, or combination thereof.

## Patentansprüche

1. Angiopoietin-ähnliches 8 (Angiopoietin-like 8, ANGPTL8) zur Verwendung in einem Verfahren zur Behandlung eines herzbezogenen medizinischen Zustands durch myokardiale Regeneration in einem Individuum, das den Schritt der Bereitstellung einer wirksamen Menge des ANGPTL8 an das Individuum umfasst.

2. ANGPTL8 zur Verwendung nach Anspruch 1, wobei das Verfahren zur Behandlung eines herzbezogenen medizinischen Zustands dient, der ausgewählt ist aus Herzkrankheit, Kardiomyopathie, Kardiotoxizität, kongestiver Herzinsuffizienz, Myokardinfarkt, kardialer Ischämie, Perikarditis, kardialer systolischer Dysfunktion und Arryhthmie.

3. ANGPTL8 zur Verwendung nach Anspruch 1 oder 2, in Kombination mit ANGPTL1, ANGPTL2, ANGPTL3, ANGPTL4, ANGPTL5, ANGPTL6 oder ANGPTL7.

4. ANGPTL8 zur Verwendung nach einem der Ansprüche 1 bis 3, wobei das ANGPTL8 dem Individuum als ANGPTL8-Polynukleotid oder als ANGPTL8-Polypeptid bereitgestellt wird, optional
wobei das ANGPTL8-Polynukleotid oder das ANGPTL8-Polypeptid in einem Träger bereitgestellt wird.

5. ANGPTL8 zur Verwendung nach Anspruch 4, wobei das ANGPTL8-Polynukleotid oder das ANGPTL8-Polypeptid in einem Träger bereitgestellt wird, wobei der Träger ein Liposom, ein Nanopartikel oder ein lipidstabilisiertes Mikrobläschen ist.

6. ANGPTL8 zur Verwendung nach Anspruch 5, wobei, wenn das ANGPTL8-Polynukleotid oder das ANGPTL8-Polypeptid in lipidstabilisierten Mikrobläschen bereitgestellt wird, die Mikrobläschen bei der Verabreichung an das Individuum Ultraschallbedingungen ausgesetzt werden, die ausreichen, um das ANGPTL8-Polynukleotid oder das ANGPTL8-Polypeptid in die Zellen des Individuums zu befördern.

7. ANGPTL zur Verwendung nach Anspruch 5 oder 6, wobei:
(i) die Mikrobläschen ferner Albumin, eine Polymerhülle oder eine Graphithülle umfassen; oder
(ii) die Mikrobläschen ferner ein Gas umfassen, wobei das Gas optional aus der Gruppe ausgewählt wird, die aus Perfluorpropan, Luft, Schwefelhexafluorid, Perfluorbutan, Perfluorpentan und Stickstoff besteht.

8. ANGPTL8 zur Verwendung nach einem der vorhergehenden Ansprüche, wobei das ANGPTL8 dem Individuum als ANGPTL8-Polynukleotid bereitgestellt wird und das ANGPTL8-Polynukleotid in einem Vektor enthalten ist.

9. ANGPTL8 zur Verwendung nach Anspruch 8, wobei der Vektor ausgewählt ist aus der Gruppe bestehend aus einem retroviralen Vektor, lentiviralen Vektor, adenoviralen Vektor, adeno-assoziierten Vektor, Plasmid oder synthetischen linearen DNA-Strang, wobei es sich bei dem Plasmid optional um ein Piggybac-Transposon-Genabgabeplasmid handelt.

10. ANGPTL8 zur Verwendung nach Anspruch 8 oder 9, wobei die Expression des ANGPTL8-Polynukleotids durch CMV oder einen gewebespezifischen Promotor reguliert wird, wobei der gewebespezifische Promotor optional herzmuskelspezifisch ist.

11. ANGPTL8 zur Verwendung nach einem der vorhergehenden Ansprüche, wobei das Verfahren zur Behandlung von Kardiomyopathie dient, die durch ein Medikament induziert wird, wobei optional:
(a) das Medikament ein Chemotherapie-Medikament ist, wobei das Medikament ferner optional Adriamycin ist; oder
(b) der Wirkstoff ein monoklonaler Antikörper ist; oder
(c) das Arzneimittel ausgewählt ist aus der Gruppe bestehend aus Anthracyclinen, Taxanen, Fluoropyrimidinen, Cyclophosphamid, Bevacizumab, Trastuzomab, Lapatinib, Sorafenib und Sunitinib.

12. ANGPTL8 zur Verwendung nach einem der vorhergehenden Ansprüche, wobei das Verfahren zur Behandlung von Kardiomyopathie dient und die Kardiomyopathie:
(a) ischämische oder nicht-ischämische Kardiomyopathie ist; und/oder
(b) durch Folgendes verursacht wird: langanhaltenden Bluthochdruck, Herzklappenprobleme, Schädigung des Herzgewebes durch einen früheren Herzinfarkt, chronisches Herzrasen, Stoffwechselstörungen, Ernährungsmängel, Schwangerschaft, Alkoholmissbrauch, Drogenmissbrauch, Chemotherapiemedikamente, Virusinfektion, Hämochromatose, genetische Erkrankung oder eine Kombination davon.

13. ANGPTL8 zur Verwendung nach einem der vorhergehenden Ansprüche, wobei das Individuum mit einer zusätzlichen Therapie für einen herzbezogenen medizinischen Zustand versorgt wird, die aus Folgendem ausgewählt wird: ACE-Hemmer, Aldosteron-Hemmer, Angiotensin-II-Rezeptor-Blocker (ARBs), Betablocker, Kalziumkanalblocker, cholesterinsenkende Medikamente, Digoxin, Diuretika, inotrope Therapie, Kalium oder Magnesium, Vasodilatatoren, gerinnungshemmende Medikamente, Aspirin oder eine Kombination davon.

14. ANGPTL8 zur Verwendung nach einem der vorhergehenden Ansprüche, wobei der Bereitstellungsschritt eine Injektion, intravenöse Perfusion, intrakoronare arterielle Myokardperfusion, intraarterielle Organperfusion mittels Katheter oder Koronarsinusperfusionskatheter umfasst.

15. ANGPTL8 zur Verwendung nach einem der Ansprüche 5 bis 14, wobei die Mikrobläschen der Leber, dem Herzen, dem Gehirn, der Bauchspeicheldrüse oder einer Kombination davon zugeführt werden.

## Revendications

1. Angiopoïétine 8 (ANGPTL8) pour une utilisation dans un procédé de traitement d'une affection médicale cardiaque par régénération myocardique chez un individu, comprenant l'étape consistant à fournir une quantité efficace de ladite ANGPTL8 à l'individu.

2. ANGPTL8 pour l'utilisation selon la revendication 1, dans laquelle le procédé est pour le traitement d'une affection médicale cardiaque choisie parmi une maladie cardiaque, une cardiomyopathie, une cardiotoxicité, une insuffisance cardiaque congestive, un infarctus du myocarde, une ischémie cardiaque, une péricardite, un dysfonctionnement cardiaque systolique, et une arythmie.

3. ANGPTL8 pour l'utilisation selon la revendication 1 ou 2, en combinaison avec ANGPTL1, ANGPTL2, ANGPTL3, ANGPTL4, ANGPTL5, ANGPTL6 ou ANGPTL7.

4. ANGPTL8 pour l'utilisation selon l'une quelconque des revendications 1 à 3, dans laquelle l'ANGPTL8 est fourni à l'individu sous la forme d'un polynucléotide ANGPTL8 ou d'un polypeptide ANGPTL8, éventuellement
dans laquelle le polynucléotide ANGPTL8 ou le polypeptide ANGPTL8 est fourni dans un support.

5. ANGPTL8 pour l'utilisation selon la revendication 4, dans laquelle le polynucléotide ANGPTL8 ou le polypeptide ANGPTL8 est fourni dans un support, le support est un liposome, une nanoparticule ou une microbulle stabilisée par les lipides.

6. ANGPTL8 pour l'utilisation selon la revendication 5, dans laquelle lorsque le polynucléotide ANGPTL8 ou le polypeptide ANGPTL8 est fourni dans des microbulles stabilisées par les lipides, lors de l'administration à l'individu, les microbulles sont exposées à des conditions ultrasonores suffisantes pour délivrer le polynucléotide ANGPTL8 ou le polypeptide ANGPTL8 dans les cellules de l'individu.

7. ANGPTL pour l'utilisation selon la revendication 5 ou 6, dans laquelle :
(i) les microbulles comprennent en outre de l'albumine, une enveloppe polymère ou une enveloppe en graphite ; ou
(ii) les microbulles comprennent en outre un gaz, éventuellement dans lesquelles le gaz est choisi dans le groupe constitué du perfluoropropane, de l'air, de l'hexafluorure de soufre, du perfluorobutane, du perfluoropentane, et de l'azote.

8. ANGPTL8 pour l'utilisation selon l'une quelconque des revendications précédentes, dans lequel l'ANGPTL8 est fourni à l'individu sous la forme d'un polynucléotide ANGPTL8 et le polynucléotide ANGPTL8 est compris dans un vecteur.

9. ANGPTL8 pour l'utilisation selon la revendication 8, dans laquelle le vecteur est choisi dans le groupe constitué d'un vecteur rétroviral, d'un vecteur lentiviral, d'un vecteur adénoviral, d'un vecteur adéno-associé, d'un plasmide, ou d'un brin d'ADN linéaire synthétique, éventuellement dans laquelle le plasmide est un plasmide de délivrance du gène du transposon piggybac.

10. ANGPTL8 pour l'utilisation selon la revendication 8 ou 9, dans laquelle l'expression du polynucléotide ANGPTL8 est régulée par CMV ou un promoteur tissu-spécifique, éventuellement dans laquelle le promoteur tissu-spécifique est spécifique du muscle cardiaque.

11. ANGPTL8 pour l'utilisation selon l'une quelconque des revendications précédentes, dans laquelle le procédé est pour le traitement de la cardiomyopathie qui est induite par un médicament, éventuellement dans laquelle :
(a) le médicament est un médicament de chimiothérapie, en outre éventuellement dans lequel le médicament est l'adriamycine ; ou
(b) le médicament est un anticorps monoclonal ; ou
(c) le médicament est choisi dans le groupe constitué par les anthracyclines ; taxanes ; fluoropyrimidine ; cyclophosphamide ; bevacizumab ; trastuzomab ; lapatinib ; sorafénib ; et sunitinib.

12. ANGPTL8 pour l'utilisation selon l'une quelconque des revendications précédentes, dans laquelle le procédé est pour le traitement de la cardiomyopathie, et la cardiomyopathie est :
(a) une cardiomyopathie ischémique ou non ischémique ; et/ou
(b) causée par une hypertension artérielle à long terme, des problèmes de valves cardiaques, des lésions des tissus cardiaques dues à une crise cardiaque antérieure, une accélération chronique du rythme cardiaque, des troubles métaboliques, des carences nutritionnelles, une grossesse, un abus d'alcool, une toxicomanie, des médicaments de chimiothérapie, une infection virale, l'hémochromatose, une maladie génétique ou une combinaison de ceux-ci.

13. ANGPTL8 pour l'utilisation selon l'une quelconque des revendications précédentes, dans laquelle l'individu reçoit une thérapie supplémentaire pour une condition médicale cardiaque, choisie parmi les inhibiteurs de l'ECA, l'inhibiteur d'aldostérone, le bloqueur du récepteur de l'angiotensine II (ARBs) ; les bêta-bloquants, les bloqueurs des canaux calciques, les médicaments hypocholestérolémiants, la digoxine, les diurétiques, la thérapie inotrope, le potassium ou le magnésium, les vasodilatateurs, les médicaments anticoagulants, l'aspirine ou une combinaison de ceux-ci.

14. ANGPTL8 pour l'utilisation selon l'une quelconque des revendications précédentes, dans laquelle l'étape de fourniture comprend une injection, une perfusion intraveineuse, une perfusion intra-coronaire du myocarde, une perfusion intra-artérielle d'organe par cathéter, ou un cathéter de perfusion de sinus coronaire.

15. ANGPTL8 pour l'utilisation selon l'une quelconque des revendications 5 à 14, dans laquelle les microbulles sont délivrées au foie, au cœur, au cerveau, au pancréas ou à une combinaison de ceux-ci.
